# EUROPEAN PATENT APPLICATION

(11) **EP 4 556 022 A1**
(43) Date of publication of application: **21.05.2025**
(21) Application number: 23839029.8
(22) Date of filing: 13.07.2023
(51) Int. Cl.: A61K 39/395, A61P 35/00

(54) **ANTI-NECTIN-4 ANTIBODY AND USE THEREOF**

(30) Priority: 14.07.2022 CN 202210838320
(71) Applicant: Bio-Thera Solutions, Ltd., Guangzhou, Guangdong 510530 (CN)
(72) Inventor: MEI, Xingxing, Guangzhou, Guangdong 510530 (CN); FENG, Cuiying, Guangzhou, Guangdong 510530 (CN); TANG, Weijia, Guangzhou, Guangdong 510530 (CN); ZHANG, Hui, Guangzhou, Guangdong 510530 (CN); CHEN, Junyou, Guangzhou, Guangdong 510530 (CN); WANG, Zhiwei, Guangzhou, Guangdong 510530 (CN); HUANG, Xianming, Guangzhou, Guangdong 510530 (CN); YU, Jin-Chen, Guangzhou, Guangdong 510530 (CN); LI, Shengfeng, Guangzhou, Guangdong 510530 (CN)
(74) Representative: Altmann Stößel Dick Patentanwälte PartG mbB
(86) International application number: PCT/CN2023/107283
(87) International publication number: WO 2024/012539

(57) **Abstract**

Provided are an anti-Nectin-4 antibody and the use thereof. The anti-Nectin-4 antibody or antigen-binding fragment can specifically bind to Nectin-4 and has high affinity and strong endocytosis capacity, and therefore can be used for diagnosis, prevention, and/or treatment of tumors (comprising cancers and benign tumors).

## Description

### TECHNICAL FIELD

The present application relates to the field of immunology, and in particular, to an anti-Nectin-4 antibody and use thereof.

### BACKGROUND

Nectin-4 is one of the cell adhesion molecules (CAMs) in the immunoglobulin superfamily (IgSF). It is a calcium-independent cell adhesion molecule, and is also known as poliovirus receptor-like protein 4 (PVRL4) or poliovirus receptor-related 4 (PRR4). Nectin-4 is a single-pass transmembrane protein comprising an extracellular region, a transmembrane region, and an intracellular region, where the extracellular region contains three highly glycosylated domains, namely two C2 domains proximal to the cell membrane and one V domain distal to the cell membrane. The Nectin-4 molecule binds to H protein through the V domain at the N-terminal of the Nectin-4 molecule, thereby achieving the infection of a virus to cells. Nectin-4 is involved in the establishment and maintenance of adhering junctions by interacting with cadherin. Nectin-4 mediates Ca²⁺-independent adhesion, and promotes anchorage-independent growth by driving intercellular adhesion and activating matrix-independent integrin β4/SHP-2/c-Src.

Nectin-4 is expressed during fetal development, but unlike the widespread expression of other Nectins in adult tissues, its expression in adult tissues is significantly reduced. Several institutions have confirmed that Nectin-4 is overexpressed in many tumors, including breast cancer, bladder cancer, and the like. Nectin-4 has low or moderate expression in the stratum corneum of skin, skin appendages (sweat glands and hair follicles), transitional epithelium of bladder, salivary glands, esophagus, mammary glands, and stomach, and low expression in the larynx, pituitary, placenta, testis, ureter, and uterus among normal adult tissues.

### SUMMARY

The present application relates to an anti-Nectin-4 antibody and use thereof. Specifically, the present disclosure relates to the following aspects:
1. An anti-Nectin-4 (e.g., derived from a human, a monkey such as a cynomolgus monkey, or murine) antibody or antigen-binding fragment, the anti-Nectin-4 antibody comprising one or more of the following HCDR1, HCDR2 and HCDR3, or variants thereof:
   (1) according to the Kabat numbering system, the HCDR1 sequence comprising or consisting of the sequence set forth in any one of SEQ ID NO: 8, SEQ ID NOs: 181-195, SEQ ID NOs: 234-248, and SEQ ID NOs: 304-317, the HCDR2 sequence comprising or consisting of the sequence set forth in any one of SEQ ID NO: 9, SEQ ID NOs: 196-231, SEQ ID NOs: 251-303, and SEQ ID NOs: 318-358, and the HCDR3 sequence comprising or consisting of the sequence set forth in any one of SEQ ID NO: 10, SEQ ID NO: 12, SEQ ID NO: 14, SEQ ID NO: 16, SEQ ID NO: 18, SEQ ID NO: 20, SEQ ID NO: 22, and SEQ ID NO: 24;
   (2) the HCDR1, the HCDR2, and the HCDR3 contained in the heavy chain variable region set forth in SEQ ID NO: 7, wherein
      in some embodiments, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 10;
   (3) the HCDR1, the HCDR2, and the HCDR3 contained in the heavy chain variable region set forth in SEQ ID NO: 11, wherein
      in some embodiments, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 12;
   (4) the HCDR1, the HCDR2, and the HCDR3 contained in the heavy chain variable region set forth in SEQ ID NO: 13, wherein
      in some embodiments, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 14;
   (5) the HCDR1, the HCDR2, and the HCDR3 contained in the heavy chain variable region set forth in SEQ ID NO: 15, wherein
      in some embodiments, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 16;
   (6) the HCDR1, the HCDR2, and the HCDR3 contained in the heavy chain variable region set forth in SEQ ID NO: 17, wherein
      in some embodiments, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 18;
   (7) the HCDR1, the HCDR2, and the HCDR3 contained in the heavy chain variable region set forth in SEQ ID NO: 19, wherein
      in some embodiments, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 20;
   (8) the HCDR1, the HCDR2, and the HCDR3 contained in the heavy chain variable region set forth in SEQ ID NO: 21, wherein
      in some embodiments, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 22;
   (9) the HCDR1, the HCDR2, and the HCDR3 contained in the heavy chain variable region set forth in SEQ ID NO: 23, wherein
      in some embodiments, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 24; and
   (10) the HCDR1 sequence comprising or consisting of the sequence set forth in X1X2X3MS, the HCDR2 sequence comprising or consisting of the sequence set forth in X1'IX2'X3'X4'X5'X6'X7'X8'X9'YADSVKG (SEQ ID NO: 368), and the HCDR3 sequence comprising or consisting of the sequence set forth in SEQ ID NO: 12, 14, or 16, wherein X1, X2, X3, X1', X2', X3', X4', X5', X6', X7', X8', X9', and X10' denote amino acids independently selected from any one of G, A, V, L, I, S, T, C, M, N, Q, K, R, D, E, F, Y, H, P and W,
      in some embodiments, X1 denotes S, N, T, D, or G, X2 denotes Y, F, or S, X3 denotes A, G, S, or Y, X1' denotes A, R, G, I, or W, X2' denotes S, K, Y, or D, X3' denotes G, P, S, A, Q, or W, X4' denotes S, T, G, Y, H, D, W, or I, X5' denotes G, D, T, S, A, or K, X6' denotes G, S, D, A, or W, X7' denotes S, Y, T, N, D, V, E, or G, X8' denotes T, A, N, K, I, R, S, or P, and X9' denotes Y, S, H, R, N, G, F, or D;
      in some embodiments, X1 denotes S, N, T, D, or G, X2 denotes Y, N, F, or S, X3 denotes A, G, S, or absence, X1' denotes A, R, G, W, or S, X2' denotes S, K, Y, or D, X3' denotes G, P, S, T, A, Q, or Y, X4' denotes S, T, G, Y, H, D, W, or I, X5' denotes G, D, T, S, F, or K, X6' denotes G, S, D, A, W, or absence, X7' denotes S, Y, T, N, D, V, E, or G, X8' denotes T, A, N, K, I, R, S, or P, and X9' denotes Y, S, H, R, N, F, or D;
      in some embodiments, X1 denotes S, N, D, G, or T, X2 denotes Y, F, or S, X3 denotes A, D, W, S, or Y, X1' denotes A, S, G, or V, X2' denotes S, K, I, Y, or D, X3' denotes G, P, S, A, Q, Y, T, or D, X4' denotes S, T, G, Y, H, D, or W, X5' denotes G, D, T, S, or K, X6' denotes G, S, D, A, or Y, X7' denotes S, Y, T, N, D, V, or G, X8' denotes T, A, N, K, I, R, or S, and X9' denotes Y, S, H, R, N, G, F, or D;
      in some embodiments, the HCDR1 sequence comprises or consists of the sequence set forth in any one of SEQ ID NO: 8 and SEQ ID NOs: 181-195, the HCDR2 sequence comprises or consists of the sequence set forth in any one of SEQ ID NO: 9 and SEQ ID NOs: 196-231, and the HCDR3 sequence comprises or consists of the sequence set forth in SEQ ID NO: 14;
      in some embodiments, the HCDR1 sequence comprises or consists of the sequence set forth in any one of SEQ ID NO: 8, SEQ ID NO: 181, SEQ ID NO: 185, SEQ ID NO: 186, SEQ ID NO: 187, SEQ ID NO: 194, and SEQ ID NOs: 234-248, the HCDR2 sequence comprises or consists of the sequence set forth in any one of SEQ ID NO: 9, SEQ ID NO: 202 and SEQ ID NOs: 251-303, and the HCDR3 sequence comprises or consists of the sequence set forth in SEQ ID NO: 12;
      in some embodiments, the HCDR1 sequence comprises or consists of the sequence set forth in any one of SEQ ID NO: 235, SEQ ID NO: 238, SEQ ID NO: 241, and SEQ ID NOs: 304-317, the HCDR2 sequence comprises or consists of the sequence set forth in any one of SEQ ID NOs: 318-358, and the HCDR3 sequence comprises or consists of the sequence set forth in SEQ ID NO: 16;
      in some embodiments, the HCDR1 sequence comprises or consists of the sequence set forth in SEQ ID NO: 241, the HCDR2 sequence comprises or consists of the sequence set forth in SEQ ID NO: 357, and the HCDR3 sequence comprises or consists of the sequence set forth in SEQ ID NO: 16;
      in some embodiments, the HCDR1 sequence consists of the sequence set forth in SEQ ID NO: 241, the HCDR2 sequence consists of the sequence set forth in SEQ ID NO: 357, and the HCDR3 sequence consists of the sequence set forth in SEQ ID NO: 16;
      wherein the variant sequences have 3, 2, or 1 amino acid difference (preferably a conservative amino acid substitution) from or at least 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, or 99% identity to the corresponding CDR sequences, and the variants retain the binding affinity for Nectin-4, for example, the binding dissociation equilibrium constant (KD) for the binding of the variant to Nectin-4 is about 1 µM or less, or the KD is about 100 nM to about 1 pM or less.

In some embodiments, the anti-Nectin-4 antibody or antigen-binding fragment is derived from a bird or a mammal. In some embodiments, the anti-Nectin-4 antibody is derived from a human, a mouse, a donkey, a rabbit, a goat, a camel, a llama, a horse, or a chicken origin.

In some embodiments, the anti-Nectin-4 antibody comprises the HCDR1 set forth in SEQ ID NO: 8, the HCDR2 set forth in SEQ ID NO: 9, and the HCDR3 set forth in SEQ ID NO: 10.

In some embodiments, the anti-Nectin-4 antibody comprises the HCDR1 set forth in SEQ ID NO: 8, the HCDR2 set forth in SEQ ID NO: 9, and the HCDR3 set forth in SEQ ID NO: 12.

In some embodiments, the anti-Nectin-4 antibody comprises the HCDR1 set forth in SEQ ID NO: 8, the HCDR2 set forth in SEQ ID NO: 9, and the HCDR3 set forth in SEQ ID NO: 14.

In some embodiments, the anti-Nectin-4 antibody comprises the HCDR1 set forth in SEQ ID NO: 8, the HCDR2 set forth in SEQ ID NO: 9, and the HCDR3 set forth in SEQ ID NO: 16.

In some embodiments, the anti-Nectin-4 antibody comprises the HCDR1 set forth in SEQ ID NO: 8, the HCDR2 set forth in SEQ ID NO: 9, and the HCDR3 set forth in SEQ ID NO: 18.

In some embodiments, the anti-Nectin-4 antibody comprises the HCDR1 set forth in SEQ ID NO: 8, the HCDR2 set forth in SEQ ID NO: 9, and the HCDR3 set forth in SEQ ID NO: 20.

In some embodiments, the anti-Nectin-4 antibody comprises the HCDR1 set forth in SEQ ID NO: 8, the HCDR2 set forth in SEQ ID NO: 9, and the HCDR3 set forth in SEQ ID NO: 22.

In some embodiments, the anti-Nectin-4 antibody comprises the HCDR1 set forth in SEQ ID NO: 8, the HCDR2 set forth in SEQ ID NO: 9, and the HCDR3 set forth in SEQ ID NO: 24.

In some embodiments, the anti-Nectin-4 antibody comprises the HCDR1 set forth in SEQ ID NO: 181, the HCDR2 set forth in SEQ ID NO: 196, and the HCDR3 set forth in SEQ ID NO: 14.

In some embodiments, the anti-Nectin-4 antibody comprises the HCDR1 set forth in SEQ ID NO: 182, the HCDR2 set forth in SEQ ID NO: 197, and the HCDR3 set forth in SEQ ID NO: 14.

In some embodiments, the anti-Nectin-4 antibody comprises the HCDR1 set forth in SEQ ID NO: 183, the HCDR2 set forth in SEQ ID NO: 9, and the HCDR3 set forth in SEQ ID NO: 14.

In some embodiments, the anti-Nectin-4 antibody comprises the HCDR1 set forth in SEQ ID NO: 184, the HCDR2 set forth in SEQ ID NO: 199, and the HCDR3 set forth in SEQ ID NO: 14.

In some embodiments, the anti-Nectin-4 antibody comprises the HCDR1 set forth in SEQ ID NO: 185, the HCDR2 set forth in SEQ ID NO: 200, and the HCDR3 set forth in SEQ ID NO: 14.

In some embodiments, the anti-Nectin-4 antibody comprises the HCDR1 set forth in SEQ ID NO: 184, the HCDR2 set forth in SEQ ID NO: 201, and the HCDR3 set forth in SEQ ID NO: 14.

In some embodiments, the anti-Nectin-4 antibody comprises the HCDR1 set forth in SEQ ID NO: 186, the HCDR2 set forth in SEQ ID NO: 202, and the HCDR3 set forth in SEQ ID NO: 14.

In some embodiments, the anti-Nectin-4 antibody comprises the HCDR1 set forth in SEQ ID NO: 187, the HCDR2 set forth in SEQ ID NO: 203, and the HCDR3 set forth in SEQ ID NO: 14.

In some embodiments, the anti-Nectin-4 antibody comprises the HCDR1 set forth in SEQ ID NO: 183, the HCDR2 set forth in SEQ ID NO: 204, and the HCDR3 set forth in SEQ ID NO: 14.

In some embodiments, the anti-Nectin-4 antibody comprises the HCDR1 set forth in SEQ ID NO: 182, the HCDR2 set forth in SEQ ID NO: 205, and the HCDR3 set forth in SEQ ID NO: 14.

In some embodiments, the anti-Nectin-4 antibody comprises the HCDR1 set forth in SEQ ID NO: 187, the HCDR2 set forth in SEQ ID NO: 206, and the HCDR3 set forth in SEQ ID NO: 14.

In some embodiments, the anti-Nectin-4 antibody comprises the HCDR1 set forth in SEQ ID NO: 188, the HCDR2 set forth in SEQ ID NO: 207, and the HCDR3 set forth in SEQ ID NO: 14.

In some embodiments, the anti-Nectin-4 antibody comprises the HCDR1 set forth in SEQ ID NO: 189, the HCDR2 set forth in SEQ ID NO: 208, and the HCDR3 set forth in SEQ ID NO: 14.

In some embodiments, the anti-Nectin-4 antibody comprises the HCDR1 set forth in SEQ ID NO: 190, the HCDR2 set forth in SEQ ID NO: 209, and the HCDR3 set forth in SEQ ID NO: 14.

In some embodiments, the anti-Nectin-4 antibody comprises the HCDR1 set forth in SEQ ID NO: 184, the HCDR2 set forth in SEQ ID NO: 210, and the HCDR3 set forth in SEQ ID NO: 14.

In some embodiments, the anti-Nectin-4 antibody comprises the HCDR1 set forth in SEQ ID NO: 183, the HCDR2 set forth in SEQ ID NO: 211, and the HCDR3 set forth in SEQ ID NO: 14.

In some embodiments, the anti-Nectin-4 antibody comprises the HCDR1 set forth in SEQ ID NO: 185, the HCDR2 set forth in SEQ ID NO: 212, and the HCDR3 set forth in SEQ ID NO: 14.

In some embodiments, the anti-Nectin-4 antibody comprises the HCDR1 set forth in SEQ ID NO: 184, the HCDR2 set forth in SEQ ID NO: 213, and the HCDR3 set forth in SEQ ID NO: 14.

In some embodiments, the anti-Nectin-4 antibody comprises the HCDR1 set forth in SEQ ID NO: 189, the HCDR2 set forth in SEQ ID NO: 214, and the HCDR3 set forth in SEQ ID NO: 14.

In some embodiments, the anti-Nectin-4 antibody comprises the HCDR1 set forth in SEQ ID NO: 191, the HCDR2 set forth in SEQ ID NO: 215, and the HCDR3 set forth in SEQ ID NO: 14.

In some embodiments, the anti-Nectin-4 antibody comprises the HCDR1 set forth in SEQ ID NO: 183, the HCDR2 set forth in SEQ ID NO: 216, and the HCDR3 set forth in SEQ ID NO: 14.

In some embodiments, the anti-Nectin-4 antibody comprises the HCDR1 set forth in SEQ ID NO: 181, the HCDR2 set forth in SEQ ID NO: 217, and the HCDR3 set forth in SEQ ID NO: 14.

In some embodiments, the anti-Nectin-4 antibody comprises the HCDR1 set forth in SEQ ID NO: 183, the HCDR2 set forth in SEQ ID NO: 218, and the HCDR3 set forth in SEQ ID NO: 14.

In some embodiments, the anti-Nectin-4 antibody comprises the HCDR1 set forth in SEQ ID NO: 192, the HCDR2 set forth in SEQ ID NO: 219, and the HCDR3 set forth in SEQ ID NO: 14.

In some embodiments, the anti-Nectin-4 antibody comprises the HCDR1 set forth in SEQ ID NO: 8, the HCDR2 set forth in SEQ ID NO: 220, and the HCDR3 set forth in SEQ ID NO: 14.

In some embodiments, the anti-Nectin-4 antibody comprises the HCDR1 set forth in SEQ ID NO: 185, the HCDR2 set forth in SEQ ID NO: 207, and the HCDR3 set forth in SEQ ID NO: 14.

In some embodiments, the anti-Nectin-4 antibody comprises the HCDR1 set forth in SEQ ID NO: 190, the HCDR2 set forth in SEQ ID NO: 222, and the HCDR3 set forth in SEQ ID NO: 14.

In some embodiments, the anti-Nectin-4 antibody comprises the HCDR1 set forth in SEQ ID NO: 183, the HCDR2 set forth in SEQ ID NO: 223, and the HCDR3 set forth in SEQ ID NO: 14.

In some embodiments, the anti-Nectin-4 antibody comprises the HCDR1 set forth in SEQ ID NO: 184, the HCDR2 set forth in SEQ ID NO: 224, and the HCDR3 set forth in SEQ ID NO: 14.

In some embodiments, the anti-Nectin-4 antibody comprises the HCDR1 set forth in SEQ ID NO: 193, the HCDR2 set forth in SEQ ID NO: 225, and the HCDR3 set forth in SEQ ID NO: 14.

In some embodiments, the anti-Nectin-4 antibody comprises the HCDR1 set forth in SEQ ID NO: 184, the HCDR2 set forth in SEQ ID NO: 226, and the HCDR3 set forth in SEQ ID NO: 14.

In some embodiments, the anti-Nectin-4 antibody comprises the HCDR1 set forth in SEQ ID NO: 194, the HCDR2 set forth in SEQ ID NO: 227, and the HCDR3 set forth in SEQ ID NO: 14.

In some embodiments, the anti-Nectin-4 antibody comprises the HCDR1 set forth in SEQ ID NO: 184, the HCDR2 set forth in SEQ ID NO: 228, and the HCDR3 set forth in SEQ ID NO: 14.

In some embodiments, the anti-Nectin-4 antibody comprises the HCDR1 set forth in SEQ ID NO: 184, the HCDR2 set forth in SEQ ID NO: 229, and the HCDR3 set forth in SEQ ID NO: 14.

In some embodiments, the anti-Nectin-4 antibody comprises the HCDR1 set forth in SEQ ID NO: 189, the HCDR2 set forth in SEQ ID NO: 230, and the HCDR3 set forth in SEQ ID NO: 14.

In some embodiments, the anti-Nectin-4 antibody comprises the HCDR1 set forth in SEQ ID NO: 190, the HCDR2 set forth in SEQ ID NO: 231, and the HCDR3 set forth in SEQ ID NO: 14.

In some embodiments, the anti-Nectin-4 antibody comprises the HCDR1 set forth in SEQ ID NO: 195, the HCDR2 set forth in SEQ ID NO: 198, and the HCDR3 set forth in SEQ ID NO: 14.

In some embodiments, the anti-Nectin-4 antibody comprises the HCDR1 set forth in SEQ ID NO: 183, the HCDR2 set forth in SEQ ID NO: 221, and the HCDR3 set forth in SEQ ID NO: 14.

In some embodiments, the anti-Nectin-4 antibody comprises the HCDR1 set forth in SEQ ID NO: 234, the HCDR2 set forth in SEQ ID NO: 251, and the HCDR3 set forth in SEQ ID NO: 12.

In some embodiments, the anti-Nectin-4 antibody comprises the HCDR1 set forth in SEQ ID NO: 8, the HCDR2 set forth in SEQ ID NO: 252, and the HCDR3 set forth in SEQ ID NO: 12.

In some embodiments, the anti-Nectin-4 antibody comprises the HCDR1 set forth in SEQ ID NO: 8, the HCDR2 set forth in SEQ ID NO: 253, and the HCDR3 set forth in SEQ ID NO: 12.

In some embodiments, the anti-Nectin-4 antibody comprises the HCDR1 set forth in SEQ ID NO: 181, the HCDR2 set forth in SEQ ID NO: 254, and the HCDR3 set forth in SEQ ID NO: 12.

In some embodiments, the anti-Nectin-4 antibody comprises the HCDR1 set forth in SEQ ID NO: 181, the HCDR2 set forth in SEQ ID NO: 255, and the HCDR3 set forth in SEQ ID NO: 12.

In some embodiments, the anti-Nectin-4 antibody comprises the HCDR1 set forth in SEQ ID NO: 181, the HCDR2 set forth in SEQ ID NO: 256, and the HCDR3 set forth in SEQ ID NO: 12.

In some embodiments, the anti-Nectin-4 antibody comprises the HCDR1 set forth in SEQ ID NO: 235, the HCDR2 set forth in SEQ ID NO: 257, and the HCDR3 set forth in SEQ ID NO: 12.

In some embodiments, the anti-Nectin-4 antibody comprises the HCDR1 set forth in SEQ ID NO: 186, the HCDR2 set forth in SEQ ID NO: 202, and the HCDR3 set forth in SEQ ID NO: 12.

In some embodiments, the anti-Nectin-4 antibody comprises the HCDR1 set forth in SEQ ID NO: 236, the HCDR2 set forth in SEQ ID NO: 258, and the HCDR3 set forth in SEQ ID NO: 12.

In some embodiments, the anti-Nectin-4 antibody comprises the HCDR1 set forth in SEQ ID NO: 237, the HCDR2 set forth in SEQ ID NO: 259, and the HCDR3 set forth in SEQ ID NO: 12.

In some embodiments, the anti-Nectin-4 antibody comprises the HCDR1 set forth in SEQ ID NO: 181, the HCDR2 set forth in SEQ ID NO: 260, and the HCDR3 set forth in SEQ ID NO: 12.

In some embodiments, the anti-Nectin-4 antibody comprises the HCDR1 set forth in SEQ ID NO: 238, the HCDR2 set forth in SEQ ID NO: 261, and the HCDR3 set forth in SEQ ID NO: 12.

In some embodiments, the anti-Nectin-4 antibody comprises the HCDR1 set forth in SEQ ID NO: 8, the HCDR2 set forth in SEQ ID NO: 262, and the HCDR3 set forth in SEQ ID NO: 12.

In some embodiments, the anti-Nectin-4 antibody comprises the HCDR1 set forth in SEQ ID NO: 235, the HCDR2 set forth in SEQ ID NO: 263, and the HCDR3 set forth in SEQ ID NO: 12.

In some embodiments, the anti-Nectin-4 antibody comprises the HCDR1 set forth in SEQ ID NO: 237, the HCDR2 set forth in SEQ ID NO: 264, and the HCDR3 set forth in SEQ ID NO: 12.

In some embodiments, the anti-Nectin-4 antibody comprises the HCDR1 set forth in SEQ ID NO: 8, the HCDR2 set forth in SEQ ID NO: 265, and the HCDR3 set forth in SEQ ID NO: 12.

In some embodiments, the anti-Nectin-4 antibody comprises the HCDR1 set forth in SEQ ID NO: 239, the HCDR2 set forth in SEQ ID NO: 266, and the HCDR3 set forth in SEQ ID NO: 12.

In some embodiments, the anti-Nectin-4 antibody comprises the HCDR1 set forth in SEQ ID NO: 237, the HCDR2 set forth in SEQ ID NO: 267, and the HCDR3 set forth in SEQ ID NO: 12.

In some embodiments, the anti-Nectin-4 antibody comprises the HCDR1 set forth in SEQ ID NO: 236, the HCDR2 set forth in SEQ ID NO: 268, and the HCDR3 set forth in SEQ ID NO: 12.

In some embodiments, the anti-Nectin-4 antibody comprises the HCDR1 set forth in SEQ ID NO: 238, the HCDR2 set forth in SEQ ID NO: 269, and the HCDR3 set forth in SEQ ID NO: 12.

In some embodiments, the anti-Nectin-4 antibody comprises the HCDR1 set forth in SEQ ID NO: 240, the HCDR2 set forth in SEQ ID NO: 270, and the HCDR3 set forth in SEQ ID NO: 12.

In some embodiments, the anti-Nectin-4 antibody comprises the HCDR1 set forth in SEQ ID NO: 241, the HCDR2 set forth in SEQ ID NO: 271, and the HCDR3 set forth in SEQ ID NO: 12.

In some embodiments, the anti-Nectin-4 antibody comprises the HCDR1 set forth in SEQ ID NO: 237, the HCDR2 set forth in SEQ ID NO: 272, and the HCDR3 set forth in SEQ ID NO: 12.

In some embodiments, the anti-Nectin-4 antibody comprises the HCDR1 set forth in SEQ ID NO: 242, the HCDR2 set forth in SEQ ID NO: 273, and the HCDR3 set forth in SEQ ID NO: 12.

In some embodiments, the anti-Nectin-4 antibody comprises the HCDR1 set forth in SEQ ID NO: 242, the HCDR2 set forth in SEQ ID NO: 274, and the HCDR3 set forth in SEQ ID NO: 12.

In some embodiments, the anti-Nectin-4 antibody comprises the HCDR1 set forth in SEQ ID NO: 185, the HCDR2 set forth in SEQ ID NO: 275, and the HCDR3 set forth in SEQ ID NO: 12.

In some embodiments, the anti-Nectin-4 antibody comprises the HCDR1 set forth in SEQ ID NO: 243, the HCDR2 set forth in SEQ ID NO: 276, and the HCDR3 set forth in SEQ ID NO: 12.

In some embodiments, the anti-Nectin-4 antibody comprises the HCDR1 set forth in SEQ ID NO: 187, the HCDR2 set forth in SEQ ID NO: 277, and the HCDR3 set forth in SEQ ID NO: 12.

In some embodiments, the anti-Nectin-4 antibody comprises the HCDR1 set forth in SEQ ID NO: 244, the HCDR2 set forth in SEQ ID NO: 278, and the HCDR3 set forth in SEQ ID NO: 12.

In some embodiments, the anti-Nectin-4 antibody comprises the HCDR1 set forth in SEQ ID NO: 245, the HCDR2 set forth in SEQ ID NO: 279, and the HCDR3 set forth in SEQ ID NO: 12.

In some embodiments, the anti-Nectin-4 antibody comprises the HCDR1 set forth in SEQ ID NO: 240, the HCDR2 set forth in SEQ ID NO: 280, and the HCDR3 set forth in SEQ ID NO: 12.

In some embodiments, the anti-Nectin-4 antibody comprises the HCDR1 set forth in SEQ ID NO: 181, the HCDR2 set forth in SEQ ID NO: 281, and the HCDR3 set forth in SEQ ID NO: 12.

In some embodiments, the anti-Nectin-4 antibody comprises the HCDR1 set forth in SEQ ID NO: 237, the HCDR2 set forth in SEQ ID NO: 282, and the HCDR3 set forth in SEQ ID NO: 12.

In some embodiments, the anti-Nectin-4 antibody comprises the HCDR1 set forth in SEQ ID NO: 8, the HCDR2 set forth in SEQ ID NO: 283, and the HCDR3 set forth in SEQ ID NO: 12.

In some embodiments, the anti-Nectin-4 antibody comprises the HCDR1 set forth in SEQ ID NO: 246, the HCDR2 set forth in SEQ ID NO: 284, and the HCDR3 set forth in SEQ ID NO: 12.

In some embodiments, the anti-Nectin-4 antibody comprises the HCDR1 set forth in SEQ ID NO: 247, the HCDR2 set forth in SEQ ID NO: 285, and the HCDR3 set forth in SEQ ID NO: 12.

In some embodiments, the anti-Nectin-4 antibody comprises the HCDR1 set forth in SEQ ID NO: 237, the HCDR2 set forth in SEQ ID NO: 286, and the HCDR3 set forth in SEQ ID NO: 12.

In some embodiments, the anti-Nectin-4 antibody comprises the HCDR1 set forth in SEQ ID NO: 237, the HCDR2 set forth in SEQ ID NO: 287, and the HCDR3 set forth in SEQ ID NO: 12.

In some embodiments, the anti-Nectin-4 antibody comprises the HCDR1 set forth in SEQ ID NO: 8, the HCDR2 set forth in SEQ ID NO: 288, and the HCDR3 set forth in SEQ ID NO: 12.

In some embodiments, the anti-Nectin-4 antibody comprises the HCDR1 set forth in SEQ ID NO: 194, the HCDR2 set forth in SEQ ID NO: 289, and the HCDR3 set forth in SEQ ID NO: 12.

In some embodiments, the anti-Nectin-4 antibody comprises the HCDR1 set forth in SEQ ID NO: 181, the HCDR2 set forth in SEQ ID NO: 290, and the HCDR3 set forth in SEQ ID NO: 12.

In some embodiments, the anti-Nectin-4 antibody comprises the HCDR1 set forth in SEQ ID NO: 243, the HCDR2 set forth in SEQ ID NO: 291, and the HCDR3 set forth in SEQ ID NO: 12.

In some embodiments, the anti-Nectin-4 antibody comprises the HCDR1 set forth in SEQ ID NO: 8, the HCDR2 set forth in SEQ ID NO: 292, and the HCDR3 set forth in SEQ ID NO: 12.

In some embodiments, the anti-Nectin-4 antibody comprises the HCDR1 set forth in SEQ ID NO: 181, the HCDR2 set forth in SEQ ID NO: 255, and the HCDR3 set forth in SEQ ID NO: 12.

In some embodiments, the anti-Nectin-4 antibody comprises the HCDR1 set forth in SEQ ID NO: 244, the HCDR2 set forth in SEQ ID NO: 293, and the HCDR3 set forth in SEQ ID NO: 12.

In some embodiments, the anti-Nectin-4 antibody comprises the HCDR1 set forth in SEQ ID NO: 247, the HCDR2 set forth in SEQ ID NO: 294, and the HCDR3 set forth in SEQ ID NO: 12.

In some embodiments, the anti-Nectin-4 antibody comprises the HCDR1 set forth in SEQ ID NO: 194, the HCDR2 set forth in SEQ ID NO: 295, and the HCDR3 set forth in SEQ ID NO: 12.

In some embodiments, the anti-Nectin-4 antibody comprises the HCDR1 set forth in SEQ ID NO: 238, the HCDR2 set forth in SEQ ID NO: 296, and the HCDR3 set forth in SEQ ID NO: 12.

In some embodiments, the anti-Nectin-4 antibody comprises the HCDR1 set forth in SEQ ID NO: 181, the HCDR2 set forth in SEQ ID NO: 297, and the HCDR3 set forth in SEQ ID NO: 12.

In some embodiments, the anti-Nectin-4 antibody comprises the HCDR1 set forth in SEQ ID NO: 181, the HCDR2 set forth in SEQ ID NO: 298, and the HCDR3 set forth in SEQ ID NO: 12.

In some embodiments, the anti-Nectin-4 antibody comprises the HCDR1 set forth in SEQ ID NO: 8, the HCDR2 set forth in SEQ ID NO: 299, and the HCDR3 set forth in SEQ ID NO: 12.

In some embodiments, the anti-Nectin-4 antibody comprises the HCDR1 set forth in SEQ ID NO: 248, the HCDR2 set forth in SEQ ID NO: 300, and the HCDR3 set forth in SEQ ID NO: 12.

In some embodiments, the anti-Nectin-4 antibody comprises the HCDR1 set forth in SEQ ID NO: 181, the HCDR2 set forth in SEQ ID NO: 301, and the HCDR3 set forth in SEQ ID NO: 12.

In some embodiments, the anti-Nectin-4 antibody comprises the HCDR1 set forth in SEQ ID NO: 237, the HCDR2 set forth in SEQ ID NO: 302, and the HCDR3 set forth in SEQ ID NO: 12.

In some embodiments, the anti-Nectin-4 antibody comprises the HCDR1 set forth in SEQ ID NO: 237, the HCDR2 set forth in SEQ ID NO: 9, and the HCDR3 set forth in SEQ ID NO: 12.

In some embodiments, the anti-Nectin-4 antibody comprises the HCDR1 set forth in SEQ ID NO: 181, the HCDR2 set forth in SEQ ID NO: 303, and the HCDR3 set forth in SEQ ID NO: 12.

In some embodiments, the anti-Nectin-4 antibody comprises the HCDR1 set forth in SEQ ID NO: 304, the HCDR2 set forth in SEQ ID NO: 318, and the HCDR3 set forth in SEQ ID NO: 16.

In some embodiments, the anti-Nectin-4 antibody comprises the HCDR1 set forth in SEQ ID NO: 304, the HCDR2 set forth in SEQ ID NO: 319, and the HCDR3 set forth in SEQ ID NO: 16.

In some embodiments, the anti-Nectin-4 antibody comprises the HCDR1 set forth in SEQ ID NO: 305, the HCDR2 set forth in SEQ ID NO: 320, and the HCDR3 set forth in SEQ ID NO: 16.

In some embodiments, the anti-Nectin-4 antibody comprises the HCDR1 set forth in SEQ ID NO: 306, the HCDR2 set forth in SEQ ID NO: 321, and the HCDR3 set forth in SEQ ID NO: 16.

In some embodiments, the anti-Nectin-4 antibody comprises the HCDR1 set forth in SEQ ID NO: 307, the HCDR2 set forth in SEQ ID NO: 322, and the HCDR3 set forth in SEQ ID NO: 16.

In some embodiments, the anti-Nectin-4 antibody comprises the HCDR1 set forth in SEQ ID NO: 308, the HCDR2 set forth in SEQ ID NO: 323, and the HCDR3 set forth in SEQ ID NO: 16.

In some embodiments, the anti-Nectin-4 antibody comprises the HCDR1 set forth in SEQ ID NO: 306, the HCDR2 set forth in SEQ ID NO: 324, and the HCDR3 set forth in SEQ ID NO: 16.

In some embodiments, the anti-Nectin-4 antibody comprises the HCDR1 set forth in SEQ ID NO: 306, the HCDR2 set forth in SEQ ID NO: 321, and the HCDR3 set forth in SEQ ID NO: 16.

In some embodiments, the anti-Nectin-4 antibody comprises the HCDR1 set forth in SEQ ID NO: 305, the HCDR2 set forth in SEQ ID NO: 325, and the HCDR3 set forth in SEQ ID NO: 16.

In some embodiments, the anti-Nectin-4 antibody comprises the HCDR1 set forth in SEQ ID NO: 309, the HCDR2 set forth in SEQ ID NO: 326, and the HCDR3 set forth in SEQ ID NO: 16.

In some embodiments, the anti-Nectin-4 antibody comprises the HCDR1 set forth in SEQ ID NO: 310, the HCDR2 set forth in SEQ ID NO: 327, and the HCDR3 set forth in SEQ ID NO: 16.

In some embodiments, the anti-Nectin-4 antibody comprises the HCDR1 set forth in SEQ ID NO: 311, the HCDR2 set forth in SEQ ID NO: 328, and the HCDR3 set forth in SEQ ID NO: 16.

In some embodiments, the anti-Nectin-4 antibody comprises the HCDR1 set forth in SEQ ID NO: 305, the HCDR2 set forth in SEQ ID NO: 329, and the HCDR3 set forth in SEQ ID NO: 16.

In some embodiments, the anti-Nectin-4 antibody comprises the HCDR1 set forth in SEQ ID NO: 238, the HCDR2 set forth in SEQ ID NO: 330, and the HCDR3 set forth in SEQ ID NO: 16.

In some embodiments, the anti-Nectin-4 antibody comprises the HCDR1 set forth in SEQ ID NO: 309, the HCDR2 set forth in SEQ ID NO: 331, and the HCDR3 set forth in SEQ ID NO: 16.

In some embodiments, the anti-Nectin-4 antibody comprises the HCDR1 set forth in SEQ ID NO: 305, the HCDR2 set forth in SEQ ID NO: 332, and the HCDR3 set forth in SEQ ID NO: 16.

In some embodiments, the anti-Nectin-4 antibody comprises the HCDR1 set forth in SEQ ID NO: 308, the HCDR2 set forth in SEQ ID NO: 333, and the HCDR3 set forth in SEQ ID NO: 16.

In some embodiments, the anti-Nectin-4 antibody comprises the HCDR1 set forth in SEQ ID NO: 310, the HCDR2 set forth in SEQ ID NO: 334, and the HCDR3 set forth in SEQ ID NO: 16.

In some embodiments, the anti-Nectin-4 antibody comprises the HCDR1 set forth in SEQ ID NO: 307, the HCDR2 set forth in SEQ ID NO: 335, and the HCDR3 set forth in SEQ ID NO: 16.

In some embodiments, the anti-Nectin-4 antibody comprises the HCDR1 set forth in SEQ ID NO: 312, the HCDR2 set forth in SEQ ID NO: 336, and the HCDR3 set forth in SEQ ID NO: 16.

In some embodiments, the anti-Nectin-4 antibody comprises the HCDR1 set forth in SEQ ID NO: 310, the HCDR2 set forth in SEQ ID NO: 337, and the HCDR3 set forth in SEQ ID NO: 16.

In some embodiments, the anti-Nectin-4 antibody comprises the HCDR1 set forth in SEQ ID NO: 311, the HCDR2 set forth in SEQ ID NO: 338, and the HCDR3 set forth in SEQ ID NO: 16.

In some embodiments, the anti-Nectin-4 antibody comprises the HCDR1 set forth in SEQ ID NO: 313, the HCDR2 set forth in SEQ ID NO: 339, and the HCDR3 set forth in SEQ ID NO: 16.

In some embodiments, the anti-Nectin-4 antibody comprises the HCDR1 set forth in SEQ ID NO: 305, the HCDR2 set forth in SEQ ID NO: 340, and the HCDR3 set forth in SEQ ID NO: 16.

In some embodiments, the anti-Nectin-4 antibody comprises the HCDR1 set forth in SEQ ID NO: 310, the HCDR2 set forth in SEQ ID NO: 341, and the HCDR3 set forth in SEQ ID NO: 16.

In some embodiments, the anti-Nectin-4 antibody comprises the HCDR1 set forth in SEQ ID NO: 314, the HCDR2 set forth in SEQ ID NO: 339, and the HCDR3 set forth in SEQ ID NO: 16.

In some embodiments, the anti-Nectin-4 antibody comprises the HCDR1 set forth in SEQ ID NO: 315, the HCDR2 set forth in SEQ ID NO: 342, and the HCDR3 set forth in SEQ ID NO: 16.

In some embodiments, the anti-Nectin-4 antibody comprises the HCDR1 set forth in SEQ ID NO: 310, the HCDR2 set forth in SEQ ID NO: 343, and the HCDR3 set forth in SEQ ID NO: 16.

In some embodiments, the anti-Nectin-4 antibody comprises the HCDR1 set forth in SEQ ID NO: 310, the HCDR2 set forth in SEQ ID NO: 344, and the HCDR3 set forth in SEQ ID NO: 16.

In some embodiments, the anti-Nectin-4 antibody comprises the HCDR1 set forth in SEQ ID NO: 310, the HCDR2 set forth in SEQ ID NO: 339, and the HCDR3 set forth in SEQ ID NO: 16.

In some embodiments, the anti-Nectin-4 antibody comprises the HCDR1 set forth in SEQ ID NO: 305, the HCDR2 set forth in SEQ ID NO: 345, and the HCDR3 set forth in SEQ ID NO: 16.

In some embodiments, the anti-Nectin-4 antibody comprises the HCDR1 set forth in SEQ ID NO: 305, the HCDR2 set forth in SEQ ID NO: 346, and the HCDR3 set forth in SEQ ID NO: 16.

In some embodiments, the anti-Nectin-4 antibody comprises the HCDR1 set forth in SEQ ID NO: 306, the HCDR2 set forth in SEQ ID NO: 347, and the HCDR3 set forth in SEQ ID NO: 16.

In some embodiments, the anti-Nectin-4 antibody comprises the HCDR1 set forth in SEQ ID NO: 307, the HCDR2 set forth in SEQ ID NO: 339, and the HCDR3 set forth in SEQ ID NO: 16.

In some embodiments, the anti-Nectin-4 antibody comprises the HCDR1 set forth in SEQ ID NO: 235, the HCDR2 set forth in SEQ ID NO: 348, and the HCDR3 set forth in SEQ ID NO: 16.

In some embodiments, the anti-Nectin-4 antibody comprises the HCDR1 set forth in SEQ ID NO: 314, the HCDR2 set forth in SEQ ID NO: 349, and the HCDR3 set forth in SEQ ID NO: 16.

In some embodiments, the anti-Nectin-4 antibody comprises the HCDR1 set forth in SEQ ID NO: 316, the HCDR2 set forth in SEQ ID NO: 350, and the HCDR3 set forth in SEQ ID NO: 16.

In some embodiments, the anti-Nectin-4 antibody comprises the HCDR1 set forth in SEQ ID NO: 306, the HCDR2 set forth in SEQ ID NO: 351, and the HCDR3 set forth in SEQ ID NO: 16.

In some embodiments, the anti-Nectin-4 antibody comprises the HCDR1 set forth in SEQ ID NO: 304, the HCDR2 set forth in SEQ ID NO: 352, and the HCDR3 set forth in SEQ ID NO: 16.

In some embodiments, the anti-Nectin-4 antibody comprises the HCDR1 set forth in SEQ ID NO: 309, the HCDR2 set forth in SEQ ID NO: 353, and the HCDR3 set forth in SEQ ID NO: 16.

In some embodiments, the anti-Nectin-4 antibody comprises the HCDR1 set forth in SEQ ID NO: 310, the HCDR2 set forth in SEQ ID NO: 354, and the HCDR3 set forth in SEQ ID NO: 16.

In some embodiments, the anti-Nectin-4 antibody comprises the HCDR1 set forth in SEQ ID NO: 305, the HCDR2 set forth in SEQ ID NO: 355, and the HCDR3 set forth in SEQ ID NO: 16.

In some embodiments, the anti-Nectin-4 antibody comprises the HCDR1 set forth in SEQ ID NO: 305, the HCDR2 set forth in SEQ ID NO: 356, the HCDR3 set forth in SEQ ID NO: 16, the LCDR1 set forth in SEQ ID NO: 26, the LCDR2 set forth in SEQ ID NO: 27, and the LCDR3 set forth in SEQ ID NO: 28.

In some embodiments, the anti-Nectin-4 antibody comprises the HCDR1 set forth in SEQ ID NO: 241, the HCDR2 set forth in SEQ ID NO: 357, and the HCDR3 set forth in SEQ ID NO: 16.

In some embodiments, the anti-Nectin-4 antibody comprises the HCDR1 set forth in SEQ ID NO: 317, the HCDR2 set forth in SEQ ID NO: 358, and the HCDR3 set forth in SEQ ID NO: 16.

In some embodiments, the anti-Nectin-4 antibody further comprises the LCDR1 set forth in SEQ ID NO: 26, the LCDR2 set forth in SEQ ID NO: 27, and the LCDR3 set forth in SEQ ID NO: 28.
2. The anti-Nectin-4 antibody or antigen-binding fragment according to item 1, wherein the sequences of the framework regions HFR1-HFR4 in the heavy chain variable region are set forth in SEQ ID NOs: 29-32, respectively.
3. The anti-Nectin-4 antibody or antigen-binding fragment according to item 1, wherein the sequence of the framework region HFR1 in the heavy chain variable region is set forth in the amino acid sequence of positions 1-30 of the sequence set forth in any one of SEQ ID NOs: 41-180, and the sequences of HFR2-HFR4 are set forth in SEQ ID NOs: 30-32, respectively.
4. The anti-Nectin-4 antibody or antigen-binding fragment according to item 1, wherein the anti-Nectin-4 antibody or antigen-binding fragment comprises a heavy chain variable region selected from the group consisting of the following or a variant thereof:
   (1) the heavy chain variable region comprising or consisting of the sequence set forth in SEQ ID NO: 7;
   (2) the heavy chain variable region comprising or consisting of the sequence set forth in SEQ ID NO: 11;
   (3) the heavy chain variable region comprising or consisting of the sequence set forth in SEQ ID NO: 13;
   (4) the heavy chain variable region comprising or consisting of the sequence set forth in SEQ ID NO: 15;
   (5) the heavy chain variable region comprising or consisting of the sequence set forth in SEQ ID NO: 17;
   (6) the heavy chain variable region comprising or consisting of the sequence set forth in SEQ ID NO: 19;
   (7) the heavy chain variable region comprising or consisting of the sequence set forth in SEQ ID NO: 21;
   (8) the heavy chain variable region comprising or consisting of the sequence set forth in SEQ ID NO: 23;
   (9) the heavy chain variable region comprising or consisting of the sequence set forth in any one of SEQ ID NOs: 41-180; and
   (10) the heavy chain variable region comprising or consisting of the sequence set forth in SEQ ID NO: 179,
      wherein the variant sequences have 3, 2, or 1 amino acid difference (preferably a conservative amino acid substitution) from or at least 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, or 99% identity to the corresponding variable region sequences, and the variants retain the binding affinity for Nectin-4, for example, the binding dissociation equilibrium constant (KD) for the binding of the variant to Nectin-4 is about 1 µM or less, or the KD is about 100 nM to about 1 pM or less.
5. The anti-Nectin-4 antibody or antigen-binding fragment according to any one of items 1-4, wherein the amino acid sequence of the heavy chain constant region of the anti-Nectin-4 antibody is set forth in SEQ ID NO: 37. In some embodiments, the heavy chain of the anti-Nectin-4 antibody comprises the heavy chain variable region set forth in any one of SEQ ID NO: 7, SEQ ID NO: 11, SEQ ID NO: 13, SEQ ID NO: 15, SEQ ID NO: 17, SEQ ID NO: 19, SEQ ID NO: 21, SEQ ID NO: 23, and SEQ ID NOs: 41-180, and the heavy chain constant region set forth in SEQ ID NO: 37. In some embodiments, the amino acid sequence of the heavy chain of the anti-Nectin-4 antibody is set forth in any one of SEQ ID NO: 232, SEQ ID NO: 233, SEQ ID NO: 249, and SEQ ID NOs: 360-363. In some embodiments, the amino acid sequence of the heavy chain of the anti-Nectin-4 antibody is set forth in SEQ ID NO: 360.
6. The anti-Nectin-4 antibody or antigen-binding fragment according to any one of items 1-5, wherein the antibody further comprises the LCDR1, the LCDR2, and the LCDR3 contained in the light chain variable region set forth in SEQ ID NO: 25. In some embodiments, according to the Kabat numbering system, the LCDR1 sequence comprises or consists of the sequence set forth in SEQ ID NO: 26, the LCDR2 sequence comprises or consists of the sequence set forth in SEQ ID NO: 27, and the LCDR3 sequence comprises or consists of the sequence set forth in SEQ ID NO: 28. In some embodiments, the sequences of the framework regions LFR1-LFR4 in the light chain variable region are set forth in SEQ ID NOs: 33-36, respectively. In some embodiments, the antibody or antigen-binding fragment comprises the light chain variable region set forth in SEQ ID NO: 25. In some embodiments, the antibody or antigen-binding fragment further comprises the light chain constant region set forth in SEQ ID NO: 38.
7. The anti-Nectin-4 antibody or antigen-binding fragment according to any one of items 1-6, wherein the antigen-binding fragment is selected from Fab, Fab', F(ab')₂, F(ab)₂, Fd, Fv, dAb, Fab/c, a complementarity determining region fragment, scFv, scFv multimers, disulfide-stabilized Fv (dsFv), (dsFv)₂, bispecific dsFv (dsFv-dsFv'), a diabody, a disulfide-stabilized diabody (ds-diabody), a multispecific antibody formed from a portion of an antibody comprising one or more CDRs, a single domain antibody (sdAb), a nanobody, a domain antibody, or a bivalent domain antibody.
8. A polypeptide specifically binding to Nectin-4, wherein the polypeptide specifically binding to Nectin-4 is selected from the group consisting of:
   (1) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in any one of SEQ ID NO: 8, SEQ ID NOs: 181-195, SEQ ID NOs: 234-248, and SEQ ID NOs: 304-317, the HCDR2 sequence comprises or consists of the sequence set forth in any one of SEQ ID NO: 9, SEQ ID NOs: 196-231, SEQ ID NOs: 251-303, and SEQ ID NOs: 318-358, and the HCDR3 sequence comprises or consists of the sequence set forth in any one of SEQ ID NO: 10, SEQ ID NO: 12, SEQ ID NO: 14, SEQ ID NO: 16, SEQ ID NO: 18, SEQ ID NO: 20, SEQ ID NO: 22, and SEQ ID NO: 24;
   (2) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 10;
   (3) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in any one of SEQ ID NO: 8, SEQ ID NO: 181, SEQ ID NO: 185, SEQ ID NO: 186, SEQ ID NO: 187, SEQ ID NO: 194, and SEQ ID NOs: 234-248, the HCDR2 sequence comprises or consists of the sequence set forth in any one of SEQ ID NO: 9, SEQ ID NO: 202, and SEQ ID NOs: 251-303, and the HCDR3 sequence comprises or consists of the sequence set forth in SEQ ID NO: 12;
   (4) a polypeptide, comprising sequences of an HCDR1, an HCDR2 and an HCDR3, or variants thereof, wherein the polypeptide serves as a portion of the anti-Nectin-4 antibody specifically binding to Nectin-4, wherein the HCDR1 sequence comprises or consists of the sequence set forth in any one of SEQ ID NO: 8 and SEQ ID NOs: 181-195, the HCDR2 sequence comprises or consists of the sequence set forth in any one of SEQ ID NO: 9 and SEQ ID NOs: 196-231, and the HCDR3 sequence comprises or consists of the sequence set forth in SEQ ID NO: 14;
   (5) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in any one of SEQ ID NO: 8, SEQ ID NO: 235, SEQ ID NO: 238, SEQ ID NO: 241, and SEQ ID NOs: 304-317, the HCDR2 sequence comprises or consists of the sequence set forth in any one of SEQ ID NO: 9 and SEQ ID NOs: 318-358, and the HCDR3 sequence comprises or consists of the sequence set forth in SEQ ID NO: 16;
   (6) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 18;
   (7) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 20;
   (8) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 22;
   (9) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 24;
   (10) a polypeptide, comprising the sequence set forth in SEQ ID NO: 7 or a variant thereof, wherein the polypeptide serves as a portion of an anti-Nectin-4 antibody specifically binding to Nectin-4;
   (11) a polypeptide, comprising the sequence set forth in SEQ ID NO: 11 or a variant thereof, wherein the polypeptide serves as a portion of an anti-Nectin-4 antibody specifically binding to Nectin-4;
   (12) a polypeptide, comprising the sequence set forth in SEQ ID NO: 13 or a variant thereof, wherein the polypeptide serves as a portion of an anti-Nectin-4 antibody specifically binding to Nectin-4;
   (13) a polypeptide, comprising the sequence set forth in SEQ ID NO: 15 or a variant thereof, wherein the polypeptide serves as a portion of an anti-Nectin-4 antibody specifically binding to Nectin-4;
   (14) a polypeptide, comprising the sequence set forth in SEQ ID NO: 17 or a variant thereof, wherein the polypeptide serves as a portion of an anti-Nectin-4 antibody specifically binding to Nectin-4;
   (15) a polypeptide, comprising the sequence set forth in SEQ ID NO: 19 or a variant thereof, wherein the polypeptide serves as a portion of an anti-Nectin-4 antibody specifically binding to Nectin-4;
   (16) a polypeptide, comprising the sequence set forth in SEQ ID NO: 21 or a variant thereof, wherein the polypeptide serves as a portion of an anti-Nectin-4 antibody specifically binding to Nectin-4;
   (17) a polypeptide, comprising the sequence set forth in SEQ ID NO: 23 or a variant thereof, wherein the polypeptide serves as a portion of an anti-Nectin-4 antibody specifically binding to Nectin-4; and
   (18) a polypeptide, comprising the sequence set forth in any one of SEQ ID NOs: 41-180 or a variant thereof, wherein the polypeptide serves as a portion of an anti-Nectin-4 antibody specifically binding to Nectin-4.

In some embodiments, the polypeptide further comprises the sequences set forth in SEQ ID NO: 26, SEQ ID NO: 27, and SEQ ID NO: 28. In some embodiments, the polypeptide further comprises the sequence set forth in SEQ ID NO: 25 or a variant thereof.

In some embodiments, the variant sequences have 3, 2, or 1 amino acid difference (preferably a conservative amino acid substitution) from or at least 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, or 99% identity to the corresponding sequences, and the variants retain the binding affinity for Nectin-4.

In some embodiments, the antibody or antigen-binding fragment of the present disclosure binds to Nectin-4 with an equilibrium dissociation constant (KD) of about 1 µM or less. In some embodiments, the antibody or antigen-binding fragment of the present disclosure binds to Nectin-4 with a KD of about 100 nM to about 1 pM or less. In some embodiments, the antibody or antigen-binding fragment of the present disclosure binds to Nectin-4 with a KD of about 10 nM to about 1 pM or less. In some embodiments, the antibody or antigen-binding fragment of the present disclosure binds to Nectin-4 with a KD of about 10 nM to about 1 nM or less.

In some embodiments, the antibody or antigen-binding fragment of the present disclosure binds to human Nectin-4 (e.g., the antigen hNectin-4-His set forth in SEQ ID NO: 2) with a KD of about 100 nM to about 1 pM or less. In some embodiments, the antibody or antigen-binding fragment of the present disclosure binds to Nectin-4 with a KD of about 10 nM to about 1 pM or less, or about 10 nM to about 1 nM or less.
9. A biomaterial, wherein the biomaterial is
   (1) a nucleic acid molecule, encoding the anti-Nectin-4 antibody or antigen-binding fragment according to any one of items 1-7, or encoding the polypeptide specifically binding to Nectin-4 according to item 8;
   (2) a vector, comprising the nucleic acid molecule encoding the anti-Nectin-4 antibody or antigen-binding fragment according to any one of items 1-7 or encoding the polypeptide specifically binding to Nectin-4 according to item 8; or
   (3) a host cell, comprising the nucleic acid molecule encoding the anti-Nectin-4 antibody or antigen-binding fragment according to any one of items 1-7 or encoding the polypeptide specifically binding to Nectin-4 according to item 8.
10. A conjugate, comprising the anti-Nectin-4 antibody or antigen-binding fragment according to any one of items 1-7 and a conjugated moiety, wherein the conjugated moiety is a purification tag (such as a His tag), a detectable label, a drug, a toxin, a cytokine, an enzyme, or a combination thereof; alternatively, the conjugated moiety is a radioisotope, a fluorescent substance (such as fluorescein and rhodamine, etc.), a chemiluminescent substance (such as isoluminol and acridinium esters, etc.), a colored substance (such as latex particles and colloidal gold, etc.), a chemotherapeutic agent, a biotoxin, a therapeutic agent, a prodrug, a peptide, a protein, an enzyme, a virus, a lipid, a biological response modifier, or polyethylene glycol.
11. A kit, comprising the anti-Nectin-4 antibody or antigen-binding fragment according to any one of items 1-7, the polypeptide specifically binding to Nectin-4 according to item 8, or the conjugate according to item 10. In some embodiments, the kit further comprises a second antibody specifically recognizing the anti-Nectin-4 antibody. In some embodiments, the second antibody further comprises a detectable label, such as a radioisotope, a fluorescent substance, a chemiluminescent substance, a colored substance, or an enzyme. In some embodiments, the kit is for use in detecting the presence or level of Nectin-4 in a sample. In some embodiments, the kit further comprises an antibody or antigen-binding fragment thereof for an additional antigen, and/or a cytotoxic agent, and optionally, instructions for use.
12. A pharmaceutical composition, comprising the anti-Nectin-4 antibody or antigen-binding fragment according to any one of items 1-7, the polypeptide specifically binding to Nectin-4 according to item 8, or the conjugate according to item 10, wherein optionally, the pharmaceutical composition further comprises a pharmaceutically acceptable carrier and/or excipient. In some embodiments, the pharmaceutical composition is in a form suitable for administration by subcutaneous injection, intradermal injection, intravenous injection, intramuscular injection, or intralesional injection.
13. Use of the anti-Nectin-4 antibody or antigen-binding fragment according to any one of items 1-7, the polypeptide specifically binding to Nectin-4 according to item 8, or the conjugate according to item 10 in treating and/or preventing a disease or in preparing a medicament for treating and/or preventing a disease. In some embodiments, the disease is a disease associated with the expression or overexpression of Nectin-4. In some embodiments, the disease is a disease associated with the abnormal expression of Nectin-4. In some embodiments, the disease is a tumor expressing or overexpressing Nectin-4. In some embodiments, the disease is a cancer expressing or overexpressing Nectin-4. In some embodiments, the disease is a solid tumor or a hematologic tumor. In some embodiments, the disease is selected from breast cancer, pancreatic cancer, bladder cancer, urothelial carcinoma, melanoma, lung cancer, head and neck cancer, cervical cancer, ovarian cancer, choriocarcinoma, skin cancer, esophageal cancer, gastric cancer, uterine cancer, gallbladder cancer, liver cancer, hepatocellular carcinoma, urethral carcinoma, renal pelvis cancer, ureteral cancer, colorectal cancer, colon cancer, and prostate cancer.
14. A method for treating a disease, comprising: administering to a patient in need an effective amount of the anti-Nectin-4 antibody or antigen-binding fragment thereof according to any one of items 1-7, the polypeptide specifically binding to Nectin-4 according to item 8, or the conjugate according to item 10. In some embodiments, the disease is a disease associated with the expression or overexpression of Nectin-4. In some embodiments, the disease is a disease associated with the abnormal expression of Nectin-4. In some embodiments, the disease is a tumor expressing or overexpressing Nectin-4. In some embodiments, the disease is a cancer expressing or overexpressing Nectin-4. In some embodiments, the disease is a solid tumor or a hematologic tumor. In some embodiments, the disease is selected from breast cancer, pancreatic cancer, bladder cancer, urothelial carcinoma, melanoma, lung cancer, head and neck cancer, cervical cancer, ovarian cancer, choriocarcinoma, skin cancer, esophageal cancer, gastric cancer, uterine cancer, gallbladder cancer, liver cancer, hepatocellular carcinoma, urethral carcinoma, renal pelvis cancer, ureteral cancer, colorectal cancer, colon cancer, and prostate cancer.
15. A method for preparing the anti-Nectin-4 antibody or antigen-binding fragment according to any one of items 1-7 or the polypeptide specifically binding to Nectin-4 according to item 8, comprising: culturing a host cell containing a nucleic acid molecule encoding the antibody or antigen-binding fragment or the polypeptide, and optionally, isolating the antibody or antigen-binding fragment or the polypeptide.

It will be appreciated that within the scope of the present disclosure, the above features of the present disclosure and those specifically described below (e.g., in the examples) may be combined with each other to form new or preferred technical schemes. For brevity, such technical schemes will not be recited herein.

The terms involved in the present disclosure have the common meanings known to those skilled in the art. When a term has two or more definitions used and/or acceptable in the art, the definition of the term as used herein is intended to include all meanings.

The "amino acid" refers to an organic compound containing an amino group and a carboxyl group, such as an α-amino acid that can be encoded by a nucleic acid, either directly or in the form of a precursor. A single amino acid is encoded by a nucleic acid consisting of three nucleotides (so-called codon or base triplet). Each amino acid is encoded by at least one codon. The encoding of the same amino acid by different codons is known as "codon degeneracy". The amino acids include natural amino acids and non-natural amino acids. Natural amino acids include alanine (three-letter symbol: Ala, one-letter symbol: A), arginine (Arg, R), asparagine (Asn, N), aspartic acid (Asp, D), cysteine (Cys, C), glutamine (Gln, Q), glutamic acid (Glu, E), glycine (Gly, G), histidine (His, H), isoleucine (Ile, I), leucine (Leu, L), lysine (Lys, K), methionine (Met, M), phenylalanine (Phe, F), proline (Pro, P), serine (Ser, S), threonine (Thr, T), tryptophan (Trp, W), tyrosine (Tyr, Y) and valine (Val, V).

The "antibody" refers to a polypeptide or polypeptide complex that specifically recognizes and binds to an antigen. The antibody may be an intact antibody and any antigen-binding fragment or single chain thereof. The term "antibody" thus includes any protein or peptide comprising at least a portion of an immunoglobulin molecule that has biological activity for binding to an antigen. Examples of the antibody include, but are not limited to, complementarity determining region (CDR) of a heavy chain, a light chain, or a ligand binding portion thereof, heavy chain variable regions (VH), light chain variable region (VL), heavy chain constant region (CH), light chain constant regions (CL), framework region (FR), or any portion thereof, or at least a portion of a binding protein. The CDRs include light chain CDRs (LCDR1-3) and heavy chain CDRs (HCDR1-3).

It will be appreciated by those of ordinary skill in the art that the CDR regions of the antibody are responsible for the binding specificity of the antibody to the antigen. In a case where the sequences of the heavy and light chain variable regions of the antibody are known, there are currently several methods of determining the CDR regions of the antibody, including the Kabat, IMGT, Chothia, and AbM numbering systems. However, every application of the definition of CDRs regarding the antibody or variant thereof will fall within the scope of the terms defined and used herein. Given the amino acid sequences of the variable regions of the antibody, those skilled in the art can generally determine which residues are included in a particular CDR, without relying on any experimental data other than the sequence itself.

In the art, substitution with amino acid of similar or analogous properties usually will not alter the functionality of the protein. For another example, the addition of one or more amino acids at the C terminus and/or N terminus usually will not alter the functions of the protein either. For the purpose of comparing two or more amino acid sequences, the percentage "sequence homology" (also referred to herein as "amino acid identity") between a first amino acid sequence and a second amino acid sequence can be calculated by dividing [the number of amino acid residues in the first amino acid sequence that are identical to those at corresponding positions in the second amino acid sequence] by [the total number of the amino acid residues in the first amino acid sequence] and multiplying by [100%], where every deletion, insertion, substitution, or addition of amino acid residue in the second amino acid sequence is considered as a difference in single amino acid residue (position), i.e., an "amino acid difference" as defined herein, as compared to that of the first amino acid sequence. Alternatively, the sequence identity between two amino acid sequences can be calculated using known computer algorithms, such as NCBI Multiple Alignment (https://www.ncbi.nlm.nih.gov/tools/cobalt/cobalt.cgi?LINK_LOC=BlastHomeLink). For example, some other techniques, computer algorithms, and settings for determining the sequence identity are described, for example, in WO04/037999, EP0967284, EP1085089, WO00/55318, WO00/78972, WO98/49185, and GB2357768-A. Typically, for the purpose of determining the percentage "sequence identity" between two amino acid sequences according to the calculation methods described above, the amino acid sequence with a higher number of amino acid residues is considered the "first" amino acid sequence, and the other amino acid sequence is considered the "second" amino acid sequence.

Furthermore, when determining the sequence identity between two amino acid sequences, those skilled in the art may consider a so-called "conservative" amino acid substitution, which may usually be described as an amino acid substitution in which an amino acid residue is substituted with another amino acid residue having a similar chemical structure and little or no effects on functionality, activity or other biological properties of the polypeptide. Such a conservative amino acid substitution is well known in the art, for example, from WO04/037999, GB-A-3357768, WO98/49185, WO00/46383, and WO01/09300; and (preferred) types and/or combinations of such a substitution may be selected on the basis of the relevant teachings in WO04/037999 and WO98/49185 and other references cited therein.

The "conservative amino acid substitution" is an amino acid substitution in which the amino acid residue is substituted with an amino acid residue having a similar side chain. Families of amino acid residues having similar side chains have been defined in the art, including basic side chains (e.g., lysine, arginine, and histidine), acidic side chains (e.g., aspartic acid and glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, and tryptophan), β-branched side chains (e.g., threonine, valine, and isoleucine), and aromatic side chains (e.g., tyrosine, phenylalanine, tryptophan, and histidine). Thus, non-essential amino acid residues of an immunoglobulin polypeptide are preferably substituted with other amino acid residues from the same side chain family. In some other embodiments, a series of amino acids may be substituted with a series of structurally similar amino acids, which differ in sequence and/or composition of the side chain family.

Non-limiting examples of conservative amino acid substitutions are provided in the table below, where a similarity score of 0 or higher indicates that the substitution between the two amino acids is conservative.

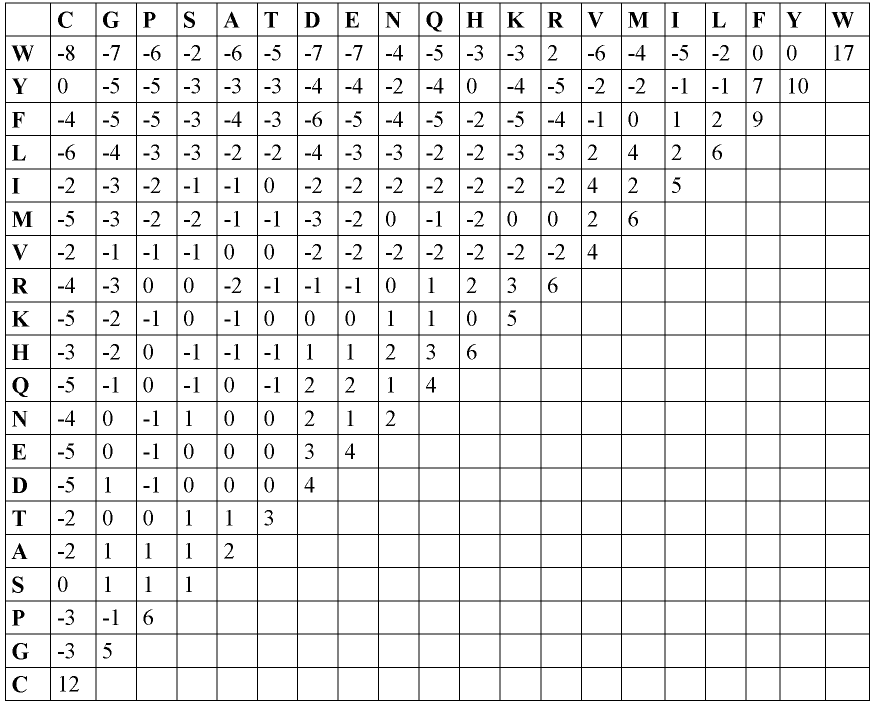

In some embodiments, the conservative substitution is such a substitution that one amino acid within the following groups (a)-(e) is substituted with another amino acid residue within the same group: (a) small aliphatic, non-polar or weakly polar residues: Ala, Ser, Thr, Pro, and Gly; (b) polar, negatively charged residues and their (uncharged) amides: Asp, Asn, Glu, and Gln; (c) polar, positively charged residues: His, Arg, and Lys; (d) large aliphatic, nonpolar residues: Met, Leu, Ile, Val, and Cys; and (e) aromatic residues: Phe, Tyr, and Trp.

In some embodiments, the conservative substitution is as follows: Ala is substituted with Gly or Ser; Arg is substituted with Lys; Asn is substituted with Gln or His; Asp is substituted with Glu; Cys is substituted with Ser; Gln is substituted with Asn; Glu is substituted with Asp; Gly is substituted with Ala or Pro; His is substituted with Asn or Gln; Ile is substituted with Leu or Val; Leu is substituted with Ile or Val; Lys is substituted with Arg, Gln, or Glu; Met is substituted with Leu, Tyr, or Ile; Phe is substituted with Met, Leu, or Tyr; Ser is substituted with Thr; Thr is substituted with Ser; Trp is substituted with Tyr; Tyr is substituted with Trp; and/or Phe is substituted with Val, Ile, or Leu.

From the general structural formula of α-amino acids, it can be seen that various α-amino acids differ in side chain R groups. According to the chemical structure of the R group, the conservative amino acid substitution is as follows: Gly, Ala, Val, Leu, and Ile may be substituted with one another; Ser and Thr may be substituted with one another; Cys and Met may be substituted with one another, Asn and Gln may be substituted with one another; Asp and Glu may be substituted with one another; Lys and Arg may be substituted with one another; Phe and Tyr may be substituted with one another.

The term "antibody fragment" or "antigen-binding fragment" includes, but is not limited to, F(ab')₂, F(ab)₂, Fab', Fab, Fv, Fd, dAb, Fab/c, complementarity determining region (CDR) fragment, single-chain Fvs (scFv), disulfide-stabilized Fv fragment (dsFv), (dsFv)₂, bispecific dsFv (dsFv-dsFv'), diabody, disulfide-stabilized diabody (ds-diabody), scFv multimer (e.g., scFv dimer, scFv trimer), multispecific antibody formed from a portion of an antibody including one or more CDRs, nanobody, single domain antibody (sdab), domain antibody, a bivalent domain antibody, or any other antibody fragments that bind to an antigen but do not contain an intact antibody structure. Regardless of structure, the antigen-binding fragment includes any polypeptide or polypeptide complex capable of binding to the same antigen to which the parent antibody or parent antibody fragment binds. The structure of dsFv is introduced in Mao C S et al, "Disulfide stabilized Fv Fragments (dsFv): a New Type of Engineering Antibody Fragments". Progress in Biochemistry and Biophysics, 1998, 25(6):525-526. The antigen-binding fragments known as "domain antibodies" (dAbs) are summarized in Holt et al, "Domain antibodies: proteins for therapy". Trends in Biotechnology (2003): Vol. 21, No. 11: 484-490, where the antigen-binding fragment only contains a VH or VL domain of the antibody, and is thus smaller than, for example, Fab and scFv. The dAbs are the smallest known antigen-binding fragments of an antibody, ranging from 11 kDa to 15 kDa. The term "antibody fragment" includes aptamers, spiegelmers, and diabodies. The term "antibody fragment" also includes any synthetic or genetically engineered proteins that, like antibodies, may bind to a particular antigen to form a complex. Typically, the antibody fragment has at least about 50 consecutive amino acids, preferably at least about 50 consecutive amino acids, more preferably at least about 80 consecutive amino acids, and most preferably at least about 100 consecutive amino acids of the antibody of the present disclosure.

The antibody or antigen-binding fragment of the present disclosure includes modified derivatives, i.e., modified by covalent linking of any type of molecules to the antibody or antigen-binding fragment, wherein the covalent linking does not prevent the antibody or antigen-binding fragment from binding to an epitope. The antibody or antigen-binding fragment may be modified by glycosylation, acetylation, pegylation, phosphorylation, amidation, derivatization by known protecting/blocking groups, proteolytic cleavage, attachment to cellular ligands or other proteins, or the like. Any one of various chemical modifications may be made by techniques in the prior art, including but not limited to specific chemical cleavage, acetylation, formylation, metabolic synthesis of tunicamycin, and the like.

In some embodiments, the antibody or antigen-binding fragment may be conjugated with a therapeutic agent, a prodrug, a peptide, a protein, an enzyme, a virus, a lipid, a biological response modifier, an agent, or polyethylene glycol (PEG).

In some embodiments, the Nectin-4 antigen is derived from human, monkey (such as a cynomolgus monkey), or a murine species.

The antibody described herein may be derived from any animals, including birds and mammals. Preferably, the antibody is derived from a human, a mouse, a donkey, a rabbit, a goat, a camel, a horse, or a chicken origin.

The term "isolated" as used herein with respect to cells, nucleic acids, polypeptides, antibodies and the like, e.g., "isolated" DNA, RNA, polypeptides or antibodies, refers to molecules that are separated from one or more other components, e.g., DNA or RNA, in the natural cellular environment. The term "isolated" as used herein also refers to nucleic acids or peptides that are substantially free of cellular materials, viral materials, or cell media when produced by recombinant DNA techniques, or free of chemical precursors or other chemicals when chemically synthesized. In addition, the "isolated nucleic acid" is intended to include nucleic acid fragments that are not and will not be present in nature. The "isolated" is also used to refer to cells or polypeptides that are separated from other cellular proteins or tissue. The isolated polypeptides are intended to include both purified and recombinant polypeptides. The isolated polypeptides, antibodies, and the like are usually prepared by at least one purification procedure. In some embodiments, the purity of the isolated nucleic acids, polypeptides, and antibodies is at least about 50%, about 60%, about 70%, about 80%, about 90%, about 95%, about 99%, or a range between any two of these values (inclusive) or any value therein.

The polynucleotide consists of a specific sequence of four nucleotide bases: adenine (A), cytosine (C), guanine (G), thymine (T)/uracil (U, instead of thymine when the polynucleotide is RNA). The "polynucleotide sequence" may be a letter representation of the polynucleotide molecule. The letter representation can be input into a database in a computer with a central processing unit and used for bioinformatics applications, such as functional genomics and homology searches.

The terms "polynucleotide" and "nucleic acid" are used interchangeably to refer to a polymeric form of nucleotides of any length, whether deoxyribonucleotides or ribonucleotides or analogs thereof. The polynucleotide may have any three-dimensional structure and may perform any functions, either known or unknown. The following are non-limiting examples of polynucleotides: genes or gene fragments (such as probes, primers, EST or SAGE tags), exons, introns, messenger RNA (mRNA), transport RNA, ribosomal RNA, ribozymes, cDNA, dsRNA, siRNA, miRNA, recombinant polynucleotides, branched polynucleotides, plasmids, vectors, isolated DNA of any sequence, isolated RNA of any sequence, and nucleic acid probes and primers. The polynucleotides may include modified nucleotides, such as methylated nucleotides and nucleotide analogs. If present, structural modifications to the nucleotide can be made before or after the assembly of the polynucleotide. The sequence of nucleotides may be interrupted by non-nucleotide components. The polynucleotide may be further modified after polymerization, such as by conjugation with a labeling component. This term also refers to double-stranded and single-stranded molecules. Unless otherwise stated or required, examples of any polynucleotide disclosed herein include a double-stranded form and each of the two complementary single-stranded forms known or predicted to constitute the double-stranded form.

The term "encoding" as applied to a polynucleotide refers to a polynucleotide known to "encode" a polypeptide. When in its native state or manipulated by methods well known to those skilled in the art, the polynucleotide can be transcribed and/or translated to produce the polypeptide and/or a fragment thereof.

The "treatment" refers to both therapeutic treatment and prophylactic or preventative measures, the purpose of which is to prevent, slow down, ameliorate, or halt undesirable physiological changes or disorders, such as the progression of a disease, including, but not limited to, alleviation of symptoms, diminishment of the extent of disease, stabilized (i.e., not worsening) state of disease, delay or slowing of progressive disease, amelioration, palliation, alleviation, or elimination (whether partial or total) of state of disease, prolongation of life expectancy as compared with that in the absence of treatment, and the like, as either detectable or undetectable. The patients in need of the treatment include patients already having a condition or disorder, patients susceptible to a condition or disorder, or patients in need of prevention of the condition or disorder, or patients who may or are expected to benefit from the administration of the antibody, the antigen-binding fragment, or the pharmaceutical composition of the present disclosure for detection, diagnostic procedures, and/or treatment.

The effective dosage and therapeutic regimen for treating a particular patient will depend upon a variety of factors including the particular antibody, the antigen-binding fragment or derivative thereof used, the age and body weight, general health condition, sex and diet of the patient, and the time of administration, frequency of excretion, drug combination, and the severity of the particular disease being treated. These factors are judged by healthcare providers, who are included within the scope of those of ordinary skill in the art. The dosage employed can be determined by pharmacological and pharmacokinetic principles well known in the art. In some embodiments, the antibody of the present disclosure is administered to the patient at 0.01 mg/kg to 100 mg/kg body weight of the patient per dose. In some embodiments, the treatment is given once weekly or monthly.

The "patient" refers to any mammal in need of diagnosis, prognosis, or treatment, including human, dog, cat, rabbit, murine, horse, cattle, and the like. In some embodiments, the patient is a human patient.

The "pharmaceutically acceptable" refers to materials listed in pharmacopeia for use in drugs for animals, and in particular for humans. In addition, the "pharmaceutically acceptable carrier and/or excipient" will generally be any type of non-toxic solid, semi-solid or liquid filler, diluent, encapsulating material, or formulation additive.

The term "carrier" refers to a diluent, an adjuvant, an excipient, or a carrier that can be administered to a patient together with the active ingredient. Such pharmaceutical carriers may be sterile liquids, such as water and oils, including oils originating from petroleum, animal, plant or synthesis, such as peanut oil, soybean oil, mineral oil, and sesame oil. Water is a preferred carrier when the pharmaceutical composition is administered intravenously. A saline aqueous solution, a glucose aqueous solution, and a glycerol solution may also be used as a liquid carrier, especially for injection. Suitable pharmaceutical excipients include starch, glucose, gelatin, malt, rice, flour, chalk, silica gel, sodium stearate, glycerol monostearate, talc, skimmed milk powder, glycerol, propylene, glycol, water, ethanol, and the like. If desired, the composition may also contain a small amount of a wetting agent, an emulsifier, or a pH buffering agent. Such compositions may be in the form of solutions, suspensions, emulsions, tablets, pills, capsules, pulvises, sustained-release formulations and the like. The composition may be formulated as a suppository using conventional binders and carriers such as triglyceride. Oral formulations may contain standard carriers such as starch, magnesium stearate, sodium saccharin, cellulose and magnesium carbonate of pharmaceutical grade. Examples of suitable pharmaceutical carriers are described in Remington's Pharmaceutical Sciences by E. W. Martin, which is incorporated herein by reference. Such compositions will contain a clinically effective dose of an antibody or antigen-binding fragment, preferably in a purified form, together with an appropriate amount of a carrier and/or excipient, to provide a form suitable for administration to a patient. The formulation should be suitable for the administration mode. The formulation may be encapsulated in an ampoule bottle, a disposable syringe, or a multi-dose vial made of glass or plastic.

In some embodiments, the composition is formulated into a pharmaceutical composition suitable for intravenous injection into a human body according to conventional procedures. A composition for intravenous administration is a solution in a sterile isotonic aqueous buffer. In general, the active ingredients are provided in a unit dosage form individually or as a mixture. For example, the active ingredients are encapsulated in sealed containers (such as ampoule bottles or sachets) that can indicate the amount of the active agent, in the form of lyophilized powder or anhydrous concentrate. Where the composition is administered by infusion, the composition can be dispensed in infusion bottles containing sterile, pharmaceutical grade water or saline. Where the composition is administered by injection, an ampoule bottle containing sterile water or saline for injection can be used, such that the active ingredients can be mixed before administration.

The antibody or antigen-binding fragment of the present disclosure includes neutral or salt forms. The pharmaceutically acceptable salts include, but are not limited to, salts formed with anions derived from acids such as hydrochloric acid, phosphoric acid, acetic acid, oxalic acid and tartaric acid, and salts formed with cations derived from, e.g., sodium, potassium, ammonium, calcium, iron hydroxide, isopropylamine, triethylamine, 2-ethylaminoethanol, histidine, and procaine.

The "about" refers to a conventional error range for corresponding values as readily understood by those skilled in the relevant art. In some embodiments, the "about" mentioned herein refers to the values described and ranges of ± 10%, ± 5%, or ± 1% therefrom.

The "EC₅₀", i.e., concentration for 50% of maximal effect, refers to a concentration that causes 50% of the maximal effect.

### Methods for Preparing Antibodies and Antibody-Encoding Polynucleotides

The present disclosure further discloses a polynucleotide or a nucleic acid molecule encoding the antibody, the antigen-binding fragment, and a derivative thereof of the present disclosure. The polynucleotide disclosed herein may encode a heavy chain variable region, a light chain variable region, an Fc region, a portion of a heavy chain variable region, a portion of a light chain variable region, a heavy chain, a light chain, or the like. Methods for preparing a polynucleotide encoding an antibody and for preparing an antibody are well known in the art and are described in the present disclosure.

The antibodies can be prepared by a variety of methods known in the art, including phage display methods using antibody libraries from immunoglobulin sequences. Reference may also be made to U.S. Pat. Nos. 4,444,887 and 4,716,111, and PCT Publication Nos. WO 98/46645, WO 98/50433, WO 98/24893, WO 98/16654, WO 96/34096, WO 96/33735 and WO 91/10741, which are incorporated herein by reference in their entireties.

Antibodies can be prepared by using conventional recombinant DNA techniques. Vectors and cell lines for antibody production can be selected, constructed, and cultured using techniques well known to those skilled in the art. Such techniques are described in various laboratory manuals and main publications, such as Recombinant DNA Technology for Production of Protein Therapeutics in Cultured Mammalian Cells, D. L. Hacker, F. M. Wurm, in Reference Module in Life Sciences, 2017, which, including supplementary content, is incorporated by reference in its entirety.

In some embodiments, the DNA encoding the antibody can be designed and synthesized according to the antibody amino acid sequence described herein by conventional methods, inserted into an expression vector, and transfected into a host cell. The transfected host cell is then cultured in a medium to produce the monoclonal antibody. In some embodiments, the vector expressing the antibody comprises at least one promoter element, an antibody encoding sequence, a transcription termination signal and a polyA tail. Other elements include enhancers, Kozak sequences, and donor and recipient sites flanking the inserted sequence for RNA splicing. Efficient transcription can be obtained by early and late promoters of SV40, long terminal repeats from retroviruses such as RSV, HTLV1 and HIVI, and early promoters of cytomegalovirus, and promoters from other cells such as actin promoter can also be used. Suitable expression vectors may include pIRES1neo, pRetro-Off, pRetro-On, PLXSN, or Plncx, pcDNA3.1 (+/-), pcDNA/Zeo (+/-), pcDNA3.1/Hygro(+/-), PSVL, PMSG, pRSVcat, pSV2dhfr, pBC12MI, pCS2, etc. Commonly used mammalian cells include 293 cells, Cos1 cells, Cos7 cells, CV1 cells, murine L cells, CHO cells, and the like.

In some embodiments, the inserted gene fragment should comprise a screening label, common ones of which include screening genes such as dihydrofolate reductase, glutamine synthetase, neomycin resistance and hygromycin resistance, to facilitate the screening and separation of cells that have been successfully transfected. The constructed plasmid is transfected to a host cell without the above genes, and the successfully transfected cells are cultured in a large quantity in a selective culture medium to produce the desired target protein.

The preparation of scFv can be found in techniques for the production of single-stranded units (U.S. Pat. No. 4,694,778; Bird, Science 242:423-442 (1988), Huston et al., Proc. Natl. Acad. Sci. USA 55:5879-5883 (1988) and Ward et al., Nature 334:544-554 (1989) and Nie et al., Antibody Therapeutics 3 (1):18-62 (2020) ). Single-chain units are formed by linking the heavy and light chain fragments of the Fv region via an amino acid bridge, resulting in a single-chain fusion peptide. Techniques for the assembly of functional Fv fragments in E. *coli* may also be used (Skerra et al., Science 242: 1038-1041 (1988)).

Examples of techniques that can be used to produce a single chain Fv (scFv) and an antibody include, for example, those described in U.S. Pat. Nos. 4,946,778 and 5,258,498, and Huston et al., Methods in Enzymology, 203:46-88 (1991), Shu et al., Proc. Natl. Sci. USA, 90:1995-1999 (1993) and Skerra et al., Science, 240:1038-1040 (1988). For certain use, including *in vivo* use of antibodies in humans and *in vitro* detection assays, a chimeric antibody, a humanized antibody, or a fully human antibody may be used. Chimeric antibodies are molecules in which different portions of the antibody are derived from different animal species, such as antibodies having variable regions of a murine monoclonal antibody and constant regions of a human immunoglobulin. Methods for producing chimeric antibodies are known in the art, see Morrison, Science 229:1202 (1985); Oi et al., BioTechniques 4:214 (1986); Gillies et al., J. Immunol. Methods 125:191-202 (1989); Neuberger et al., Nature 372:604-608 (1984); Takeda et al., Nature 314:452-454 (1985); and U.S. Pat. Nos. 5,807,715, 4,816,567 and 4,816,397, which are incorporated herein by reference in their entireties.

### BRIEF DESCRIPTION OF THE DRAWINGS

FIG. 1 shows the affinity assay of anti-Nectin-4 antibodies.
FIG. 2 shows the binding activity of anti-Nectin-4 antibodies to T-47D cells.
FIG. 3 shows the specificity assay results of anti-Nectin-4 antibodies for human Nectin-4 homologous proteins.
FIG. 4 shows the binding of anti-Nectin-4 antibodies to Nectin-4 of different species.

### DETAILED DESCRIPTION

The present disclosure will be specifically described below by way of examples. It will be appreciated by those skilled in the art that the following examples are illustrative of the present disclosure only and should not be interpreted as limiting the scope of the present disclosure. For the examples with no specified techniques or conditions, procedures were conducted according to the techniques or conditions described in the literature of the art (for example, refer to Sambrook. J. et al, Molecular Cloning: A Laboratory Manual, translated by Huang Pentang et al, 3rd edition, Science Press) or according to instructions of the products. Reagents or instruments with no specified manufacturer are conventional, commercially available products.

### Example 1: Expression of human Nectin-4 extracellular antigen

The human Nectin-4 extracellular antigen (hNectin-4-His) was prepared according to conventional methods: the signal peptide sequence of the human Nectin-4 extracellular sequence (sequence source: GenBank No. Q96NY8) was replaced with an albumin signal peptide (MKWVTFISLLFLFSSAYS, as set forth in SEQ ID NO: 1), and a histidine tag 8×His was added to the C-terminus of the albumin signal peptide to give the antigen protein (the sequence is set forth in SEQ ID NO: 2, and denoted as hNectin-4-His). The DNA sequence of the antigen protein hNectin-4-His was cloned to an expression vector. Eukaryotic cells, i.e., HEK293F cells, were transiently transfected, or CHO cells were stably transfected. Stable cell lines were selected, and purification and expression were performed.

MKWVTFISLLFLFSSAYSGELETSDVVTVVLGQDAKLPCFYRGDSGEQVGQVAWARV
DAGEGAQELALLHSKYGLHVSPAYEGRVEQPPPPRNPLDGSVLLRNAVQADEGEYEC
RVSTFPAGSFQARLRLRVLVPPLPSLNPGPALEEGQGLTLAASCTAEGSPAPSVTWDTE
VKGTTSSRSFKHSRSAAVTSEFHLVPSRSMNGQPLTCVVSHPGLLQDQRITHILHVSFL
AEASVRGLEDQNLWHIGREGAMLKCLSEGQPPPSYNWTRLDGPLPSGVRVDGDTLG
FPPLTTEHSGIYVCHVSNEFSSRDSQVTVDVLDPQEDSGKQVDLVSASHHHHHHHH
(SEQ ID NO: 2, with the albumin signal peptide underlined).

### Example 2: Expression of reference antibody

The amino acid sequences of the heavy and light chains of the reference antibody ASG-22 are shown in Table 1. For the convenience of expression in a host cell, signal peptides were added to N-termini of the heavy and light chains, where the signal peptide sequence linked to the N-terminus of the heavy chain was MELGLCWVFLVAILEGVQC (as set forth in SEQ ID NO: 3) and the signal peptide sequence linked to the N-terminus of the light chain was MDMRVPAQLLGLLLLWFPGSRC (as set forth in SEQ ID NO: 4). DNA sequences of the heavy and light chains were synthesized by conventional methods, and the antibody normally bound to hNectin-4-His expressed in Example 1.

**Table 1. Amino acid sequences of reference antibody ASG-22**

| | Amino acid sequence (with CDR sequences underlined, according to the Kabat numbering system) | SEQ ID NO: |
|---|---|---|
| Heavy chain | EVQLVESGGGLVQPGGSLRLSCAASGFTFSSYNMNWVRQAP GKGLEWVSYISSSSSTIYYADSVKGRFTISRDNAKNSLSLQM NSLRDEDTAVYYCARAYYYGMDVWGQGTLVTVSSASTKGP SVFPLAPSSKSTSGGTAALGCLVKDYFPEPVTVSWNSGALTS GVHTFPAVLQSSGLYSLSSVVTVPSSSLGTQTYICNVNHKPSN TKVDKKVEPKSCDKTHTCPPCPAPELLGGPSVFLFPPKPKDT LMISRTPEVTCVVVDVSHEDPEVKFNWYVDGVEVHNAKTK PREEQYNSTYRVVSVLTVLHQDWLNGKEYKCKVSNKALPA PIEKTISKAKGQPREPQVYTLPPSRDELTKNQVSLTCLVKGFY PSDIAVEWESNGQPENNYKTTPPVLDSDGSFFLYSKLTVDKS RWQQGNVFSCSVMHEALHNHYTQKSLSLSPGK | 5 |
| Light chain | DIQMTQSPSSVSASVGDRVTITCRASQGISGWLAWYQQKPG KAPKFLIYAASTLQSGVPSRFSGSGSGTDFTLTISSLQPEDFAT YYCQQANSFPPTFGGGTKVEIKRTVAAPSVFIFPPSDEQLKSG TASVVCLLNNFYPREAKVQWKVDNALQSGNSQESVTEQDS KDSTYSLSSTLTLSKADYEKHKVYACEVTHQGLSSPVTKSFN RGEC | 6 |

### Example 3: Preparation of anti-Nectin-4 antibodies

### 1) Antibody sequences

Table 2 shows the light and heavy chain variable regions of scFv, where the light chain variable regions have identical amino acid sequences.

**Table 2: Sequences of heavy chain variable region and light chain variable region**

| Clone No. | Type | Amino acid sequence (with CDR sequences underlined, according to the Kabat numbering system) |
|---|---|---|
| 1B4 | VH | EVQLVESGGGLVQPGGSLRLSCAASGFTFSSYAMSWVRQAP GKGLEWVSAISGSGGSTYYADSVKGRFTISRDNSKNTLYLQM NSLRAEDTAVYYCARQTEDRYYGYDLDYWGQGTLVTVSS (SEQ ID NO:7) |
| | | HCDR1: SYAMS (SEQ ID NO:8) |
| | | HCDR2: AISGSGGSTYYADSVKG (SEQ ID NO:9) |
| | | HCDR3: QTEDRYYGYDLDY (SEQ ID NO:10) |
| 1G8 | VH | EVQLVESGGGLVQPGGSLRLSCAASGFTFSSYAMSWVRQAP GKGLEWVSAISGSGGSTYYADSVKGRFTISRDNSKNTLYLQM NSLRAEDTAVYYCARQTEDRSYGYDLDYWGQGTLVTVSS (SEQ ID NO:11) |
| | | HCDR1: SYAMS (SEQ ID NO:8) |
| | | HCDR2: AISGSGGSTYYADSVKG (SEQ ID NO:9) |
| | | HCDR3: QTEDRSYGYDLDY (SEQ ID NO:12) |
| 1F3 | VH | EVQLVESGGGLVQPGGSLRLSCAASGFTFSSYAMSWVRQAP GKGLEWVSAISGSGGSTYYADSVKGRFTISRDNSKNTLYLQM NSLRAEDTAVYYCARAELLAFDVYTASDYWGQGTLVTVSS (SEQ ID NO:13) |
| | | HCDR1: SYAMS (SEQ ID NO:8) |
| | | HCDR2: AISGSGGSTYYADSVKG (SEQ ID NO:9) |
| | | HCDR3: AELLAFDVYTASDY (SEQ ID NO:14) |
| 10F4 | VH | EVQLVESGGGLVQPGGSLRLSCAASGFTFSSYAMSWVRQAP GKGLEWVSAISGSGGSTYYADSVKGRFTISRDNSKNTLYLQM NSLRAEDTAVYYCAREYSDVVDYVSVRTPLDYWGQGTLVT VSS (SEQ ID NO:15) |
| | | HCDR1: SYAMS (SEQ ID NO:8) |
| | | HCDR2: AISGSGGSTYYADSVKG (SEQ ID NO:9) |
| | | HCDR3: EYSDVVDYVSVRTPLDY (SEQ ID NO:16) |
| 3A10 | VH | EVQLVESGGGLVQPGGSLRLSCAASGFTFSSYAMSWVRQAP GKGLEWVSAISGSGGSTYYADSVKGRFTISRDNSKNTLYLQM NSLRAEDTAVYYCARDRSDVYSYSYFPTPLDYWGQGTLVTV SS (SEQ ID NO:17) |
| | | HCDR1: SYAMS (SEQ ID NO:8) |
| | | HCDR2: AISGSGGSTYYADSVKG (SEQ ID NO:9) |
| | | HCDR3: DRSDVYSYSYFPTPLDY (SEQ ID NO:18) |
| 10A4 | VH | EVQLVESGGGLVQPGGSLRLSCAASGFTFSSYAMSWVRQAP GKGLEWVSAISGSGGSTYYADSVKGRFTISRDNSKNTLYLQM NSLRAEDTAVYYCARQTEDRSYGYDMDYWGQGTLVTVSS (SEQ ID NO:19) |
| | | HCDR1: SYAMS (SEQ ID NO:8) |
| | | HCDR2: AISGSGGSTYYADSVKG (SEQ ID NO:9) |
| | | HCDR3: QTEDRSYGYDMDY (SEQ ID NO:20) |
| 1G7 | VH | EVQLVESGGGLVQPGGSLRLSCAASGFTFSSYAMSWVRQAP GKGLEWVSAISGSGGSTYYADSVKGRFTISRDNSKNTLYLQM NSLRAEDTAVYYCARERSDATYLRTPLDYWGQGTLVTVSS (SEQ ID NO:21) |
| | | HCDR1: SYAMS (SEQ ID NO:8) |
| | | HCDR2: AISGSGGSTYYADSVKG (SEQ ID NO:9) |
| | | HCDR3: ERSDATYLRTPLDY (SEQ ID NO:22) |
| 1G12 | VH | EVQLVESGGGLVQPGGSLRLSCAASGFTFSSYAMSWVRQAP GKGLEWVSAISGSGGSTYYADSVKGRFTISRDNSKNTLYLQM NSLRAEDTAVYYCARHTEDRYYGYYLDYWGQGTLVTVSS (SEQ ID NO:23) |
| | | HCDR1: SYAMS (SEQ ID NO:8) |
| | | HCDR2: AISGSGGSTYYADSVKG (SEQ ID NO:9) |
| | | HCDR3: HTEDRYYGYYLDY (SEQ ID NO:24) |
| Common light chain variable region | VL | DIQMTQSPSSLSASVGDRVTITCRASQGISSYLAWYQQKPGK APKLLIYAASSLQSGVPSRFSGSGSGTDFTLTISSLQPEDFATY YCQQHYTTPPTFGQGTKVEIKR (SEQ ID NO:25) |
| | | LCDR1: RASQGISSYLA (SEQ ID NO:26) |
| | | LCDR2: AASSLQS (SEQ ID NO:27) |
| | | LCDR3: QQHYTTPPT (SEQ ID NO:28) |

The sequences of the framework regions HFR1-HFR4 in the VH are set forth in SEQ ID NOs: 29-32, and the sequences of the framework regions LFR1-LFR4 in the VL are set forth in SEQ ID NOs: 33-36.

### Framework regions in VH:

HFR1: EVQLVESGGGLVQPGGSLRLSCAASGFTFS (SEQ ID NO:29)
HFR2: WVRQAPGKGLEWVS (SEQ ID NO:30)
HFR3: RFTISRDNSKNTLYLQMNSLRAEDTAVYYCAR (SEQ ID NO:31)
HFR4: WGQGTLVTVSS (SEQ ID NO:32)

### Framework regions in VL:

LFR1: DIQMTQSPSSLSASVGDRVTITC (SEQ ID NO:33)
LFR2: WYQQKPGKAPKLLIY (SEQ ID NO:34)
LFR3: GVPSRFSGSGSGTDFTLTISSLQPEDFATYYC (SEQ ID NO:35)
LFR4: FGQGTKVEIKR (SEQ ID NO:36)

### 2) Preparation of scFv

The HCDR3 of the above 1F3, 1G8, and 10F4 were cloned into scFv phage libraries of HCDR1 and HCDR2, and supernatants of phage clones were screened and selected for detection. The supernatants were diluted 50-fold, and the binding OD values of the diluted supernatants to hNectin-4-His were measured by ELISA. The samples were serially 3-fold diluted, and the EC₅₀ titer for the binding of the supernatants to the antigen was measured by ELISA. Clones with higher OD values were selected for sequencing to give the heavy chain variable region sequences and the HCDR1 and HCDR2 sequences of all the clones, as shown in Tables 3-8. The light chain variable region sequence is set forth in SEQ ID NO: 25. The OD value is the measurement of ELISA, and the EC₅₀ value denotes the EC₅₀ value of the binding activity titer of the supernatant to the antigen.

### 3) Preparation of antibody

Construction of antibody: the heavy chain variable regions shown in Tables 2-5 and a heavy chain constant regions (CH) were combined to give the heavy chains of the antibodies, and the light chain variable region set forth in SEQ ID NO: 25 and a light chain constant region (CL) were combined to give the light chain of the antibodies; the amino acid sequence of CH is set forth in SEQ ID NO: 37, and the amino acid sequence of CL is set forth in SEQ ID NO: 38. For the convenience of expression in a host cell, signal peptides were added to N-termini of the heavy and light chains, where the signal peptide sequence linked to the N-terminus of the heavy chain was MEWSWVFLFFLSVTTGVHS (as set forth in SEQ ID NO: 39) and the signal peptide sequence linked to the N-terminus of the light chain was MDMRVPAQLLGLLLLWLPGARC (as set forth in SEQ ID NO: 40). The antibody numbers are consistent with the clone numbers corresponding to the heavy chain variable regions. The amino acid sequence of the light chain is set forth in SEQ ID NO: 250, and the nucleic acid sequence of the light chain is set forth in SEQ ID NO: 359. The nucleic acid sequence was synthesized according to the antibody sequences. For example, for antibody 1F3, the amino acid sequence of the heavy chain is set forth in SEQ ID NO: 232; for antibody 1G8, the amino acid sequence of the heavy chain is set forth in SEQ ID NO: 233; for antibody 10F4, the amino acid sequence of the heavy chain is set forth in SEQ ID NO: 249; for antibody 10F4-3, the amino acid sequence of the heavy chain is set forth in SEQ ID NO: 360, and the nucleic acid sequence of the heavy chain is set forth in SEQ ID NO: 364; for antibody 1F3-1E4, the amino acid sequence of the heavy chain is set forth in SEQ ID NO: 361, and the nucleic acid sequence of the heavy chain is set forth in SEQ ID NO: 365; for antibody 1F3-2B9, the amino acid sequence of the heavy chain is set forth in SEQ ID NO: 362, and the nucleic acid sequence of the heavy chain is set forth in SEQ ID NO: 366; for antibody 1G8-1C10, the amino acid sequence of the heavy chain is set forth in SEQ ID NO: 363, and the nucleic acid sequence of the heavy chain is set forth in SEQ ID NO: 367. The light and heavy chain expression vectors were constructed according to conventional operation methods. HEK293F cells were transformed, and the acquired antibodies were subjected to further detection.

**Table 3: Heavy chain variable region sequences of 1F3-series antibodies**

| Clone No. | VH sequences (with CDR sequences underlined, according to the Kabat numbering system) | OD value | EC₅₀ |
|---|---|---|---|
| 1F3 | EVQLVESGGGLVQPGGSLRLSCAASGFTFSSYAMSWVRQAPGK GLEWVSAISGSGGSTYYADSVKGRFTISRDNSKNTLYLQMNSL RAEDTAVYYCARAELLAFDVYTASDYWGQGTLVTVSS (SEQ ID NO:13) | NA | NA |
| 1F3-1A9 | EVQLVESGGGLVQPGGSLRLSCAASGYSFDNYAMSWVRQAPG KGLEWVSRISPTGGYASYADSVKGRFTISRDNSKNTLYLQMNSL RAEDTAVYYCARAELLAFDVYTASDYWGQGTLVTVSS (SEQ ID NO:41) | 1.635 | 309.2 |
| 1F3-1B1 | EVQLVESGGGLVQPGGSLRLSCAASGFTFGTYGMSWVRQAPG KGLEWVSGIKPGDSTTYYADSVKGRFTISRDNSKNTLYLQMNS LRAEDTAVYYCARAELLAFDVYTASDYWGQGTLVTVSS (SEQ ID NO:42) | 1.651 | 195.1 |
| 1F3-1C11 | EVQLVESGGGLVQPGGSLRLSCAASGFNFSSYGMSWVRQAPG KGLEWVSAISGSGGSTYYADSVKGRFTISRDNSKNTLYLQMNS LRAEDTAVYYCARAELLAFDVYTASDYWGQGTLVTVSS (SEQ ID NO:43) | 1.633 | 78.51 |
| 1F3-1C4 | EVQLVESGGGLVQPGGSLRLSCAASGFTFGDYGMSWVRQAPG KGLEWVSGIKPWGSGTYYADSVKGRFTISRDNSKNTLYLQMNS LRAEDTAVYYCARAELLAFDVYTASDYWGQGTLVTVSS (SEQ ID NO:44) | 1.591 | 238.3 |
| 1F3-1D1 | EVQLVESGGGLVQPGGSLRLSCAASGDIFTNYSMSWVRQAPGK GLEWVSGIYPSGDNNRYADSVKGRFTISRDNSKNTLYLQMNSL RAEDTAVYYCARAELLAFDVYTASDYWGQGTLVTVSS (SEQ ID NO:45) | 1.287 | 78.33 |
| 1F3-1D12 | EVQLVESGGGLVQPGGSLRLSCAASGYNFTDYGMSWVRQAPG KGLEWVSGISGSGDNKYYADSVKGRFTISRDNSKNTLYLQMNS LRAEDTAVYYCARAELLAFDVYTASDYWGQGTLVTVSS (SEQ ID NO:46) | 1.633 | 323.6 |
| 1F3-1D6 | EVQLVESGGGLVQPGGSLRLSCAASGGSFYGFGMSWVRQAPG KGLEWVSGIYSHDADKRYADSVKGRFTISRDNSKNTLYLQMNS LRAEDTAVYYCARAELLAFDVYTASDYWGQGTLVTVSS (SEQ ID NO:47) | 1.441 | 97.88 |
| 1F3-1E1 | EVQLVESGGGLVQPGGSLRLSCAASGFSFDDYAMSWVRQAPG KGLEWVSIIYPTGSTNYYADSVKGRFTISRDNSKNTLYLQMNSL RAEDTAVYYCARAELLAFDVYTASDYWGQGTLVTVSS (SEQ ID NO:48) | 1.643 | 214.1 |
| 1F3-1E10 | EVQLVESGGGLVQPGGSLRLSCAASGFNFSSYGMSWVRQAPG KGLEWVSRISPYTDNKYYADSVKGRFTISRDNSKNTLYLQMNS LRAEDTAVYYCARAELLAFDVYTASDYWGQGTLVTVSS (SEQ ID NO:49) | 1.545 | 187.9 |
| 1F3-1E4 | EVQLVESGGGLVQPGGSLRLSCAASGFSFGTYGMSWVRQAPG KGLEWVSRISGDSANIRYADSVKGRFTISRDNSKNTLYLQMNSL RAEDTAVYYCARAELLAFDVYTASDYWGQGTLVTVSS (SEQ ID NO:50) | 1.615 | 386.4 |
| 1F3-1E9 | EVQLVESGGGLVQPGGSLRLSCAASGFTFDDYAMSWVRQAPG KGLEWVSGIKPSSWTTYYADSVKGRFTISRDNSKNTLYLQMNS LRAEDTAVYYCARAELLAFDVYTASDYWGQGTLVTVSS (SEQ ID NO:51) | 1.555 | 192 |
| 1F3-1F4 | EVQLVESGGGLVQPGGSLRLSCAASGDIFTGSSMSWVRQAPGK GLEWVSGIYASGSDKRYADSVKGRFTISRDNSKNTLYLQMNSL RAEDTAVYYCARAELLAFDVYTASDYWGQGTLVTVSS (SEQ ID NO:52) | 1.421 | 147.1 |
| 1F3-1F8 | EVQLVESGGGLVQPGGSLRLSCAASGFNFGNYGMSWVRQAPG KGLEWVSRISGDGSNINYADSVKGRFTISRDNSKNTLYLQMNSL RAEDTAVYYCARAELLAFDVYTASDYWGQGTLVTVSS (SEQ ID NO:53) | 1.671 | 677 |
| 1F3-1G11 | EVQLVESGGGLVQPGGSLRLSCAASGYSFTGYGMSWVRQAPG KGLEWVSGISPSGGTIGYADSVKGRFTISRDNSKNTLYLQMNSL RAEDTAVYYCARAELLAFDVYTASDYWGQGTLVTVSS (SEQ ID NO:54) | 1.652 | 417.3 |
| 1F3-1G2 | EVQLVESGGGLVQPGGSLRLSCAASGYTFGDYGMSWVRQAPG KGLEWVSRIDPWGASIRYADSVKGRFTISRDNSKNTLYLQMNS LRAEDTAVYYCARAELLAFDVYTASDYWGQGTLVTVSS (SEQ ID NO:55) | 1.598 | 164.2 |
| 1F3-1G5 | EVQLVESGGGLVQPGGSLRLSCAASGFSFGSYGMSWVRQAPG KGLEWVSGIKPSTSTTYYADSVKGRFTISRDNSKNTLYLQMNSL RAEDTAVYYCARAELLAFDVYTASDYWGQGTLVTVSS (SEQ ID NO:56) | 1.565 | 122.6 |
| 1F3-1G7 | EVQLVESGGGLVQPGGSLRLSCAASGDIFSNYSMSWVRQAPGK GLEWVSGIYPSGDSRRYADSVKGRFTISRDNSKNTLYLQMNSL RAEDTAVYYCARAELLAFDVYTASDYWGQGTLVTVSS (SEQ ID NO:57) | 1.56 | 198.3 |
| 1F3-1H11 | EVQLVESGGGLVQPGGSLRLSCAASGFNFTDYGMSWVRQAPG KGLEWVSRISQWGDTSNYADSVKGRFTISRDNSKNTLYLQMNS LRAEDTAVYYCARAELLAFDVYTASDYWGQGTLVTVSS (SEQ ID NO:58) | 1.65 | 162 |
| 1F3-1H3 | EVQLVESGGGLVQPGGSLRLSCAASGFNFYNYGMSWVRQAPG KGLEWVSGIKPHSDVAHYADSVKGRFTISRDNSKNTLYLQMNS LRAEDTAVYYCARAELLAFDVYTASDYWGQGTLVTVSS (SEQ ID NO:59) | 1.725 | 313.6 |
| 1F3-2A11 | EVQLVESGGGLVQPGGSLRLSCAASGDNFYTFYMSWVRQAPG KGLEWVSGIKPTGSYINYADSVKGRFTISRDNSKNTLYLQMNSL RAEDTAVYYCARAELLAFDVYTASDYWGQGTLVTVSS (SEQ ID NO:60) | 1.525 | 108.6 |
| 1F3-2A12 | EVQLVESGGGLVQPGGSLRLSCAASGYSFDSYGMSWVRQAPG KGLEWVSGIKPTGASTYYADSVKGRFTISRDNSKNTLYLQMNS LRAEDTAVYYCARAELLAFDVYTASDYWGQGTLVTVSS (SEQ ID NO:61) | 1.646 | 264.7 |
| 1F3-2B1 | EVQLVESGGGLVQPGGSLRLSCAASGYNFDNYAMSWVRQAPG KGLEWVSGIKPDSATTYYADSVKGRFTISRDNSKNTLYLQMNS LRAEDTAVYYCARAELLAFDVYTASDYWGQGTLVTVSS (SEQ ID NO:62) | 1.617 | 268.7 |
| 1F3-2B2 | EVQLVESGGGLVQPGGSLRLSCAASGYNFYSYGMSWVRQAPG KGLEWVSIISPDGGYKRYADSVKGRFTISRDNSKNTLYLQMNSL RAEDTAVYYCARAELLAFDVYTASDYWGQGTLVTVSS (SEQ ID NO:63) | 1.574 | 171.7 |
| 1F3-2B2-2 | EVQLVESGGGLVQPGGSLRLSCAASGYSFSDFGMSWVRQAPG KGLEWVSRIDPSGWSTSYADSVKGRFTISRDNSKNTLYLQMNS LRAEDTAVYYCARAELLAFDVYTASDYWGQGTLVTVSS (SEQ ID NO:64) | 1.593 | 217 |
| 1F3-2B9 | EVQLVESGGGLVQPGGSLRLSCAASGFNFDSYAMSWVRQAPG KGLEWVSGIKPHTDDIYYADSVKGRFTISRDNSKNTLYLQMNS LRAEDTAVYYCARAELLAFDVYTASDYWGQGTLVTVSS (SEQ ID NO:65) | 1.568 | 289 |
| 1F3-2C6 | EVQLVESGGGLVQPGGSLRLSCAASGDIFTNYSMSWVRQAPGK GLEWVSGIYASGSDKRYADSVKGRFTISRDNSKNTLYLQMNSL RAEDTAVYYCARAELLAFDVYTASDYWGQGTLVTVSS (SEQ ID NO:66) | 1.382 | 65.37 |
| 1F3-2C9 | EVQLVESGGGLVQPGGSLRLSCAASGFNFGGYGMSWVRQAPG KGLEWVSRISPGDSTPRYADSVKGRFTISRDNSKNTLYLQMNSL RAEDTAVYYCARAELLAFDVYTASDYWGQGTLVTVSS (SEQ ID NO:67) | 1.586 | 191.4 |
| IF3-2D2 | EVQLVESGGGLVQPGGSLRLSCAASGFNFSSYGMSWVRQAPG KGLEWVSRISPYTGTTHYADSVKGRFTISRDNSKNTLYLQMNS LRAEDTAVYYCARAELLAFDVYTASDYWGQGTLVTVSS (SEQ ID NO:68) | 1.589 | 180.9 |
| 1F3-2D8 | EVQLVESGGGLVQPGGSLRLSCAASGYSFSDYGMSWVRQAPG KGLEWVSRISPSKAVKDYADSVKGRFTISRDNSKNTLYLQMNS LRAEDTAVYYCARAELLAFDVYTASDYWGQGTLVTVSS (SEQ ID NO:69) | 1.56 | 155.8 |
| 1F3-2E12 | EVQLVESGGGLVQPGGSLRLSCAASGGNFTNSGMSWVRQAPG KGLEWVSGIKWSSDETYYADSVKGRFTISRDNSKNTLYLQMNS LRAEDTAVYYCARAELLAFDVYTASDYWGQGTLVTVSS (SEQ ID NO:70) | 1.519 | 95.06 |
| 1F3-2E8 | EVQLVESGGGLVQPGGSLRLSCAASGYSFSDYGMSWVRQAPG KGLEWVSWISPSKGSASYADSVKGRFTISRDNSKNTLYLQMNS LRAEDTAVYYCARAELLAFDVYTASDYWGQGTLVTVSS (SEQ ID NO:71) | 1.556 | 209.9 |
| 1F3-2F1 | EVQLVESGGGLVQPGGSLRLSCAASGDIFTGYSMSWVRQAPGK GLEWVSGIYQTGSNTRYADSVKGRFTISRDNSKNTLYLQMNSL RAEDTAVYYCARAELLAFDVYTASDYWGQGTLVTVSS (SEQ ID NO:72) | 1.025 | NA |
| 1F3-2F3 | EVQLVESGGGLVQPGGSLRLSCAASGFNFSDYGMSWVRQAPG KGLEWVSRIDWDGGNTRYADSVKGRFTISRDNSKNTLYLQMN SLRAEDTAVYYCARAELLAFDVYTASDYWGQGTLVTVSS (SEQ ID NO:73) | 1.567 | 325.3 |
| 1F3-2F6 | EVQLVESGGGLVQPGGSLRLSCAASGFTFSDYGMSWVRQAPG KGLEWVSRISQYGGGIRYADSVKGRFTISRDNSKNTLYLQMNS LRAEDTAVYYCARAELLAFDVYTASDYWGQGTLVTVSS (SEQ ID NO:74) | 1.519 | 129.4 |
| 1F3-2G10 | EVQLVESGGGLVQPGGSLRLSCAASGFNFSNYGMSWVRQAPG KGLEWVSRIKPTDGSTHYADSVKGRFTISRDNSKNTLYLQMNS LRAEDTAVYYCARAELLAFDVYTASDYWGQGTLVTVSS (SEQ ID NO:75) | 1.54 | 120.2 |
| 1F3-2G9 | EVQLVESGGGLVQPGGSLRLSCAASGFTFGGYGMSWVRQAPG KGLEWVSWISAWDGSIYYADSVKGRFTISRDNSKNTLYLQMNS LRAEDTAVYYCARAELLAFDVYTASDYWGQGTLVTVSS (SEQ ID NO:76) | 1.38 | 49.33 |
| 1F3-2H12 | EVQLVESGGGLVQPGGSLRLSCAASGDSFDSSGMSWVRQAPG KGLEWVSGIKWITGESYYADSVKGRFTISRDNSKNTLYLQMNS LRAEDTAVYYCARAELLAFDVYTASDYWGQGTLVTVSS (SEQ ID NO:77) | 1.59 | 130.5 |
| IF3-2H6 | EVQLVESGGGLVQPGGSLRLSCAASGYTFSSYGMSWVRQAPG KGLEWVSRIKSDGGDTHYADSVKGRFTISRDNSKNTLYLQMNS LRAEDTAVYYCARAELLAFDVYTASDYWGQGTLVTVSS (SEQ ID NO:78) | 1.311 | 53.49 |

**Table 4: Heavy chain variable region sequences of 1G8-series antibodies**

| Clone No. | VH sequences (with CDR sequences underlined, according to the Kabat numbering system) | OD value | EC₅₀ |
|---|---|---|---|
| 1G8 | EVQLVESGGGLVQPGGSLRLSCAASGFTFSSYAMSWVRQAPGK GLEWVSAISGSGGSTYYADSVKGRFTISRDNSKNTLYLQMNSL RAEDTAVYYCARQTEDRSYGYDLDYWGQGTLVTVSS(SEQ ID NO:11) | NA | NA |
| 1G8-1A12 | EVQLVESGGGLVQPGGSLRLSCAASGGTFDTNAMSWVRQAPG KGLEWVSAISTGGDNKRYADSVKGRFTISRDNSKNTLYLQMNS LRAEDTAVYYCARQTEDRSYGYDLDYWGQGTLVTVSS(SEQ ID NO:79) | 1.612 | 518.8 |
| 1G8-1A3 | EVQLVESGGGLVQPGGSLRLSCAASGYNFGSYAMSWVRQAPG KGLEWVSRIYPGSWSPDYADSVKGRFTISRDNSKNTLYLQMNS LRAEDTAVYYCARQTEDRSYGYDLDYWGQGTLVTVSS(SEQ ID NO:80) | 1.625 | 316.4 |
| 1G8-1A7 | EVQLVESGGGLVQPGGSLRLSCAASGYTFDSYAMSWVRQAPG KGLEWVSRIKTTDDGKSYADSVKGRFTISRDNSKNTLYLQMNS LRAEDTAVYYCARQTEDRSYGYDLDYWGQGTLVTVSS(SEQ ID NO:81) | 1.573 | 307.6 |
| 1G8-1B11 | EVQLVESGGGLVQPGGSLRLSCAASGYNFSNYAMSWVRQAPG KGLEWVSAIDASGGDTRYADSVKGRFTISRDNSKNTLYLQMNS LRAEDTAVYYCARQTEDRSYGYDLDYWGQGTLVTVSS(SEQ ID NO:82) | 1.582 | 597.6 |
| 1G8-1B7 | EVQLVESGGGLVQPGGSLRLSCAASGYSFSNYAMSWVRQAPG KGLEWVSRISAWGSTNNYADSVKGRFTISRDNSKNTLYLQMNS LRAEDTAVYYCARQTEDRSYGYDLDYWGQGTLVTVSS(SEQ ID NO:83) | 1.558 | 295.9 |
| 1G8-1C1 | EVQLVESGGGLVQPGGSLRLSCAASGYIFSNYAMSWVRQAPGK GLEWVSRISTGFAGPDYADSVKGRFTISRDNSKNTLYLQMNSL RAEDTAVYYCARQTEDRSYGYDLDYWGQGTLVTVSS(SEQ ID NO:84) | 1.502 | 294.5 |
| 1G8-1C10 | EVQLVESGGGLVQPGGSLRLSCAASGFNFSDYSMSWVRQAPG KGLEWVSRIKASSGGSDYADSVKGRFTISRDNSKNTLYLQMNS LRAEDTAVYYCARQTEDRSYGYDLDYWGQGTLVTVSS(SEQ ID NO:85) | 1.606 | 618.1 |
| 1G8-1C12 | EVQLVESGGGLVQPGGSLRLSCAASGGSFYGFGMSWVRQAPG KGLEWVSGIYSHDADKRYADSVKGRFTISRDNSKNTLYLQMNS LRAEDTAVYYCARQTEDRSYGYDLDYWGQGTLVTVSS(SEQ ID NO:86) | 1.415 | 24.26 |
| 1G8-1C8 | EVQLVESGGGLVQPGGSLRLSCAASGFSFGSSAMSWVRQAPGK GLEWVSAISGSSDSPRYADSVKGRFTISRDNSKNTLYLQMNSLR AEDTAVYYCARQTEDRSYGYDLDYWGQGTLVTVSS(SEQ ID NO:87) | 1.591 | 601.6 |
| 1G8-1D11 | EVQLVESGGGLVQPGGSLRLSCAASGDIFTGYAMSWVRQAPG KGLEWVSRIDGTGDSKFYADSVKGRFTISRDNSKNTLYLQMNS LRAEDTAVYYCARQTEDRSYGYDLDYWGQGTLVTVSS(SEQ ID NO:88) | 1.591 | 326.2 |
| 1G8-1D7 | EVQLVESGGGLVQPGGSLRLSCAASGGSFSNYAMSWVRQAPG KGLEWVSWIDTTSAYASYADSVKGRFTISRDNSKNTLYLQMNS LRAEDTAVYYCARQTEDRSYGYDLDYWGQGTLVTVSS(SEQ ID NO:89) | 1.491 | 190.4 |
| 1G8-1E10 | EVQLVESGGGLVQPGGSLRLSCAASGDSFSTYAMSWVRQAPG KGLEWVSRISGSGDGKDYADSVKGRFTISRDNSKNTLYLQMNS LRAEDTAVYYCARQTEDRSYGYDLDYWGQGTLVTVSS(SEQ ID NO:90) | 1.602 | 512.5 |
| 1G8-1E12 | EVQLVESGGGLVQPGGSLRLSCAASGFNFTSYAMSWVRQAPG KGLEWVSRISAHKWDASYADSVKGRFTISRDNSKNTLYLQMN SLRAEDTAVYYCARQTEDRSYGYDLDYWGQGTLVTVSS(SEQ ID NO:91) | 1.613 | 272.3 |
| 1G8-1E6 | EVQLVESGGGLVQPGGSLRLSCAASGYIFTDYSMSWVRQAPGK GLEWVSRIYTDGWEKDYADSVKGRFTISRDNSKNTLYLQMNS LRAEDTAVYYCARQTEDRSYGYDLDYWGQGTLVTVSS(SEQ ID NO:92) | 1.481 | 225 |
| 1G8-1E8 | EVQLVESGGGLVQPGGSLRLSCAASGFTFDGYAMSWVRQAPG KGLEWVSRISSYSWNPDYADSVKGRFTISRDNSKNTLYLQMNS LRAEDTAVYYCARQTEDRSYGYDLDYWGQGTLVTVSS(SEQ ID NO:93) | 1.546 | 303.5 |
| 1G8-1F11 | EVQLVESGGGLVQPGGSLRLSCAASGDTFSSYAMSWVRQAPG KGLEWVSRISQTGGTTDYADSVKGRFTISRDNSKNTLYLQMNS LRAEDTAVYYCARQTEDRSYGYDLDYWGQGTLVTVSS(SEQ ID NO:94) | 1.601 | 580.7 |
| 1G8-1F5 | EVQLVESGGGLVQPGGSLRLSCAASGDIFDTYMSWVRQAPGK GLEWVSRIYTDGSSDYADSVKGRFTISRDNSKNTLYLQMNSLR AEDTAVYYCARQTEDRSYGYDLDYWGQGTLVTVSS(SEQ ID NO:95) | 1.564 | 551.9 |
| 1G8-1F7 | EVQLVESGGGLVQPGGSLRLSCAASGYSFYGYAMSWVRQAPG KGLEWVSAIDAIGGNKRYADSVKGRFTISRDNSKNTLYLQMNS LRAEDTAVYYCARQTEDRSYGYDLDYWGQGTLVTVSS(SEQ ID NO:96) | 1.576 | 423 |
| 1G8-1G12 | EVQLVESGGGLVQPGGSLRLSCAASGYNFTSSAMSWVRQAPG KGLEWVSRIYSISGNTDYADSVKGRFTISRDNSKNTLYLQMNSL RAEDTAVYYCARQTEDRSYGYDLDYWGQGTLVTVSS(SEQ ID NO:97) | 1.546 | 204.2 |
| 1G8-1G3 | EVQLVESGGGLVQPGGSLRLSCAASGFNFGTYAMSWVRQAPG KGLEWVSRISGISAGTNYADSVKGRFTISRDNSKNTLYLQMNSL RAEDTAVYYCARQTEDRSYGYDLDYWGQGTLVTVSS(SEQ ID NO:98) | 1.581 | 355.4 |
| 1G8-1G5 | EVQLVESGGGLVQPGGSLRLSCAASGYNFTTSAMSWVRQAPG KGLEWVSRIYADTAYNDYADSVKGRFTISRDNSKNTLYLQMNS LRAEDTAVYYCARQTEDRSYGYDLDYWGQGTLVTVSS(SEQ ID NO:99) | 1.482 | 170.1 |
| 1G8-1G9 | EVQLVESGGGLVQPGGSLRLSCAASGFNFGDFAMSWVRQAPG KGLEWVSRIYSGDDNASYADSVKGRFTISRDNSKNTLYLQMNS LRAEDTAVYYCARQTEDRSYGYDLDYWGQGTLVTVSS(SEQ ID NO:100) | 1.546 | 348 |
| 1G8-1H1 | EVQLVESGGGLVQPGGSLRLSCAASGDTFSGYAMSWVRQAPG KGLEWVSRIYPYSSVRDYADSVKGRFTISRDNSKNTLYLQMNS LRAEDTAVYYCARQTEDRSYGYDLDYWGQGTLVTVSS(SEQ ID NO:101) | 1.398 | 89.93 |
| 1G8-1H6 | EVQLVESGGGLVQPGGSLRLSCAASGFNFSGFAMSWVRQAPG KGLEWVSRIYPTSSESDYADSVKGRFTISRDNSKNTLYLQMNSL RAEDTAVYYCARQTEDRSYGYDLDYWGQGTLVTVSS(SEQ ID NO:102) | 1.584 | 386.9 |
| 1G8-2A1 | EVQLVESGGGLVQPGGSLRLSCAASGYSFDGFAMSWVRQAPG KGLEWVSRISGHDWSSSYADSVKGRFTISRDNSKNTLYLQMNS LRAEDTAVYYCARQTEDRSYGYDLDYWGQGTLVTVSS(SEQ ID NO:103) | 1.597 | 338.7 |
| 1G8-2A11 | EVQLVESGGGLVQPGGSLRLSCAASGDSFYNYSMSWVRQAPG KGLEWVSRISSDGWETFYADSVKGRFTISRDNSKNTLYLQMNS LRAEDTAVYYCARQTEDRSYGYDLDYWGQGTLVTVSS(SEQ ID NO:104) | 1.538 | 352.9 |
| 1G8-2A4 | EVQLVESGGGLVQPGGSLRLSCAASGDTFSSNAMSWVRQAPG KGLEWVSRIYGGSWDTDYADSVKGRFTISRDNSKNTLYLQMN SLRAEDTAVYYCARQTEDRSYGYDLDYWGQGTLVTVSS(SEQ ID NO:105) | 1.543 | 217.4 |
| 1G8-2A7 | EVQLVESGGGLVQPGGSLRLSCAASGYSFSDYAMSWVRQAPG KGLEWVSRISAYGWGKSYADSVKGRFTISRDNSKNTLYLQMNS LRAEDTAVYYCARQTEDRSYGYDLDYWGQGTLVTVSS(SEQ ID NO:106) | 1.602 | 311.7 |
| 1G8-2B10 | EVQLVESGGGLVQPGGSLRLSCAASGFSFTNNAMSWVRQAPG KGLEWVSRIDTIGGTSSYADSVKGRFTISRDNSKNTLYLQMNSL RAEDTAVYYCARQTEDRSYGYDLDYWGQGTLVTVSS(SEQ ID NO:107) | 1.559 | 344 |
| 1G8-2B12 | EVQLVESGGGLVQPGGSLRLSCAASGDTFGTSSMSWVRQAPG KGLEWVSRIDTWGWYKFYADSVKGRFTISRDNSKNTLYLQMN SLRAEDTAVYYCARQTEDRSYGYDLDYWGQGTLVTVSS(SEQ ID NO:108) | 1.392 | NA |
| 1G8-2B3 | EVQLVESGGGLVQPGGSLRLSCAASGYNFTTSAMSWVRQAPG KGLEWVSRIYQDSSTTDYADSVKGRFTISRDNSKNTLYLQMNS LRAEDTAVYYCARQTEDRSYGYDLDYWGQGTLVTVSS(SEQ ID NO:109) | 1.569 | 396.5 |
| 1G8-2B5 | EVQLVESGGGLVQPGGSLRLSCAASGYNFGNYAMSWVRQAPG KGLEWVSRIKSHDSTSHYADSVKGRFTISRDNSKNTLYLQMNS LRAEDTAVYYCARQTEDRSYGYDLDYWGQGTLVTVSS(SEQ ID NO:110) | 1.578 | NA |
| 1G8-2B6 | EVQLVESGGGLVQPGGSLRLSCAASGGSFGGYAMSWVRQAPG KGLEWVSRIYAYKSENDYADSVKGRFTISRDNSKNTLYLQMNS LRAEDTAVYYCARQTEDRSYGYDLDYWGQGTLVTVSS(SEQ ID NO:111) | 1.565 | 225.1 |
| 1G8-2C6 | EVQLVESGGGLVQPGGSLRLSCAASGDNFSSYAMSWVRQAPG KGLEWVSRIYGSDSTPDYADSVKGRFTISRDNSKNTLYLQMNS LRAEDTAVYYCARQTEDRSYGYDLDYWGQGTLVTVSS(SEQ ID NO:112) | 1.578 | 615.7 |
| 1G8-2C7 | EVQLVESGGGLVQPGGSLRLSCAASGYSFTNFAMSWVRQAPG KGLEWVSRISQDGGNKNYADSVKGRFTISRDNSKNTLYLQMN SLRAEDTAVYYCARQTEDRSYGYDLDYWGQGTLVTVSS(SEQ ID NO:113) | 1.585 | 404.3 |
| 1G8-2D11 | EVQLVESGGGLVQPGGSLRLSCAASGYSFSGNAMSWVRQAPG KGLEWVSRIDGWGSYTDYADSVKGRFTISRDNSKNTLYLQMN SLRAEDTAVYYCARQTEDRSYGYDLDYWGQGTLVTVSS(SEQ ID NO:114) | 1.53 | 298.6 |
| 1G8-2D4 | EVQLVESGGGLVQPGGSLRLSCAASGYNFGGYAMSWVRQAPG KGLEWVSRISGTSGNNDYADSVKGRFTISRDNSKNTLYLQMNS LRAEDTAVYYCARQTEDRSYGYDLDYWGQGTLVTVSS(SEQ ID NO:115) | 1.577 | 492.1 |
| 1G8-2D6 | EVQLVESGGGLVQPGGSLRLSCAASGFSFDGYAMSWVRQAPG KGLEWVSRISAHSWNKDYADSVKGRFTISRDNSKNTLYLQMN SLRAEDTAVYYCARQTEDRSYGYDLDYWGQGTLVTVSS(SEQ ID NO:116) | 1.629 | 523.9 |
| 1G8-2D7 | EVQLVESGGGLVQPGGSLRLSCAASGDSFTSYAMSWVRQAPG KGLEWVSRISADSASPSYADSVKGRFTISRDNSKNTLYLQMNSL RAEDTAVYYCARQTEDRSYGYDLDYWGQGTLVTVSS(SEQ ID NO:117) | 1.544 | 418.6 |
| 1G8-2D9 | EVQLVESGGGLVQPGGSLRLSCAASGDIFDGYSMSWVRQAPG KGLEWVSRIDTGGSVKNYADSVKGRFTISRDNSKNTLYLQMNS LRAEDTAVYYCARQTEDRSYGYDLDYWGQGTLVTVSS(SEQ ID NO:118) | 1.537 | 436.6 |
| 1G8-2E7 | EVQLVESGGGLVQPGGSLRLSCAASGYTFGNYAMSWVRQAPG KGLEWVSRISPSTWNIDYADSVKGRFTISRDNSKNTLYLQMNS LRAEDTAVYYCARQTEDRSYGYDLDYWGQGTLVTVSS(SEQ ID NO:119) | 1.556 | 599.5 |
| 1G8-2F11 | EVQLVESGGGLVQPGGSLRLSCAASGYTFGSNAMSWVRQAPG KGLEWVSRISPDSSGISYADSVKGRFTISRDNSKNTLYLQMNSL RAEDTAVYYCARQTEDRSYGYDLDYWGQGTLVTVSS(SEQ ID NO:120) | 1.574 | 617 |
| 1G8-2F2 | EVQLVESGGGLVQPGGSLRLSCAASGDIFGSYAMSWVRQAPGK GLEWVSRISSHGASRDYADSVKGRFTISRDNSKNTLYLQMNSL RAEDTAVYYCARQTEDRSYGYDLDYWGQGTLVTVSS(SEQ ID NO:121) | 1.599 | 566.8 |
| 1G8-2F9 | EVQLVESGGGLVQPGGSLRLSCAASGYSFSNYAMSWVRQAPG KGLEWVSRISAWGSTNNYADSVKGRFTISRDNSKNTLYLQMNS LRAEDTAVYYCARQTEDRSYGYDLDYWGQGTLVTVSS(SEQ ID NO:122) | 1.565 | 358.8 |
| 1G8-2G12 | EVQLVESGGGLVQPGGSLRLSCAASGFTFSNNAMSWVRQAPG KGLEWVSAIYASTDGRSYADSVKGRFTISRDNSKNTLYLQMNS LRAEDTAVYYCARQTEDRSYGYDLDYWGQGTLVTVSS(SEQ ID NO:123) | 1.505 | 300.9 |
| 1G8-2G3 | EVQLVESGGGLVQPGGSLRLSCAASGFIFGGNAMSWVRQAPG KGLEWVSRISSDKGTTNYADSVKGRFTISRDNSKNTLYLQMNS LRAEDTAVYYCARQTEDRSYGYDLDYWGQGTLVTVSS(SEQ ID NO:124) | 1.583 | 272.1 |
| 1G8-2G4 | EVQLVESGGGLVQPGGSLRLSCAASGFTFGGYSMSWVRQAPG KGLEWVSRISTDGAYTNYADSVKGRFTISRDNSKNTLYLQMNS LRAEDTAVYYCARQTEDRSYGYDLDYWGQGTLVTVSS(SEQ ID NO:125) | 1.58 | 311.9 |
| 1G8-2G7 | EVQLVESGGGLVQPGGSLRLSCAASGGTFYTYAMSWVRQAPG KGLEWVSRIYSTKGTPDYADSVKGRFTISRDNSKNTLYLQMNS LRAEDTAVYYCARQTEDRSYGYDLDYWGQGTLVTVSS(SEQ ID NO:126) | 1.489 | 136 |
| 1G8-2G8 | EVQLVESGGGLVQPGGSLRLSCAASGFNFTNYAMSWVRQAPG KGLEWVSRISTYGDTTHYADSVKGRFTISRDNSKNTLYLQMNS LRAEDTAVYYCARQTEDRSYGYDLDYWGQGTLVTVSS(SEQ ID NO:127) | 1.584 | 370.9 |
| 1G8-2G9 | EVQLVESGGGLVQPGGSLRLSCAASGYIFTNYAMSWVRQAPG KGLEWVSRISQSGGTNDYADSVKGRFTISRDNSKNTLYLQMNS LRAEDTAVYYCARQTEDRSYGYDLDYWGQGTLVTVSS(SEQ ID NO:128) | 1.545 | 285.1 |
| 1G8-2H1 | EVQLVESGGGLVQPGGSLRLSCAASGYSFYSYAMSWVRQAPG KGLEWVSRISQGGGVTHYADSVKGRFTISRDNSKNTLYLQMNS LRAEDTAVYYCARQTEDRSYGYDLDYWGQGTLVTVSS(SEQ ID NO:129) | 1.585 | 277.5 |
| 1G8-2H10 | EVQLVESGGGLVQPGGSLRLSCAASGYNFTNSAMSWVRQAPG KGLEWVSSIYYHSGVPRYADSVKGRFTISRDNSKNTLYLQMNS LRAEDTAVYYCARQTEDRSYGYDLDYWGQGTLVTVSS(SEQ ID NO:130) | 1.535 | 214.2 |
| 1G8-2H11 | EVQLVESGGGLVQPGGSLRLSCAASGFNFDNYAMSWVRQAPG KGLEWVSRIYGWDWYASYADSVKGRFTISRDNSKNTLYLQMN SLRAEDTAVYYCARQTEDRSYGYDLDYWGQGTLVTVSS(SEQ ID NO:131) | 1.347 | NA |
| 1G8-2H3 | EVQLVESGGGLVQPGGSLRLSCAASGYSFGGYAMSWVRQAPG KGLEWVSRISGIKAYNDYADSVKGRFTISRDNSKNTLYLQMNS LRAEDTAVYYCARQTEDRSYGYDLDYWGQGTLVTVSS(SEQ ID NO:132) | 1.531 | 208.9 |
| 1G8-2H4 | EVQLVESGGGLVQPGGSLRLSCAASGDSFTGYAMSWVRQAPG KGLEWVSAISGSGGSTYYADSVKGRFTISRDNSKNTLYLQMNS LRAEDTAVYYCARQTEDRSYGYDLDYWGQGTLVTVSS(SEQ ID NO:133) | 1.578 | 440.5 |
| 1G8-2H8 | EVQLVESGGGLVQPGGSLRLSCAASGDIFGSYAMSWVRQAPGK GLEWVSAISGSGGSTYYADSVKGRFTISRDNSKNTLYLQMNSL RAEDTAVYYCARQTEDRSYGYDLDYWGQGTLVTVSS(SEQ ID NO:134) | 1.574 | 487.2 |
| 1G8-2H9 | EVQLVESGGGLVQPGGSLRLSCAASGFNFGNYAMSWVRQAPG KGLEWVSAIYSYFDEPRYADSVKGRFTISRDNSKNTLYLQMNS LRAEDTAVYYCARQTEDRSYGYDLDYWGQGTLVTVSS(SEQ ID NO:135) | 1.459 | 176.2 |

**Table 5: Heavy chain variable region sequences of 10F4-series antibodies**

| Clone No. | VH sequences (with CDR sequences underlined, according to the Kabat numbering system) | OD value |
|---|---|---|
| 10F4 | EVQLVESGGGLVQPGGSLRLSCAASGFTFSSYAMSWVRQAPGKGLEWVS AISGSGGSTYYADSVKGRFTISRDNSKNTLYLQMNSLRAEDTAVYYCARE YSDVVDYVSVRTPLDYWGQGTLVTVSS(SEQ ID NO:15) | |
| 10F4-1A1 | EVQLVESGGGLVQPGGSLRLSCAASGFSFDNYDMSWVRQAPGKGLEWV SSIKPGSAYKFYADSVKGRFTISRDNSKNTLYLQMNSLRAEDTAVYYCAR EYSDVVDYVSVRTPLDYWGQGTLVTVSS(SEQ ID NO:136) | 1.691 |
| 10F4-1A3 | EVQLVESGGGLVQPGGSLRLSCAASGYSFSNYDMSWVRQAPGKGLEWV SGIKGWKSYTGYADSVKGRFTISRDNSKNTLYLQMNSLRAEDTAVYYCA REYSDVVDYVSVRTPLDYWGQGTLVTVSS(SEQ ID NO:137) | 1.534 |
| 10F4-1B2 | EVQLVESGGGLVQPGGSLRLSCAASGFSFGNYWMSWVRQAPGKGLEWV SGIDGHSSYAFYADSVKGRFTISRDNSKNTLYLQMNSLRAEDTAVYYCAR EYSDVVDYVSVRTPLDYWGQGTLVTVSS(SEQ ID NO:138) | 1.635 |
| 10F4-1B5 | EVQLVESGGGLVQPGGSLRLSCAASGFNFTDYWMSWVRQAPGKGLEWV SSIIGDDSTNHYADSVKGRFTISRDNSKNTLYLQMNSLRAEDTAVYYCAR EYSDVVDYVSVRTPLDYWGQGTLVTVSS(SEQ ID NO:139) | 1.526 |
| 10F4-1B7 | EVQLVESGGGLVQPGGSLRLSCAASGFNFSGFDMSWVRQAPGKGLEWV SGIKAYKSTADYADSVKGRFTISRDNSKNTLYLQMNSLRAEDTAVYYCAR EYSDVVDYVSVRTPLDYWGQGTLVTVSS(SEQ ID NO:140) | 1.605 |
| 10F4-1B9 | EVQLVESGGGLVQPGGSLRLSCAASGFNFDNYYMSWVRQAPGKGLEWV SSIYQTDASIFYADSVKGRFTISRDNSKNTLYLQMNSLRAEDTAVYYCARE YSDVVDYVSVRTPLDYWGQGTLVTVSS(SEQ ID NO:141) | 1.503 |
| 10F4-1C3 | EVQLVESGGGLVQPGGSLRLSCAASGYSFSDYWMSWVRQAPGKGLEWV SSIKQTGGVKHYADSVKGRFTISRDNSKNTLYLQMNSLRAEDTAVYYCA REYSDVVDYVSVRTPLDYWGQGTLVTVSS(SEQ ID NO:142) | 1.586 |
| 10F4-1C4 | EVQLVESGGGLVQPGGSLRLSCAASGFNFTDYWMSWVRQAPGKGLEWV SSIIGDDSTNHYADSVKGRFTISRDNSKNTLYLQMNSLRAEDTAVYYCAR EYSDVVDYVSVRTPLDYWGQGTLVTVSS(SEQ ID NO:143) | 1.458 |
| 10F4-1C7 | EVQLVESGGGLVQPGGSLRLSCAASGDSFDNYWMSWVRQAPGKGLEW VSAIDTHSDNSFYADSVKGRFTISRDNSKNTLYLQMNSLRAEDTAVYYCA REYSDVVDYVSVRTPLDYWGQGTLVTVSS(SEQ ID NO:144) | 1.573 |
| 10F4-1C10 | EVQLVESGGGLVQPGGSLRLSCAASGYNFGSYDMSWVRQAPGKGLEWV SGIKTYGGNTHYADSVKGRFTISRDNSKNTLYLQMNSLRAEDTAVYYCA REYSDVVDYVSVRTPLDYWGQGTLVTVSS(SEQ ID NO:145) | 1.518 |
| 10F4-1D3 | EVQLVESGGGLVQPGGSLRLSCAASGFSFYDYDMSWVRQAPGKGLEWV SSIIQSGSTAFYADSVKGRFTISRDNSKNTLYLQMNSLRAEDTAVYYCARE YSDVVDYVSVRTPLDYWGQGTLVTVSS(SEQ ID NO:146) | 1.521 |
| 10F4-1D11 | EVQLVESGGGLVQPGGSLRLSCAASGFIFSDFDMSWVRQAPGKGLEWVS GIKQSSAGINYADSVKGRFTISRDNSKNTLYLQMNSLRAEDTAVYYCARE YSDVVDYVSVRTPLDYWGQGTLVTVSS(SEQ ID NO:147) | 1.46 |
| 10F4-1E1 | EVQLVESGGGLVQPGGSLRLSCAASGDIFGNYWMSWVRQAPGKGLEWV SSIDASSGNRFYADSVKGRFTISRDNSKNTLYLQMNSLRAEDTAVYYCAR EYSDVVDYVSVRTPLDYWGQGTLVTVSS(SEQ ID NO:148) | 1.627 |
| 10F4-1E10 | EVQLVESGGGLVQPGGSLRLSCAASGDTFDTYAMSWVRQAPGKGLEWV SGISQWGAYASYADSVKGRFTISRDNSKNTLYLQMNSLRAEDTAVYYCA REYSDVVDYVSVRTPLDYWGQGTLVTVSS(SEQ ID NO:149) | 1.289 |
| 10F4-1E12 | EVQLVESGGGLVQPGGSLRLSCAASGYNFSSYDMSWVRQAPGKGLEWV SGISDTGDYANYADSVKGRFTISRDNSKNTLYLQMNSLRAEDTAVYYCAR EYSDVVDYVSVRTPLDYWGQGTLVTVSS(SEQ ID NO:150) | 1.521 |
| 10F4-1F1 | EVQLVESGGGLVQPGGSLRLSCAASGFSFSNYWMSWVRQAPGKGLEWV SAISPGDGTIFYADSVKGRFTISRDNSKNTLYLQMNSLRAEDTAVYYCARE YSDVVDYVSVRTPLDYWGQGTLVTVSS(SEQ ID NO:151) | 1.632 |
| 10F4-1F2 | EVQLVESGGGLVQPGGSLRLSCAASGYNFTNYYMSWVRQAPGKGLEWV SSISAYSDNRNYADSVKGRFTISRDNSKNTLYLQMNSLRAEDTAVYYCAR EYSDVVDYVSVRTPLDYWGQGTLVTVSS(SEQ ID NO:152) | 1.397 |
| 10F4-1F8 | EVQLVESGGGLVQPGGSLRLSCAASGYTFTDYDMSWVRQAPGKGLEWV SSIITDGGGAFYADSVKGRFTISRDNSKNTLYLQMNSLRAEDTAVYYCAR EYSDVVDYVSVRTPLDYWGQGTLVTVSS(SEQ ID NO:153) | 1.492 |
| 10F4-1F9 | EVQLVESGGGLVQPGGSLRLSCAASGFSFGGFDMSWVRQAPGKGLEWV SGIKTYGGYRSYADSVKGRFTISRDNSKNTLYLQMNSLRAEDTAVYYCA REYSDVVDYVSVRTPLDYWGQGTLVTVSS(SEQ ID NO:154) | 1.484 |
| 10F4-1G5 | EVQLVESGGGLVQPGGSLRLSCAASGYTFDSSWMSWVRQAPGKGLEWV SSIDAYGDYNFYADSVKGRFTISRDNSKNTLYLQMNSLRAEDTAVYYCAR EYSDVVDYVSVRTPLDYWGQGTLVTVSS(SEQ ID NO:155) | 1.408 |
| 10F4-1G7 | EVQLVESGGGLVQPGGSLRLSCAASGYNFGDYDMSWVRQAPGKGLEW VSSIIGHGDTKFYADSVKGRFTISRDNSKNTLYLQMNSLRAEDTAVYYCA REYSDVVDYVSVRTPLDYWGQGTLVTVSS(SEQ ID NO:156) | 1.545 |
| 10F4-1G8 | EVQLVESGGGLVQPGGSLRLSCAASGFNFSDFDMSWVRQAPGKGLEWV SVIKPSGDYINYADSVKGRFTISRDNSKNTLYLQMNSLRAEDTAVYYCAR EYSDVVDYVSVRTPLDYWGQGTLVTVSS(SEQ ID NO:157) | 1.52 |
| 10F4-1H1 | EVQLVESGGGLVQPGGSLRLSCAASGYSFGNFDMSWVRQAPGKGLEWV SGIKQYKGYTSYADSVKGRFTISRDNSKNTLYLQMNSLRAEDTAVYYCA REYSDVVDYVSVRTPLDYWGQGTLVTVSS(SEQ ID NO:158) | 1.611 |
| 10F4-1H4 | EVQLVESGGGLVQPGGSLRLSCAASGFNFTNYWMSWVRQAPGKGLEWV SAISGTGGYTYYADSVKGRFTISRDNSKNTLYLQMNSLRAEDTAVYYCAR EYSDVVDYVSVRTPLDYWGQGTLVTVSS(SEQ ID NO:159) | 1.375 |
| 10F4-1H6 | EVQLVESGGGLVQPGGSLRLSCAASGFNFYDYDMSWVRQAPGKGLEWV SGIKTHSGGNGYADSVKGRFTISRDNSKNTLYLQMNSLRAEDTAVYYCA REYSDVVDYVSVRTPLDYWGQGTLVTVSS(SEQ ID NO:160) | 1.478 |
| 10F4-1H7 | EVQLVESGGGLVQPGGSLRLSCAASGYSFDTYDMSWVRQAPGKGLEWV SGIKQYKGYTSYADSVKGRFTISRDNSKNTLYLQMNSLRAEDTAVYYCA REYSDVVDYVSVRTPLDYWGQGTLVTVSS(SEQ ID NO:161) | 1.49 |
| 10F4-1H11 | EVQLVESGGGLVQPGGSLRLSCAASGYTFTGFWMSWVRQAPGKGLEWV SSISPDSGTKHYADSVKGRFTISRDNSKNTLYLQMNSLRAEDTAVYYCAR EYSDVVDYVSVRTPLDYWGQGTLVTVSS(SEQ ID NO:162) | 1.562 |
| 10F4-2A1 | EVQLVESGGGLVQPGGSLRLSCAASGYTFDDYDMSWVRQAPGKGLEWV SGIKAHSDNKRYADSVKGRFTISRDNSKNTLYLQMNSLRAEDTAVYYCA REYSDVVDYVSVRTPLDYWGQGTLVTVSS(SEQ ID NO:163) | 1.758 |
| 10F4-2A5 | EVQLVESGGGLVQPGGSLRLSCAASGYSFTDYDMSWVRQAPGKGLEWV SGIKTYKSDARYADSVKGRFTISRDNSKNTLYLQMNSLRAEDTAVYYCAR EYSDVVDYVSVRTPLDYWGQGTLVTVSS(SEQ ID NO:164) | 1.59 |
| 10F4-2A9 | EVQLVESGGGLVQPGGSLRLSCAASGYTFTDYDMSWVRQAPGKGLEWV SGIKQYKGYTSYADSVKGRFTISRDNSKNTLYLQMNSLRAEDTAVYYCA REYSDVVDYVSVRTPLDYWGQGTLVTVSS(SEQ ID NO:165) | 1.567 |
| 10F4-2B11 | EVQLVESGGGLVQPGGSLRLSCAASGGSFTNYWMSWVRQAPGKGLEWV SVIDSTGSNRFYADSVKGRFTISRDNSKNTLYLQMNSLRAEDTAVYYCAR EYSDVVDYVSVRTPLDYWGQGTLVTVSS(SEQ ID NO:166) | 1.548 |
| 10F4-2C2 | EVQLVESGGGLVQPGGSLRLSCAASGFNFSNYWMSWVRQAPGKGLEWV SVIDAHSANIRYADSVKGRFTISRDNSKNTLYLQMNSLRAEDTAVYYCAR EYSDVVDYVSVRTPLDYWGQGTLVTVSS(SEQ ID NO:167) | 1.584 |
| 10F4-2C4 | EVQLVESGGGLVQPGGSLRLSCAASGYSFGDYWMSWVRQAPGKGLEW VSVIDSGSGNKRYADSVKGRFTISRDNSKNTLYLQMNSLRAEDTAVYYCA REYSDVVDYVSVRTPLDYWGQGTLVTVSS(SEQ ID NO:168) | 1.534 |
| 10F4-2C6 | EVQLVESGGGLVQPGGSLRLSCAASGFNFGGFDMSWVRQAPGKGLEWV SGIKQYKGYTSYADSVKGRFTISRDNSKNTLYLQMNSLRAEDTAVYYCA REYSDVVDYVSVRTPLDYWGQGTLVTVSS(SEQ ID NO:169) | 1.517 |
| 10F4-2C12 | EVQLVESGGGLVQPGGSLRLSCAASGYNFGDYSMSWVRQAPGKGLEWV SVIDTTSAYTRYADSVKGRFTISRDNSKNTLYLQMNSLRAEDTAVYYCAR EYSDVVDYVSVRTPLDYWGQGTLVTVSS(SEQ ID NO:170) | 1.447 |
| 10F4-2E1 | EVQLVESGGGLVQPGGSLRLSCAASGFTFTTYDMSWVRQAPGKGLEWV SGIKYSKYYTHYADSVKGRFTISRDNSKNTLYLQMNSLRAEDTAVYYCA REYSDVVDYVSVRTPLDYWGQGTLVTVSS(SEQ ID NO:171) | 1.596 |
| 10F4-2E4 | EVQLVESGGGLVQPGGSLRLSCAASGYTFDSYWMSWVRQAPGKGLEWV SAIDGTDSVAYYADSVKGRFTISRDNSKNTLYLQMNSLRAEDTAVYYCAR EYSDVVDYVSVRTPLDYWGQGTLVTVSS(SEQ ID NO:172) | 1.429 |
| 10F4-2E6 | EVQLVESGGGLVQPGGSLRLSCAASGFSFGDYWMSWVRQAPGKGLEWV SAIDQGKATSFYADSVKGRFTISRDNSKNTLYLQMNSLRAEDTAVYYCAR EYSDVVDYVSVRTPLDYWGQGTLVTVSS(SEQ ID NO:173) | 1.454 |
| 10F4-2E8 | EVQLVESGGGLVQPGGSLRLSCAASGFNFGNYDMSWVRQAPGKGLEWV SSIIPHGDYSFYADSVKGRFTISRDNSKNTLYLQMNSLRAEDTAVYYCARE YSDVVDYVSVRTPLDYWGQGTLVTVSS(SEQ ID NO:174) | 1.376 |
| 10F4-2F11 | EVQLVESGGGLVQPGGSLRLSCAASGYNFTSYDMSWVRQAPGKGLEWV SGIKTGGGSARYADSVKGRFTISRDNSKNTLYLQMNSLRAEDTAVYYCAR EYSDVVDYVSVRTPLDYWGQGTLVTVSS(SEQ ID NO:175) | 1.455 |
| 10F4-2G2 | EVQLVESGGGLVQPGGSLRLSCAASGYNFTDYDMSWVRQAPGKGLEWV SSIIASGSGNYYADSVKGRFTISRDNSKNTLYLQMNSLRAEDTAVYYCAR EYSDVVDYVSVRTPLDYWGQGTLVTVSS(SEQ ID NO:176) | 1.485 |
| 10F4-2H5 | EVQLVESGGGLVQPGGSLRLSCAASGYIFTNYWMSWVRQAPGKGLEWV SSIDQGSGNIFYADSVKGRFTISRDNSKNTLYLQMNSLRAEDTAVYYCAR EYSDVVDYVSVRTPLDYWGQGTLVTVSS(SEQ ID NO:177) | 1.442 |
| 10F4-1 | EVQLVESGGGLVQPGGSLRLSCAASGDSFTNYWMSWVRQAPGKGLEWV SGISQGGSYAYYADSVKGRFTISRDNSKNTLYLQMNSLRAEDTAVYYCAR EYSDVVDYVSVRTPLDYWGQGTLVTVSS(SEQ ID NO:178) | 1.389 |
| 10F4-3 | EVQLVESGGGLVQPGGSLRLSCAASGFSFGDFAMSWVRQAPGKGLEWV SVIDGHTAYNSYADSVKGRFTISRDNSKNTLYLQMNSLRAEDTAVYYCAR EYSDVVDYVSVRTPLDYWGQGTLVTVSS(SEQ ID NO:179) | 1.325 |
| 10F4-5 | EVQLVESGGGLVQPGGSLRLSCAASGFIFDGYDMSWVRQAPGKGLEWV SVIKDHGGYKFYADSVKGRFTISRDNSKNTLYLQMNSLRAEDTAVYYCA REYSDVVDYVSVRTPLDYWGQGTLVTVSS(SEQ ID NO:180) | 1.308 |

| | | |
|---|---|---|
| NA indicates not available | | |

**Table 6: CDR sequences of heavy chain variable region of 1F3-series antibodies**

| No. | HCDR1 | | | | | HCDR2 | | | | | | | | | | | | HCDR3 |
|---|---|---|---|---|---|---|---|---|---|---|---|---|---|---|---|---|---|---|
| 1F3 | S | Y | A | MS | SEQ ID NO:8 | A | I | S | G | S | G | G | S | T | Y | YADSV KG | SEQ ID NO:9 | AELLAF DVYTAS DY |
| 1F3-1A9 | N | Y | A | MS | SEQ ID NO:181 | R | I | S | P | T | G | G | Y | A | S | YADSV KG | SEQ ID NO:196 | |
| 1F3-1B1 | T | Y | G | MS | SEQ ID NO:182 | G | I | K | P | G | D | S | T | T | Y | YADSV KG | SEQ ID NO: 197 | (SEQ ID NO:14) |
| 1F3-1C11 | S | Y | G | MS | SEQ ID NO:183 | A | I | S | G | S | G | G | S | T | Y | YADSV KG | SEQ ID NO:9 | |
| 1F3-1C4 | D | Y | G | MS | SEQ ID NO:184 | G | I | K | P | W | G | S | G | T | Y | YADSV KG | SEQ ID NO:199 | |
| 1F3-1D1 | N | Y | S | MS | SEQ ID NO:185 | G | I | Y | P | S | G | D | N | N | R | YADSV KG | SEQ ID NO:200 | |
| 1F3-1D12 | D | Y | G | MS | SEQ ID NO:184 | G | I | S | G | S | G | D | N | K | Y | YADSV KG | SEQ ID NO:201 | |
| 1F3-1D6 | G | F | G | MS | SEQ ID NO:196 | G | I | Y | S | H | D | A | D | K | R | YADSV KG | SEQ ID NO:202 | |
| 1F3-1E1 | D | Y | A | MS | SEQ ID NO:187 | I | I | Y | P | T | G | S | T | N | Y | YADSV KG | SEQ ID NO:203 | |
| 1F3-1E10 | S | Y | G | MS | SEQ ID NO:183 | R | I | S | P | Y | T | D | N | K | Y | YADSV KG | SEQ ID NO:204 | |
| 1F3-1E4 | T | Y | G | MS | SEQ ID NO:182 | R | I | S | G | D | S | A | N | I | R | YADSV KG | SEQ ID NO:205 | |
| 1F3-1E9 | D | Y | A | MS | SEQ ID NO:187 | G | I | K | P | S | S | W | T | T | Y | YADSV KG | SEQ ID NO:206 | |
| 1F3-1F4 | G | S | S | MS | SEQ ID NO:188 | G | I | Y | A | s | G | S | D | K | R | YADSV KG | SEQ ID NO:207 | |
| 1F3-1F8 | N | Y | G | MS | SEQ ID NO:189 | R | I | S | G | D | G | S | N | I | N | YADSV KG | SEQ ID NO:208 | |
| 1F3-1G11 | G | Y | G | MS | SEQ ID NO:190 | G | I | S | P | S | G | G | T | I | G | YADSV KG | SEQ ID NO:209 | |
| 1F3-1G2 | D | Y | G | MS | SEQ ID NO: 184 | R | I | D | P | W | G | A | S | I | R | YADSV KG | SEQ ID NO:210 | |
| 1F3-1G5 | S | Y | G | MS | SEQ ID NO:183 | G | I | K | P | S | T | S | T | T | Y | YADSV KG | SEQ ID NO:211 | |
| 1F3-1G7 | N | Y | S | MS | SEQ ID NO:185 | G | I | Y | P | S | G | D | S | R | R | YADSV KG | SEQ ID NO:212 | |
| 1F3-1H11 | D | Y | G | MS | SEQ ID NO:184 | R | I | S | Q | W | G | D | T | S | N | YADSV KG | SEQ ID NO:213 | |
| 1F3-1H3 | N | Y | G | MS | SEQ ID NO:189 | G | I | K | P | H | S | D | V | A | H | YADSV KG | SEQ ID NO:214 | |
| 1F3-2A11 | T | F | Y | MS | SEQ ID NO:191 | G | I | K | P | T | G | S | Y | I | N | YADSV KG | SEQ ID NO:215 | |
| 1F3-2A12 | S | Y | G | MS | SEQ ID NO:183 | G | I | K | P | T | G | A | S | T | Y | YADSV KG | SEQ ID NO:216 | |
| 1F3-2B1 | N | Y | A | MS | SEQ ID NO:181 | G | I | K | P | D | S | A | T | T | Y | YADSV KG | SEQ ID NO:217 | |
| 1F3-2B2 | S | Y | G | MS | SEQ ID NO:183 | I | I | S | P | D | G | G | Y | K | R | YADSV KG | SEQ ID NO:218 | |
| IF3-2B2-2 | D | F | G | MS | SEQ ID NO:192 | R | I | D | P | S | G | W | S | T | S | YADSV KG | SEQ ID NO:219 | |
| 1F3-2B9 | S | Y | A | MS | SEQ ID NO:8 | G | I | K | P | H | T | D | D | I | Y | YADSV KG | SEQ ID NO:220 | |
| IF3-2C6 | N | Y | S | MS | SEQ ID NO:185 | G | I | Y | A | S | G | S | D | K | R | YADSV KG | SEQ ID NO:207 | |
| 1F3-2C9 | G | Y | G | MS | SEQ ID NO:190 | R | I | S | P | G | D | S | T | P | R | YADSV KG | SEQ ID NO:222 | |
| 1F3-2D2 | S | Y | G | MS | SEQ ID NO:183 | R | I | S | P | Y | T | G | T | T | H | YADSV KG | SEQ ID NO:223 | |
| 1F3-2D8 | D | Y | G | MS | SEQ ID NO:184 | R | I | S | P | S | K | A | V | K | D | YADSV KG | SEQ ID NO:224 | |
| 1F3-2E12 | N | S | G | MS | SEQ ID NO:193 | G | I | K | W | S | S | D | E | T | Y | YADSV KG | SEQ ID NO:225 | |
| IF3-2E8 | D | Y | G | MS | SEQ ID NO:184 | W | I | S | P | S | K | G | S | A | S | YADSV KG | SEQ ID NO:226 | |
| 1F3-2F1 | G | Y | S | MS | SEQ ID NO:194 | G | I | Y | Q | T | G | S | N | T | R | YADSV KG | SEQ ID NO:227 | |
| IF3-2F3 | D | Y | G | MS | SEQ ID NO:184 | R | I | D | W | D | G | G | N | T | R | YADSV KG | SEQ ID NO:228 | |
| IF3-2F6 | D | Y | G | MS | SEQ ID NO:184 | R | I | S | Q | Y | G | G | I G | I | R | YADSV KG | SEQ ID NO:229 | |
| 1F3-2G10 | N | Y | G | MS | SEQ ID NO:189 | R | I | K | P | T | D | G | S | T | H | YADSV KG | SEQ ID NO:230 | |
| 1F3-2G9 | G | Y | G | MS | SEQ ID NO:190 | W | I | S | A | W | D | G | S | I | Y | YADSV KG | SEQ ID NO:231 | |
| 1F3-2H12 | S | S | G | MS | SEQ ID NO:195 | G | I | K | W | I | T | G | E | S | Y | YADSV KG | SEQ ID NO:198 | |
| 1F3-2H6 | S | Y | G | MS | SEQ ID NO:183 | R | I | K | S | D | G | G | D | T | H | YADSV KG | SEQ ID NO:221 | |

**Table 7: CDR sequences of heavy chain variable region of 1G8-series antibodies**

| No. | HCDR1 | | | | | HCDR2 | | | | | | | | | | | | HCDR3 |
|---|---|---|---|---|---|---|---|---|---|---|---|---|---|---|---|---|---|---|
| 1G8 | S | Y | A | MS | SEQ ID NO:8 | A | I | S | G | S | G | G | S | T | Y | YADS VKG | SEQ ID NO:9 | |
| 1G8-1A12 | T | N | A | MS | SEQ ID NO:234 | A | I | S | T | G | G | D | N | K | R | YADS VKG | SEQ ID NO:251 | |
| 1G8-1A3 | S | Y | A | MS | SEQ ID NO:8 | R | I | Y | P | G | S | W | S | P | D | YADS VKG | SEQ ID NO:252 | |
| 1G8-1A7 | S | Y | A | MS | SEQ ID NO:8 | R | I | K | T | T | D | D | G | K | S | YADS VKG | SEQ ID NO:253 | |
| 1G8-1B11 | N | Y | A | MS | SEQ ID NO:181 | A | I | D | A | S | G | G | D | T | R | YADS VKG | SEQ ID NO:254 | |
| 1G8-1B7 | N | Y | A | MS | SEQ ID NO:181 | R | I | S | A | W | G | S | T | N | N | YADS VKG | SEQ ID NO:255 | |
| 1G8-1C1 | N | Y | A | MS | SEQ ID NO:181 | R | I | S | T | G | F | A | G | P | D | YADS VKG | SEQ ID NO:256 | |
| 1G8-1C10 | D | Y | S | MS | SEQ ID NO:235 | R | I | K | A | S | S | G | G | S | D | YADS VKG | SEQ ID NO:257 | QTEDRS YGYDLD Y SEQ ID NO:12 |
| 1G8-1C12 | G | F | G | MS | SEQ ID NO:186 | G | I | Y | S | H | D | A | D | K | R | YADS VKG | SEQ ID NO:202 | |
| 1G8-1C8 | S | S | A | MS | SEQ ID NO:236 | A | I | S | G | S | S | D | S | P | R | YADS VKG | SEQ ID NO:258 | |
| 1G8-1D11 | G | Y | A | MS | SEQ ID NO:237 | R | I | D | G | T | G | D | S | K | F | YADS VKG | SEQ ID NO:259 | |
| 1G8-1D7 | N | Y | A | MS | SEQ ID NO:181 | W | I | D | T | T | S | A | Y | A | S | YADS VKG | SEQ ID NO:260 | |
| 1G8-1E10 | T | Y | A | MS | SEQ ID NO:238 | R | I | S | G | S | G | D | G | K | D | YADS VKG | SEQ ID NO:261 | |
| 1G8-1E12 | S | Y | A | MS | SEQ ID NO:8 | R | I | S | A | H | K | W | D | A | S | YADS VKG | SEQ ID NO:262 | |
| 1G8-1E6 | D | Y | S | MS | SEQ ID NO:235 | R | I | Y | T | D | G | W | E | K | D | YADS VKG | SEQ ID NO:263 | |
| 1G8-1E8 | G | Y | A | MS | SEQ ID NO:237 | R | I | S | S | Y | S | W | N | P | D | YADS VKG | SEQ ID NO:264 | |
| 1G8-1F11 | S | Y | A | MS | SEQ ID NO:8 | R | I | S | Q | T | G | G | T | T | D | YADS VKG | SEQ ID NO:265 | |
| 1G8-1F5 | T | Y | | MS | SEQ ID NO:239 | R | I | Y | T | D | G | | S | S | D | YADS VKG | SEQ ID NO:266 | |
| 1G8-1F7 | G | Y | A | MS | SEQ ID NO:237 | A | I | D | A | I | G | G | N | K | R | YADS VKG | SEQ ID NO:267 | |
| 1G8-1G12 | S | S | A | MS | SEQ ID NO:236 | R | I | Y | S | I | S | G | N | T | D | YADS VKG | SEQ ID NO:268 | |
| 1G8-1G3 | T | Y | A | MS | SEQ ID NO:238 | R | I | S | G | I | S | A | G | T | N | YADS VKG | SEQ ID NO:269 | |
| 1G8-1G5 | T | S | A | MS | SEQ ID NO:240 | R | I | Y | A | D | T | A | Y | N | D | YADS VKG | SEQ ID NO:270 | |
| 1G8-1G9 | D | F | A | MS | SEQ ID NO:241 | R | I | Y | S | G | D | D | N | A | S | YADS VKG | SEQ ID NO:271 | |
| 1G8-1H1 | G | Y | A | MS | SEQ ID NO:237 | R | I | Y | P | Y | S | S | V | R | D | YADS VKG | SEQ ID NO:272 | |
| 1G8-1H6 | G | F | A | MS | SEQ ID NO:242 | R | I | Y | P | T | S | S | E | S | D | YADS VKG | SEQ ID NO:273 | |
| 1G8-2A1 | G | F | A | MS | SEQ ID NO: 242 | R | I | S | G | H | D | W | S | S | S | YADS VKG | SEQ ID NO:274 | |
| 1G8-2A11 | N | Y | S | MS | SEQ ID NO:195 | R | I | S | S | D | G | W | E | T | F | YADS VKG | SEQ ID NO:275 | |
| 1 G8-2A4 | S | N | A | MS | SEQ ID NO:243 | R | I | Y | G | G | S | W | D | T | D | YADS VKG | SEQ ID NO:276 | |
| 1G8-2A7 | D | Y | A | MS | SEQ ID NO:187 | R | I | S | A | Y | G | W | G | K | S | YADS VKG | SEQ ID NO:277 | |
| 1G8-2B10 | N | N | A | MS | SEQ ID NO:244 | R | I | D | T | I | G | G | T | S | S | YADS VKG | SEQ ID NO:278 | |
| 1G8-2B12 | T | S | S | MS | SEQ ID NO:245 | R | I | D | T | W | G | W | Y | K | F | YADS VKG | SEQ ID NO:279 | |
| 1G8-2B3 | T | S | A | MS | SEQ ID NO:240 | R | I | Y | Q | D | S | S | T | T | D | YADS VKG | SEQ ID NO:280 | |
| 1G8-2B5 | N | Y | A | MS | SEQ ID NO:181 | R | I | K | S | H | D | S | T | S | H | YADS VKG | SEQ ID NO:281 | |
| 1G8-2B6 | G | Y | A | MS | SEQ ID NO:237 | R | I | Y | A | Y | K | S | E | N | D | YADS VKG | SEQ ID NO:282 | |
| 1G8-2C6 | S | Y | A | MS | SEQ ID NO:8 | R | I | Y | G | S | D | S | T | P | D | YADS VKG | SEQ ID NO:283 | |
| 1G8-2C7 | N | F | A | MS | SEQ ID NO:246 | R | I | S | Q | D | G | G | N | K | N | YADS VKG | SEQ ID NO:284 | |
| 1G8-2D11 | G | N | A | MS | SEQ ID NO:247 | R | I | D | G | W | G | S | Y | T | D | YADS VKG | SEQ ID NO:285 | |
| 1G8-2D4 | G | Y | A | MS | SEQ ID NO:237 | R | I | S | G | T | S | G | N | N | D | YADS VKG | SEQ ID NO:286 | |
| 1G8-2D6 | G | Y | A | MS | SEQ ID NO:237 | R | I | S | A | H | S | W | N | K | D | YADS VKG | SEQ ID NO:287 | |
| 1G8-2D7 | S | Y | A | MS | SEQ ID NO:8 | R | I | S | A | D | S | A | S | P | S | YADS VKG | SEQ ID NO:288 | |
| 1G8-2D9 | G | Y | S | MS | SEQ ID NO:194 | R | I | D | T | G | G | S | V | K | N | YADS VKG | SEQ ID NO:289 | |
| 1G8-2E7 | N | Y | A | MS | SEQ ID NO:181 | R | I | S | P | S | T | W | N | I | D | YADS VKG | SEQ ID NO:290 | |
| 1G8-2F11 | S | N | A | MS | SEQ ID NO:243 | R | I | S | P | D | S | S | G | I | S | YADS VKG | SEQ ID NO:291 | |
| 1G8-2F2 | S | Y | A | MS | SEQ ID NO:8 | R | I | S | S | H | G | A | S | R | D | YADS VKG | SEQ ID NO:292 | |
| 1G8-2F9 | N | Y | A | MS | SEQ ID NO:181 | R | I | S | A | W | G | S | T | N | N | YADS VKG | SEQ ID NO:255 | |
| 1G8-2G12 | N | N | A | MS | SEQ ID NO:244 | A | I | Y | A | S | T | D | G | R | S | YADS VKG | SEQ ID NO:293 | |
| 1G8-2G3 | G | N | A | MS | SEQ ID NO:247 | R | I | S | S | D | K | G | T | T | N | YADS VKG | SEQ ID NO:294 | |
| 1G8-2G4 | G | Y | S | MS | SEQ ID NO:194 | R | I | S | T | D | G | A | Y | T | N | YADS VKG | SEQ ID NO:295 | |
| 1G8-2G7 | T | Y | A | MS | SEQ ID NO:238 | R | I | Y | S | T | K | G | T | P | D | YADS VKG | SEQ ID NO:296 | |
| 1G8-2G8 | N | Y | A | MS | SEQ ID NO:181 | R | I | S | T | Y | G | D | T | T | H | YADS VKG | SEQ ID NO:297 | |
| 1G8-2G9 | N | Y | A | MS | SEQ ID NO:181 | R | I | S | Q | S | G | G | T | N | D | YADS VKG | SEQ ID NO:298 | |
| 1G8-2H1 | S | Y | A | MS | SEQ ID NO:8 | R | I | S | Q | G | G | G | J | T | H | YADS VKG | SEQ ID NO:299 | |
| 1G8-2H10 | N | S | A | MS | SEQ ID NO:248 | S | I | Y | Y | H | S | G | J | P | R | YADS VKG | SEQ ID NO:300 | |
| 1G8-2H11 | N | Y | A | MS | SEQ ID NO:181 | R | I | Y | G | W | D | W | Y | A | S | YADS VKG | SEQ ID NO:301 | |
| 1G8-2H3 | G | Y | A | MS | SEQ ID NO:237 | R | I | S | G | I | K | A | Y | N | D | YADS VKG | SEQ ID NO: 302 | |
| 1G8-2H4 | G | Y | A | MS | SEQ ID NO:237 | A | I | S | G | S | G | G | S | T | Y | YADS VKG | SEQ ID NO:9 | |
| 1G8-2H8 | S | Y | A | MS | SEQ ID NO:8 | A | I | S | G | S | G | G | S | T | Y | YADS VKG | SEQ ID NO:9 | |
| 1 G8-2H9 | N | Y | A | MS | SEQ ID NO:181 | A | I | Y | S | Y | F | D | E | P | R | YADS VKG | SEQ ID NO: 303 | |

**Table 8: CDR sequences of heavy chain variable region of 10F4-series antibodies**

| No. | HCDR1 | | | | | HCDR2 | | | | | | | | | | | | HCDR3 |
|---|---|---|---|---|---|---|---|---|---|---|---|---|---|---|---|---|---|---|
| 10F4 | S | Y | A | MS | SEQ ID NO: 8 | A | I | S | G | S | G | G | S | T | Y | YADS VKG | SEQ ID NO:9 | |
| 10F4-1A1 | N | Y | D | MS | SEQ ID NO:304 | S | I | K | P | G | S | A | Y | K | F | YADS VKG | SEQ ID NO:318 | |
| 10F4-1A3 | N | Y | D | MS | SEQ ID NO:304 | G | I | K | G | W | K | S | Y | T | G | YADS VKG | SEQ ID NO:319 | |
| 10F4-1B2 | N | Y | W | MS | SEQ ID NO:305 | G | I | D | G | H | S | S | Y | A | F | YADS VKG | SEQ ID NO:320 | |
| 10F4-1B5 | D | Y | W | MS | SEQ ID NO:306 | S | I | I | G | D | D | S | T | N | H | YADS VKG | SEQ ID NO:321 | |
| 10F4-1B7 | G | F | D | MS | SEQ ID NO:307 | G | I | K | A | Y | K | S | T | A | D | YADS VKG | SEQ ID NO:322 | EYSDVVDY VSVRTPLDY SEQ ID NO:16 |
| 10F4-1B9 | N | Y | Y | MS | SEQ ID NO:308 | S | I | Y | Q | T | D | A | S | I | F | YADS VKG | SEQ ID NO:323 | |
| 10F4-1C3 | D | Y | W | MS | SEQ ID NO:306 | S | I | K | Q | T | G | G | V | K | H | YADS VKG | SEQ ID NO: 324 | |
| 10F4-1C4 | D | Y | W | MS | SEQ ID NO:306 | S | I | I | G | D | D | S | T | N | H | YADS VKG | SEQ ID NO:321 | |
| 10F4-1C7 | N | Y | W | MS | SEQ ID NO:305 | A | I | D | T | H | S | D | N | S | F | YADS VKG | SEQ ID NO: 325 | |
| 10F4-1C10 | S | Y | D | MS | SEQ ID NO:309 | G | I | K | T | Y | G | G | N | T | H | YADS VKG | SEQ ID NO326 | |
| 10F4-1D3 | D | Y | D | MS | SEQ ID NO:310 | S | I | I | Q | S | G | S | T | A | F | YADS VKG | SEQ ID NO: 327 | |
| 10F4-1D11 | D | F | D | MS | SEQ ID NO:311 | G | I | K | Q | S | S | A | G | I | N | YADS VKG | SEQ ID NO:328 | |
| 10F4-1E1 | N | Y | W | MS | SEQ ID NO:305 | S | I | D | A | S | S | G | N | R | F | YADS VKG | SEQ ID NO:329 | |
| 10F4-1E10 | T | Y | A | MS | SEQ ID NO:238 | G | I | S | Q | W | G | A | Y | A | S | YADS VKG | SEQ ID NO:330 | |
| 10F4-1E12 | S | Y | D | MS | SEQ ID NO:309 | G | I | S | D | T | G | D | Y | A | N | YADS VKG | SEQ ID NO:331 | |
| 10F4-1F1 | N | Y | W | MS | SEQ ID NO:305 | A | I | S | P | G | D | G | T | I | F | YADS VKG | SEQ ID NO:332 | |
| 10F4-1F2 | N | Y | Y | MS | SEQ ID NO:308 | S | I | S | A | Y | S | D | N | R | N | YADS VKG | SEQ ID NO:333 | |
| 10F4-1F8 | D | Y | D | MS | SEQ ID NO:310 | S | I | I | T | D | G | G | G | A | F | YADS VKG | SEQ ID NO:334 | |
| 10F4-1F9 | G | F | D | MS | SEQ ID NO:307 | G | I | K | T | Y | G | G | Y | R | S | YADS VKG | SEQ ID NO:335 | |
| 10F4-1G5 | S | S | W | MS | SEQ ID NO:312 | S | I | D | A | Y | G | D | Y | N | F | YADS VKG | SEQ ID NO: 336 | |
| 10F4-1G7 | D | Y | D | MS | SEQ ID NO:310 | S | I | I | G | H | G | D | T | K | F | YADS VKG | SEQ ID NO:337 | |
| 10F4-1G8 | D | F | D | MS | SEQ ID NO:311 | V | I | K | P | S | G | D | Y | I | N | YADS VKG | SEQ ID NO:338 | |
| 10F4-1H1 | N | F | D | MS | SEQ ID NO:313 | G | I | K | Q | Y | K | G | Y | T | S | YADS VKG | SEQ ID NO:339 | |
| 10F4-1H4 | N | Y | W | MS | SEQ ID NO:305 | A | I | S | G | T | G | G | Y | T | Y | YADS VKG | SEQ ID NO: 340 | |
| 10F4-1H6 | D | Y | D | MS | SEQ ID NO:310 | G | I | K | T | H | S | G | G | N | G | YADS VKG | SEQ ID NO:341 | |
| 10F4-1H7 | T | Y | D | MS | SEQ ID NO:314 | G | I | K | Q | Y | K | G | Y | T | S | YADS VKG | SEQ ID NO:339 | |
| 10F4-1H11 | G | F | W | MS | SEQ ID NO:315 | S | I | S | P | D | S | G | T | K | H | YADS VKG | SEQ ID NO: 342 | |
| 10F4-2A1 | D | Y | D | MS | NO:310 | G | I | K | A | H | S | D | N | K | R | YADS VKG | SEQ ID NO: 343 | |
| 10F4-2A5 | D | Y | D | MS | NO:310 | G | I | K | T | Y | K | S | D | A | R | YADS VKG | SEQ ID NO: 344 | |
| 10F4-2A9 | D | Y | D | MS | SEQ ID NO:310 | G | I | K | Q | Y | K | G | Y | T | S | YADS VKG | SEQ ID NO:339 | |
| 10F4-2B11 | N | Y | W | MS | SEQ ID NO:305 | V | I | D | S | T | G | S | N | R | F | YADS VKG | SEQ ID NO:345 | |
| 10F4-2C2 | N | Y | W | MS | SEQ ID NO:305 | V | I | D | A | H | S | A | N | I | R | YADS VKG | SEQ ID NO:346 | |
| 10F4-2C4 | D | Y | W | MS | SEQ ID NO:306 | V | I | D | S | G | S | G | N | K | R | YADS VKG | SEQ ID NO:347 | |
| 10F4-2C6 | G | F | D | MS | SEQ ID NO:307 | G | I | K | Q | Y | K | G | Y | T | S | YADS VKG | SEQ ID NO:339 | |
| 10F4-2C12 | D | Y | S | MS | SEQ ID NO:235 | v | I | D | T | T | S | A | Y | T | R | YADS VKG | SEQ ID NO:348 | |
| 10F4-2E1 | T | Y | D | MS | SEQ ID NO:314 | G | I | K | Y | S | K | Y | Y | T | H | YADS VKG | SEQ ID NO:349 | |
| 10F4-2E4 | S | Y | W | MS | SEQ ID NO:316 | A | I | D | G | T | D | S | V | A | Y | YADS VKG | SEQ ID NO:350 | |
| 10F4-2E6 | D | Y | W | MS | SEQ ID NO:306 | A | I | D | Q | G | K | A | T | S | F | YADS VKG | SEQ ID NO:351 | |
| 10F4-2E8 | N | Y | D | MS | SEQ ID NO:304 | S | I | I | P | H | G | D | Y | S | F | YADS VKG | SEQ ID NO:352 | |
| 10F4-2F11 | S | Y | D | MS | SEQ ID NO:309 | G | I | K | T | G | G | G | S | A | R | YADS VKG | SEQ ID NO:353 | |
| 10F4-2G2 | D | Y | D | MS | SEQ ID NO:310 | S | I | I | A | S | G | S | G | N | Y | YADS VKG | SEQ ID NO:354 | |
| 10F4-2H5 | N | Y | W | MS | SEQ ID NO:305 | S | I | D | Q | G | S | G | N | I | F | YADS VKG | SEQ ID NO:355 | |
| 10F4-1 | N | Y | W | MS | SEQ ID NO:305 | G | I | S | Q | G | G | S | Y | A | Y | YADS VKG | SEQ ID NO:356 | |
| 10F4-3 | D | F | A | MS | SEQ ID NO:241 | V | I | D | G | H | T | A | Y | N | S | YADS VKG | SEQ ID NO:357 | |
| 10F4-5 | G | Y | D | MS | SEQ ID NO:317 | V | I | K | D | H | G | G | Y | K | F | YADS VKG | SEQ ID NO:358 | |

**Amino acid sequence of antibody heavy chain constant region (CH):** 





 (SEQ ID NO: 37)
Amino acid sequence of antibody light chain constant region (CL): 
TVAAPSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC (SEQ ID NO:
38)
**The amino acid sequence of the heavy chain of antibody 1F3 is as follows:** 
EVQLVESGGGLVQPGGSLRLSCAASGFTFSSYAMSWVRQAPGKGLEWVSAISGSGGS
TYYADSVKGRFTISRDNSKNTLYLQMNSLRAEDTAVYYCARAELLAFDVYTASDYW
GQGTLVTVSSASTKGPSVFPLAPSSKSTSGGTAALGCLVKDYFPEPVTVSWNSGALTS
GVHTFPAVLQSSGLYSLSSVVTVPSSSLGTQTYICNVNHKPSNTKVDKKVEPKSCDKT
HTCPPCPAPELLGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIEKTISK
AKGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGK (SEQ ID
NO: 232)
**The amino acid sequence of the heavy chain of antibody 1G8 is as follows:** 
EVQLVESGGGLVQPGGSLRLSCAASGFTFSSYAMSWVRQAPGKGLEWVSAISGSGGS
TYYADSVKGRFTISRDNSKNTLYLQMNSLRAEDTAVYYCARQTEDRSYGYDLDYWG
QGTLVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCDKTH
TCPPCPAPELLGGPSVFLFPPKPKDTLMISRTPEVTCVVVDVSHEDPEVKFNWYVDGV
EVHNAKTKPREEQYNSTYRVVSVLTVLHQDWLNGKEYKCKVSNKALPAPIEKTISKA
KGQPREPQVYTLPPSRDELTKNQVSLTCLVKGFYPSDIAVEWESNGQPENNYKTTPPV
LDSDGSFFLYSKLTVDKSRWQQGNVFSCSVMHEALHNHYTQKSLSLSPGK (SEQ ID
NO: 233)
**The amino acid sequence of the heavy chain of antibody 10F4 is as follows:** 
EVQLVESGGGLVQPGGSLRLSCAASGFTFSSYAMSWVRQAPGKGLEWVSAISGSGGS
TYYADSVKGRFTISRDNSKNTLYLQMNSLRAEDTAVYYCAREYSDVVDYVSVRTPLD
YWGQGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTI
 
SKAKGQPREPQVYTLPPSRDELTKNQVSLTCLVKGFYPSDIAVEWESNGQPENNYKTT
PPVLDSDGSFFLYSKLTVDKSRWQQGNVFSCSVMHEALHNHYTQKSLSLSPGK (SEQ
ID NO: 249)
**The amino acid sequence of the heavy chain of antibody 10F4-3 is as follows:** 
EVQLVESGGGLVQPGGSLRLSCAASGFSFGDFAMSWVRQAPGKGLEWVSVIDGHTA
YNSYADSVKGRFTISRDNSKNTLYLQMNSLRAEDTAVYYCAREYSDVVDYVSVRTPL
DYWGQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVEPKSC
DKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIEK
TISKAKGQPREPQVYTLPPSRDELTKNQVSLTCLVKGFYPSDIAVEWESNGQPENNYK
TTPPVLDSDGSFFLYSKLTVDKSRWQQGNVFSCSVMHEALHNHYTQKSLSLSPGK
(SEQ ID NO: 360)
**The nucleic acid sequence of the heavy chain of antibody 10F4-3 is as follows:** 
gaagtgcaattggtggaaagcggcggcggcctggtgcagccgggcggcagcctgcgcctgagctgcgcggcgtccggattctctttt
ggtgacttcgctatgagctgggtgcgccaggcaccgggcaaaggcctcgaatgggtgagcgttattgacggtcatactgcttacaact
cttatgcggatagcgtgaaaggccgctttaccattagccgcgataattcgaaaaacaccctgtatctgcaaatgaacagcctgcgtgcg
gaagatactgcagtgtattattgcgcgcgtgaatactctgacgttgttgactacgtttctgttcgtactccgctggattattggggccaaggc
accctggtgacggtgagcagcgcctctaccaagggcccctctgtgtttcctctggctccctccagcaagtctacctctggtggaacagct
gccctgggctgcctggtcaaggattactttcctgagcctgtcaccgtgtcctggaactctggcgctctgacatctggcgtgcacacctttc
cagctgtgctccagtcctccggcctgtactctctgtcctctgtcgtgaccgtgccttctagctctctgggcacccagacctacatctgcaat
gtgaaccacaagccttccaacaccaaggtggacaagaaggtggaacccaagtcctgcgacaagacccacacctgtcctccatgtcct
gctccagaactgctcggcggaccttccgtgttcctgtttcctccaaagcctaaggacaccctgatgatctctcggacccctgaagtgacc
tgcgtggtggtggatgtgtctcacgaagatcccgaagtgaagttcaactggtacgtggacggcgtggaagtgcacaacgccaagacc
aagcctagagaggaacagtacaactccacctacagagtggtgtccgtgctgaccgtgctgcaccaggattggctgaacggcaaagag
tacaagtgcaaggtgtccaacaaggccctgcctgctcctatcgaaaagaccatctccaaggccaagggccagcctagggaacccca
ggtttacaccttgcctccatctcgggacgagctgaccaagaaccaggtgtccctgacctgtctcgtgaagggcttctacccctccgacat
cgccgtggaatgggagtctaatggccagcctgagaacaactacaagacaacccctcctgtgctggactccgacggctcattcttcctgt
actccaagctgacagtggacaagtccagatggcagcagggcaacgtgttctcctgctccgtgatgcacgaggccctgcacaatcacta
cacccagaagtccctgtctctgtcccctggcaaataa (SEQ ID NO: 364)
**The amino acid sequence of the heavy chain of antibody 1F3-1E4 is as follows:** 
EVQLVESGGGLVQPGGSLRLSCAASGFSFGTYGMSWVRQAPGKGLEWVSRISGDSA
NIRYADSVKGRFTISRDNSKNTLYLQMNSLRAEDTAVYYCARAELLAFDVYTASDYW
GQGTLVTVSSASTKGPSVFPLAPSSKSTSGGTAALGCLVKDYFPEPVTVSWNSGALTS
GVHTFPAVLQSSGLYSLSSVVTVPSSSLGTQTYICNVNHKPSNTKVDKKVEPKSCDKT
HTCPPCPAPELLGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIEKTISK
AKGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGK (SEQ ID
NO: 361)
**The nucleic acid sequence of the heavy chain of antibody 1F3-1E4 is as follows:** 
gaagtgcaattggtggaaagcggcggcggcctggtgcagccgggcggcagcctgcgcctgagctgcgcggcgtccggattctctttt
ggtacttacggtatgagctgggtgcgccaggcaccgggcaaaggcctcgaatgggtgagccgtatttctggtgactctgctaacatcc
gttatgcggatagcgtgaaaggccgctttaccattagccgcgataattcgaaaaacaccctgtatctgcaaatgaacagcctgcgtgcg
gaagatactgcagtgtattattgcgcgcgtgctgaactgctggctttcgacgtttacactgcttctgattattggggccaaggcaccctggt
gacggtgagcagcgcctctaccaagggcccctctgtgtttcctctggctccctccagcaagtctacctctggtggaacagctgccctgg
gctgcctggtcaaggattactttcctgagcctgtcaccgtgtcctggaactctggcgctctgacatctggcgtgcacacctttccagctgt
gctccagtcctccggcctgtactctctgtcctctgtcgtgaccgtgccttctagctctctgggcacccagacctacatctgcaatgtgaacc
 
acaagccttccaacaccaaggtggacaagaaggtggaacccaagtcctgcgacaagacccacacctgtcctccatgtcctgctccag
aactgctcggcggaccttccgtgttcctgtttcctccaaagcctaaggacaccctgatgatctctcggacccctgaagtgacctgcgtgg
tggtggatgtgtctcacgaagatcccgaagtgaagttcaactggtacgtggacggcgtggaagtgcacaacgccaagaccaagccta
gagaggaacagtacaactccacctacagagtggtgtccgtgctgaccgtgctgcaccaggattggctgaacggcaaagagtacaagt
gcaaggtgtccaacaaggccctgcctgctcctatcgaaaagaccatctccaaggccaagggccagcctagggaaccccaggtttaca
ccttgcctccatctcgggacgagctgaccaagaaccaggtgtccctgacctgtctcgtgaagggcttctacccctccgacatcgccgtg
gaatgggagtctaatggccagcctgagaacaactacaagacaacccctcctgtgctggactccgacggctcattcttcctgtactccaa
gctgacagtggacaagtccagatggcagcagggcaacgtgttctcctgctccgtgatgcacgaggccctgcacaatcactacaccca
gaagtccctgtctctgtcccctggcaaataa (SEQ ID NO: 365)
**The amino acid sequence of the heavy chain of antibody 1F3-2B9 is as follows:** 
EVQLVESGGGLVQPGGSLRLSCAASGFNFDSYAMSWVRQAPGKGLEWVSGIKPHTD
DIYYADSVKGRFTISRDNSKNTLYLQMNSLRAEDTAVYYCARAELLAFDVYTASDYW
GQGTLVTVSSASTKGPSVFPLAPSSKSTSGGTAALGCLVKDYFPEPVTVSWNSGALTS
GVHTFPAVLQSSGLYSLSSVVTVPSSSLGTQTYICNVNHKPSNTKVDKKVEPKSCDKT
HTCPPCPAPELLGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIEKTISK
AKGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGK (SEQ ID
NO: 362)
**The nucleic acid sequence of the heavy chain of antibody 1F3-2B9 is as follows:** 
gaagtgcaattggtggaaagcggcggcggcctggtgcagccgggcggcagcctgcgcctgagctgcgcggcgtccggattcaact
ttgactcttacgctatgagctgggtgcgccaggcaccgggcaaaggcctcgaatgggtgagcggtattaaaccgcatactgacgacat
ctactatgcggatagcgtgaaaggccgctttaccattagccgcgataattcgaaaaacaccctgtatctgcaaatgaacagcctgcgtgc
ggaagatactgcagtgtattattgcgcgcgtgctgaactgctggctttcgacgtttacactgcttctgattattggggccaaggcaccctg
gtgacggtgagcagcgcctctaccaagggcccctctgtgtttcctctggctccctccagcaagtctacctctggtggaacagctgccct
gggctgcctggtcaaggattactttcctgagcctgtcaccgtgtcctggaactctggcgctctgacatctggcgtgcacacctttccagct
gtgctccagtcctccggcctgtactctctgtcctctgtcgtgaccgtgccttctagctctctgggcacccagacctacatctgcaatgtgaa
ccacaagccttccaacaccaaggtggacaagaaggtggaacccaagtcctgcgacaagacccacacctgtcctccatgtcctgctcc
agaactgctcggcggaccttccgtgttcctgtttcctccaaagcctaaggacaccctgatgatctctcggacccctgaagtgacctgcgt
ggtggtggatgtgtctcacgaagatcccgaagtgaagttcaactggtacgtggacggcgtggaagtgcacaacgccaagaccaagcc
tagagaggaacagtacaactccacctacagagtggtgtccgtgctgaccgtgctgcaccaggattggctgaacggcaaagagtacaa
gtgcaaggtgtccaacaaggccctgcctgctcctatcgaaaagaccatctccaaggccaagggccagcctagggaaccccaggttta
caccttgcctccatctcgggacgagctgaccaagaaccaggtgtccctgacctgtctcgtgaagggcttctacccctccgacatcgccg
tggaatgggagtctaatggccagcctgagaacaactacaagacaacccctcctgtgctggactccgacggctcattcttcctgtactcca
agctgacagtggacaagtccagatggcagcagggcaacgtgttctcctgctccgtgatgcacgaggccctgcacaatcactacaccc
agaagtccctgtctctgtcccctggcaaataa (SEQ ID NO: 366)
**The nucleic acid sequence of the heavy chain of antibody 1G8-1C10 is as follows:** 
EVQLVESGGGLVQPGGSLRLSCAASGFNFSDYSMSWVRQAPGKGLEWVSRIKASSGG
SDYADSVKGRFTISRDNSKNTLYLQMNSLRAEDTAVYYCARQTEDRSYGYDLDYWG
QGTLVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCDKTH
TCPPCPAPELLGGPSVFLFPPKPKDTLMISRTPEVTCVVVDVSHEDPEVKFNWYVDGV
EVHNAKTKPREEQYNSTYRVVSVLTVLHQDWLNGKEYKCKVSNKALPAPIEKTISKA
KGQPREPQVYTLPPSRDELTKNQVSLTCLVKGFYPSDIAVEWESNGQPENNYKTTPPV
LDSDGSFFLYSKLTVDKSRWQQGNVFSCSVMHEALHNHYTQKSLSLSPGK (SEQ ID
NO: 363)
**The nucleic acid sequence of the heavy chain of antibody 1G8-1C10 is as follows:** 
gaagtgcaattggtggaaagcggcggcggcctggtgcagccgggcggcagcctgcgcctgagctgcgcggcgtccggattcaact
tttctgactactctatgagctgggtgcgccaggcaccgggcaaaggcctcgaatgggtgagccgtattaaagcttcttctggtggttctg
actatgcggatagcgtgaaaggccgctttaccattagccgcgataattcgaaaaacaccctgtatctgcaaatgaacagcctgcgtgcg
gaagatactgcagtgtattattgcgcgcgtcagactgaagaccgttcttacggttacgacctggattattggggccaaggcaccctggtg
acggtgagcagcgcctctaccaagggcccctctgtgtttcctctggctccctccagcaagtctacctctggtggaacagctgccctggg
ctgcctggtcaaggattactttcctgagcctgtcaccgtgtcctggaactctggcgctctgacatctggcgtgcacacctttccagctgtg
ctccagtcctccggcctgtactctctgtcctctgtcgtgaccgtgccttctagctctctgggcacccagacctacatctgcaatgtgaacca
caagccttccaacaccaaggtggacaagaaggtggaacccaagtcctgcgacaagacccacacctgtcctccatgtcctgctccaga
actgctcggcggaccttccgtgttcctgtttcctccaaagcctaaggacaccctgatgatctctcggacccctgaagtgacctgcgtggt
ggtggatgtgtctcacgaagatcccgaagtgaagttcaactggtacgtggacggcgtggaagtgcacaacgccaagaccaagcctag
agaggaacagtacaactccacctacagagtggtgtccgtgctgaccgtgctgcaccaggattggctgaacggcaaagagtacaagtg
caaggtgtccaacaaggccctgcctgctcctatcgaaaagaccatctccaaggccaagggccagcctagggaaccccaggtttacac
cttgcctccatctcgggacgagctgaccaagaaccaggtgtccctgacctgtctcgtgaagggcttctacccctccgacatcgccgtgg
aatgggagtctaatggccagcctgagaacaactacaagacaacccctcctgtgctggactccgacggctcattcttcctgtactccaag
ctgacagtggacaagtccagatggcagcagggcaacgtgttctcctgctccgtgatgcacgaggccctgcacaatcactacacccag
aagtccctgtctctgtcccctggcaaataa (SEQ ID NO: 367)
**The amino acid sequence of the common light chain is as follows:** 
DIQMTQSPSSLSASVGDRVTITCRASQGISSYLAWYQQKPGKAPKLLIYAASSLQSGVP
SRFSGSGSGTDFTLTISSLQPEDFATYYCQQHYTTPPTFGQGTKVEIKRTVAAPSVFIFPP
SDEQLKSGTASVVCLLNNFYPREAKVQWKVDNALQSGNSQESVTEQDSKDSTYSLSS
TLTLSKADYEKHKVYACEVTHQGLSSPVTKSFNRGEC (SEQ ID NO: 250)
**The nucleic acid sequence of the common light chain is as follows:**

gatatccagatgacccagtcccccagctccctgagcgctagcgtgggcgaccgggtgaccatcacctgcagggcctcccagggcat
cagctcctacctggcttggtatcagcagaagcctggcaaggcccctaagctgctgatctatgccgcttcctccctgcagtccggcgtgc
cttccaggttcagcggctccggcagcggcaccgacttcaccctgaccatctcctccctgcagcctgaggacttcgccacctattattgcc
agcagcactataccacccctcctacctttggccagggcaccaaggtggagatcaagcgtacggtggctgcaccatctgtcttcatcttcc
cgccatctgatgagcagttgaaatctggaactgcctctgttgtgtgcctgctgaataacttctatcccagagaggccaaagtacagtgga
aggtggataacgccctccaatcgggtaactcccaggagagtgtcacagagcaggacagcaaggacagcacctacagcctcagcag
caccctgacgctgagcaaagcagactacgagaaacacaaagtctacgcctgcgaagtcacccatcagggcctgagctcgcccgtca
caaagagcttcaacaggggagagtgttga (SEQ ID NO: 359)

### Example 4: Binding activity assay

The binding activity of intact antibodies was detected by ELISA. The procedures are briefly described as follows: the hNectin-4-His antigen protein of Example 1 was immobilized at a concentration of 1 µg/mL on a plate; the plate was blocked with skimmed milk powder overnight, and then, serially diluted antibodies of interest were added for co-incubation (antibodies in Table 9 were serially 2-fold diluted from 2 µg/mL; antibodies in Tables 10 and 11 were serially 3-fold diluted from 10 µg/mL) at room temperature for 2 h; then an HRP-labeled anti-human-kappa (Sigma, A7164) secondary antibody was added for co-incubation for 1 h, followed by the addition of TMB (Huzhou InnoReagents, TMB-S-001) for chromogenic reaction, and the reaction was stopped with 0.1 M H₂SO₄; after the absorbance at OD450 was read on a microplate reader, the binding EC₅₀ value for each antibody was calculated by four-parameter fitting, and the binding activities of the antibodies were compared. The results are shown in Tables 9-11.

**Table 9: Binding activity of anti-Nectin-4 antibodies**

| Antibody No. | EC₅₀ (µg/mL) | Antibody No. | EC₅₀ (µg/mL) |
|---|---|---|---|
| ASG-22 | 0.042 | 1G8 | 0.068 |
| 3A10 | 0.068 | 1G7 | 0.022 |
| 10F4 | 0.038 | 1B4 | 0.025 |
| 10A4 | 0.041 | 1F3 | 0.041 |
| 1G12 | 0.073 | | |

**Table 10: Binding activity of antibodies**

| Antibody No. | EC₅₀ (µg/mL) | Antibody No. | EC₅₀ (µg/mL) |
|---|---|---|---|
| 1G8-1E10 | 0.006 | 1F3-1C4 | 0.011 |
| 1G8-1C10 | 0.005 | 1F3-1D7 | 0.015 |
| 1G8-2B3 | 0.005 | 1F3-1E1 | 0.017 |
| 1G8-1G3 | 0.004 | 1F3-1F4 | 0.009 |
| 1G8-2C6 | 0.005 | 1F3-1F8 | 0.008 |
| 1G8-2G8 | 0.006 | 1F3-1G7 | 0.008 |
| 1G8-2F2 | 0.012 | 1F3-1G11 | 0.007 |
| 1G8-2F11 | 0.004 | 1F3-1D12 | 0.007 |
| 1G8-2H8 | 0.005 | 1F3-1H3 | 0.008 |
| 1F3-1A9 | 0.007 | 1F3-2B9 | 0.009 |
| 1G8-2H4 | 0.039 | 1F3-2D2 | 0.015 |
| ASG-22 | 0.019 | 1F3-2E8 | 0.009 |
| 1F3 | 0.005 | 1F3-2F3 | 0.011 |
| 1F3-1B1 | 0.016 | 1F3-2B1 | 0.014 |

**Table 11: Binding activity of antibodies**

| Antibody No. | EC₅₀ (µg/mL) | Antibody No. | EC₅₀ (µg/mL) |
|---|---|---|---|
| ASG-22 | 0.042 | ASG-22 | 0.046 |
| 10F4-1A1 | 0.051 | 10F4-1G7 | 0.068 |
| 10F4-1C3 | 0.053 | 10F4-1H6 | 0.063 |
| 10F4-1C10 | 0.058 | 10F4-1H11 | 0.059 |
| 10F4-1F1 | 0.057 | 10F4-2C6 | 0.09 |
| 10F4-1E1 | 0.067 | 10F4-2C12 | 0.06 |
| 10F4-1F8 | 0.054 | 10F4-2F11 | 0.078 |
| 10F4 | 0.041 | 10F4 | 0.043 |

### Example 5: Affinity assay

In this study, the affinity of the antibodies for the human Nectin-4 extracellular antigen was detected by surface plasmon resonance (SPR) technique (BIAcore: GE Healthcare, T200).

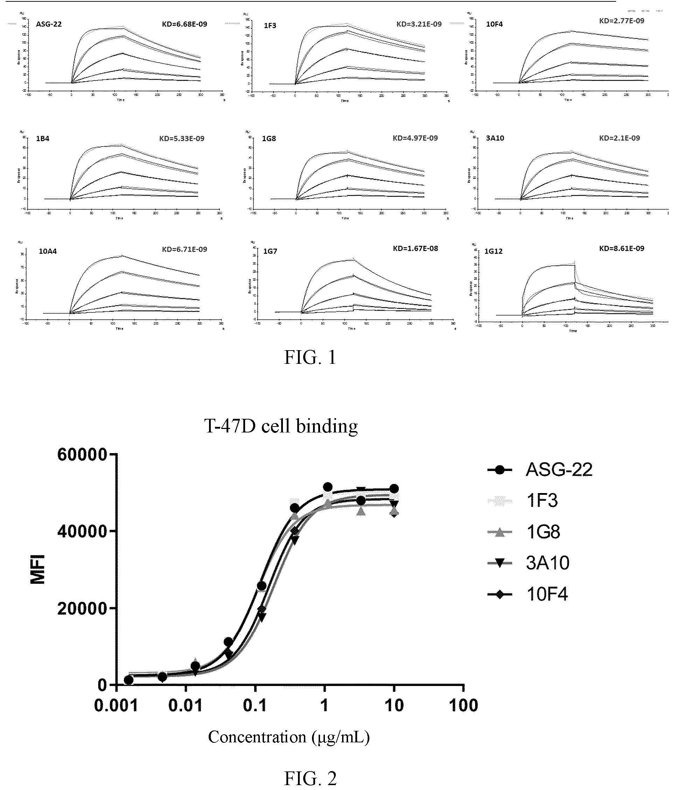

Capture was performed using a Protein A chip (Cat#29127556, GE Healthcare), and 5 µg/mL antibody dilutions (in HBS-EP+(10×): GE Healthcare, Cat# BR-1006-69, with a working concentration of 1×) were directed through the experimental flow channels (Fc2 and Fc4) at a flow rate of 10 µL/min for 10 s; then, the flow rate was adjusted to 30 µL/min, the hNectin-4-His dilutions at different concentrations (0 nM, 1.23 nM, 3.7 nM, 11.1 nM, 33.3 nM, and 100 nM) were sequentially added, and simultaneously directed through the surfaces of the experimental flow channels (Fc2 and Fc4) and reference flow channels (Fc1 and Fc3) with an association time of 120 s and a dissociation time of 180 s, and finally, the chip was regenerated in Glycine 1.5. Fitting analysis was performed using BiaEvaluation 3.2 with a 1:1 fitting model, and the fitting results are shown in Tables 12-13 and FIG. 1.

**Table 12: Affinity of anti-Nectin-4 antibodies to hNectin-4-His**

| Antibody No. | KD (M) | Ka (1/Ms) | Kd (1/s) | Chi²(RU²) |
|---|---|---|---|---|
| 1G8 | 4.97E-09 | 6.05E+05 | 3.00E-03 | 0.626 |
| ASG-22 | 6.03E-09 | 7.23E+05 | 4.36E-03 | 4.9 |
| 1G12 | 8.61E-09 | 5.39E+05 | 4.64E-03 | 0.891 |
| 1B4 | 5.33E-09 | 6.00E+05 | 3.20E-03 | 0.873 |
| 1G7 | 1.67E-08 | 3.77E+05 | 6.31E-03 | 0.124 |
| 10F4 | 2.77E-09 | 3.73E+05 | 1.03E-03 | 3.52 |
| 10A4 | 6.71E-09 | 3.46E+05 | 2.32E-03 | 1.15 |
| 3A10 | 2.1E-09 | 4.13E+05 | 8.67E-04 | 3.24 |
| 1F3 | 3.21E-09 | 8.13E+05 | 2.61E-03 | 8.81 |

**Table 13: Affinity of antibodies**

| Sample | ka (1/Ms) | kd (1/s) | KD (M) | Rmax (RU) | Chi² (RU²) |
|---|---|---|---|---|---|
| 1F3 | 7.85E+05 | 1.95E-03 | 2.49E-09 | 127.6 | 0.795 |
| 1F3-1B1 | 1.20E+06 | 2.04E-04 | 1.7E-10 | 142.5 | 0.974 |
| 1F3-1A9 | 1.55E+06 | 9.94E-04 | 6.41E-10 | 120.8 | 0.949 |
| 1F3-1E4 | 1.94E+06 | 8.25E-04 | 4.26E-10 | 132 | 1.16 |
| 1F3-1E1 | 1.51E+06 | 2.12E-04 | 1.41E-10 | 131.5 | 0.172 |
| 1F3-1G7 | 8.02E+05 | 5.96E-04 | 7.43E-10 | 135.8 | 1.96 |
| 1F3-1F8 | 1.71E+06 | 9.66E-04 | 5.64E-10 | 124.2 | 0.701 |
| 1F3-1H3 | 1.69E+06 | 1.05E-03 | 6.2E-10 | 130.8 | 0.972 |
| 1F3-1G11 | 1.41E+06 | 1.15E-03 | 8.15E-10 | 132.6 | 0.89 |
| 1F3-2B9 | 1.82E+06 | 1.01E-03 | 5.52E-10 | 124.4 | 0.91 |
| 1F3-2B1 | 1.32E+06 | 3.17E-04 | 2.41E-10 | 130.2 | 0.399 |
| 1F3-2F3 | 1.40E+06 | 5.19E-04 | 3.7E-10 | 125.4 | 0.694 |
| 1F3-2E8 | 1.17E+06 | 1.02E-03 | 8.72E-10 | 144.8 | 0.864 |
| 1G8 | 6.05E+05 | 3.00E-03 | 4.97E-09 | 48.4 | 0.626 |
| 1G8-1G3 | 4.71E+05 | 1.41E-03 | 3.00E-09 | 135.4 | 1.47 |
| 1G8-1E10 | 6.65E+05 | 8.70E-04 | 1.31E-09 | 109.6 | 1.71 |
| 1G8-2C6 | 7.34E+05 | 1.37E-03 | 1.87E-09 | 122.1 | 1.27 |
| 1G8-2B3 | 7.42E+05 | 1.47E-03 | 1.98E-09 | 102.6 | 0.817 |
| 1G8-2G8 | 7.58E+05 | 1.42E-03 | 1.87E-09 | 100.8 | 0.974 |
| 1G8-2F2 | 7.84E+05 | 1.66E-03 | 2.12E-09 | 53.8 | 0.516 |
| ASG-22 | 5.77E+05 | 5.81E-03 | 1.01E-08 | 84.9 | 0.69 |
| 1G8-1G3 | 4.71E+05 | 1.41E-03 | 3E-09 | 135.4 | 1.47 |
| 10F4 | 3.75E+05 | 1.21E-03 | 3.23E-09 | 29.4 | 0.232 |
| 10F4-1 | 4.15E+05 | 2.26E-04 | 5.45E-10 | 94.8 | 1.16 |
| 10F4-5 | 3.43E+05 | 1.37E-04 | 3.99E-10 | 135.8 | 1.79 |
| 10F4-3 | 3.46E+05 | 1.22E-04 | 3.52E-10 | 110.3 | 1.26 |
| ASG-22 | 5.40E+05 | 5.90E-03 | 1.09E-08 | 78.3 | 0.714 |
| 10F4-1C3 | 3.70E+05 | 2.18E-04 | 5.89E-10 | 123.6 | 1.3 |
| 10F4-1A1 | 4.30E+05 | 1.86E-04 | 4.32E-10 | 106.1 | 1.47 |
| 10F4-1C10 | 2.65E+05 | 1.16E-04 | 4.35E-10 | 108.3 | 0.238 |
| 10F4-1F8 | 4.24E+05 | 2.37E-04 | 5.58E-10 | 95.8 | 0.764 |
| 10F4-1H6 | 2.99E+05 | 1.51E-04 | 5.05E-10 | 118.7 | 0.348 |
| 10F4-1G7 | 3.89E+05 | 1.45E-04 | 3.74E-10 | 91 | 0.711 |
| 10F4-2C6 | 2.94E+05 | 8.36E-05 | 2.85E-10 | 108.6 | 0.927 |
| 10F4-1H11 | 3.50E+05 | 2.12E-04 | 6.05E-10 | 119 | 1.14 |
| 10F4-2F11 | 4.06E+05 | 1.61E-04 | 3.96E-10 | 81 | 1.15 |
| 10F4-2C12 | 5.70E+05 | 3.04E-04 | 5.33E-10 | 89.4 | 2.15 |

### Example 6: Endocytosis efficiency assay

In this assay, the relative amount of antibodies remaining on the cell surface after co-incubation at 37 °C for a certain period of time was detected by FACS to reflect the endocytosis efficiency. The procedures are briefly described as follows: T-47D cells (human ductal breast cancer cells, derived from the Cell Bank of Chinese Academy of Sciences, Cat.No. TCHu 87); the antibodies were adjusted to 10 µg/mL and co-incubated with the cells on ice for 1 h; the cells were washed 3 times with pre-cooled PBS, and then placed at 37 °C and incubated for 0 h, 2 h and 4 h; 0.2% N₃Na was added to stop the endocytosis reaction; the cells were washed with PBS thrice, followed by the addition of the PE-labeled anti-human Fc secondary antibody (Invitrogen 12-4998-82); the cells were incubated at 4 °C for 30 min; the red fluorescence signal was detected on a flow cytometer. The endocytosis results of the antibodies of interest are shown in Tables 14-15.

**Table 14: Endocytosis results of anti-Nectin-4 antibodies**

| Antibody No. | MFI (mean fluorescence intensity) | | | Endocytosis rate at 2 h | Endocytosis rate at 4 h |
|---|---|---|---|---|---|
| | 0 h | 2 h | 2 h | | |
| ASG-22 | 107097.7 | 53739.06 | 27694.23 | 49.8% | 74.1% |
| 1B4 | 107856.3 | 52035.69 | 26450.5 | 51.8% | 75.5% |
| 1F3 | 121007.3 | 60512.22 | 28271.26 | 50.0% | 76.6% |
| 1G8 | 104517.6 | 56508.13 | 27760.32 | 45.9% | 73.4% |
| 3A10 | 105072.4 | 47336.81 | 20035.45 | 54.9% | 80.9% |
| 10A4 | 107039.3 | 53157.58 | 21358.23 | 50.3% | 80.0% |
| 10F4 | 104284.8 | 56913.51 | 24043.91 | 45.4% | 76.9% |

**Table 15: Endocytosis results of anti-Nectin-4 antibodies**

| Antibody No. | MFI (mean fluorescence intensity) | | | Endocytosis rate at 2 h | Endocytosis rate at 4 h |
|---|---|---|---|---|---|
| | 0 h | 2h | 4h | | |
| ASG-22 | 58,591.2 | 37,973.0 | 37,844.0 | 35.2% | 35.4% |
| 10F4-1 | 58,616.6 | 31,049.2 | 29,267.8 | 47.0% | 50.1% |
| 10F4-3 | 61,231.7 | 34,751.4 | 27,044.8 | 43.2% | 55.8% |
| 10F4-5 | 63,449.1 | 37,060.1 | 20,132.5 | 41.6% | 68.3% |
| 1F3-1B1 | 65,333.1 | 36,442.5 | 33,275.2 | 44.2% | 49.1% |
| 1F3-1E1 | 73,387.8 | 45,103.4 | 31,632.0 | 38.5% | 56.9% |
| 1F3-1G11 | 60,470.8 | 48,303.1 | 42,145.4 | 20.1% | 30.3% |
| 1F3-2B1 | 59,430.8 | 38,232.6 | 30,476.3 | 35.7% | 48.7% |
| 1F3-2E8 | 58,160.4 | 36,480.4 | 32,490.0 | 37.3% | 44.1% |
| 1F3-2F3 | 68,970.9 | 45,880.7 | 31,065.2 | 33.5% | 55.0% |

### Example 7: Cell binding curves

In this study, the binding activity of different antibodies to T-47D cells positive for Nectin-4 expression was detected by FACS, and the EC₅₀ for the binding of antibodies to the cells was analyzed by a four-parameter method. The procedures are briefly described as follows: T-47D cells (the Cell Bank of Chinese Academy of Sciences, TCHu 87) were trypsinized; the antibodies were serially diluted and co-incubated with T-47D cells at 4 °C for 0.5-1 h, followed by the addition of the secondary PE-labeled anti-human Fc antibody (Invitrogen, 12-4998-82); the signal intensity of red fluorescence was detected on a flow cytometer, and curves were plotted using Graphpad Prism and the EC₅₀ was calculated. The binding curves and the EC₅₀ results are shown in Table 16 and FIG. 2.

**Table 16: EC₅₀ value for binding of anti-Nectin-4 antibody to cells**

| EC₅₀ | ASG-22 | 1F3 | 1G8 | 3A10 | 10F4 |
|---|---|---|---|---|---|
| µg/mL | 0.118 | 0.116 | 0.109 | 0.186 | 0.156 |
| nM | 0.788 | 0.770 | 0.727 | 1.241 | 1.039 |

### Example 8: Specificity assay

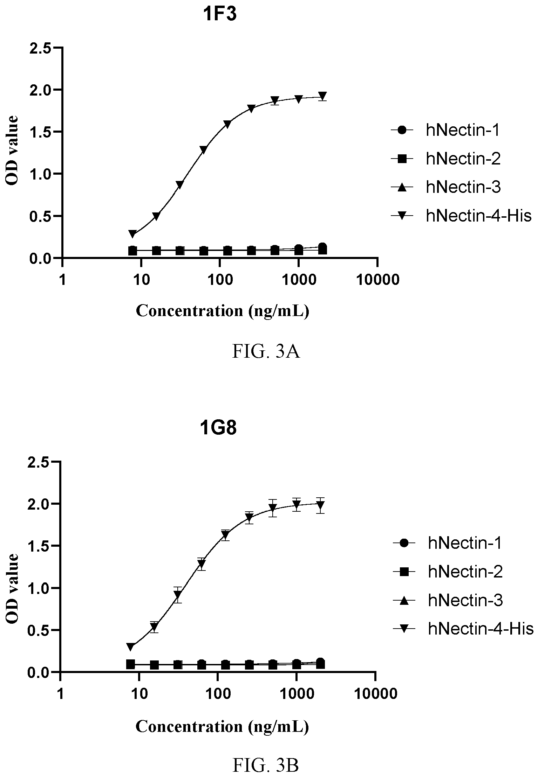

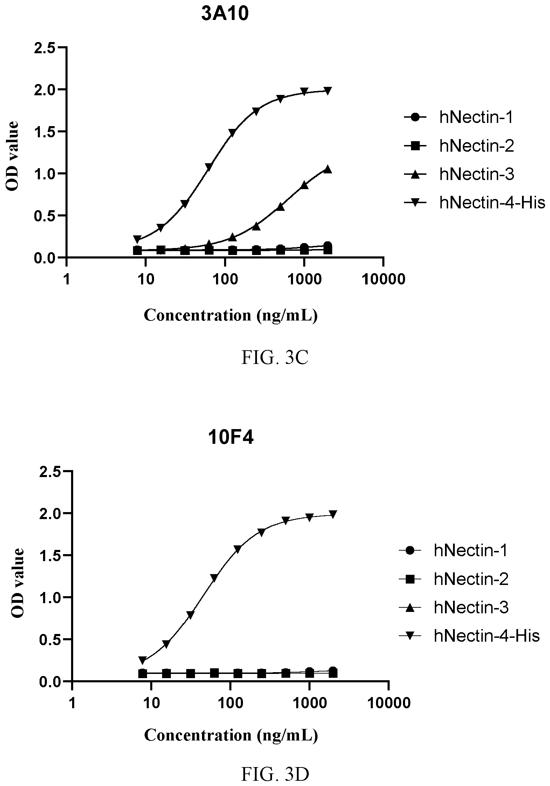

In this assay, the binding of different antibodies to human Nectin-4 homologous proteins hNectin-1 (ACRO, PV1-H5223), hNectin-2 (ACRO, PV2-H52E2), hNectin-3 (ACRO, PV3-H52E4), and hNectin-4-His was detected by ELISA to determine the specificity of the antibodies. For the methodology and procedures, see Example 4. Antigens of interest (1 µg/mL) were immobilized; after blocking, serially diluted antibodies (2-fold serially diluted from 2 µg/mL) were added, and HRP-labeled anti-human-Kappa secondary antibody was added for chromogenic reaction. The results are shown in Table 17. Antibody 3A10 exhibited weaker binding to hNectin-3, while the remaining antibodies exhibited good specificity, which did not bind to the homologous proteins hNectin-1/2/3 but bound to hNectin-4-His. The binding curves are shown in FIG. 3.

**Table 17: Binding of anti-Nectin-4 antibodies to human Nectin-4 homologous proteins**

| Sample | 1F3 | 1G8 | 3A10 | 10F4 |
|---|---|---|---|---|
| | EC₅₀ (µg/mL) | | | |
| hNectin-1 | NA | NA | NA | NA |
| hNectin-2 | NA | NA | NA | NA |
| hNectin-3 | NA | NA | 0.626 | NA |
| hNectin-4-His | 0.040 | 0.041 | 0.062 | 0.045 |

| | | | | |
|---|---|---|---|---|
| Note: NA indicates not available in this assay. | | | | |

### Example 9: Species specificity assay

Nectin-4 has high homology in different species.

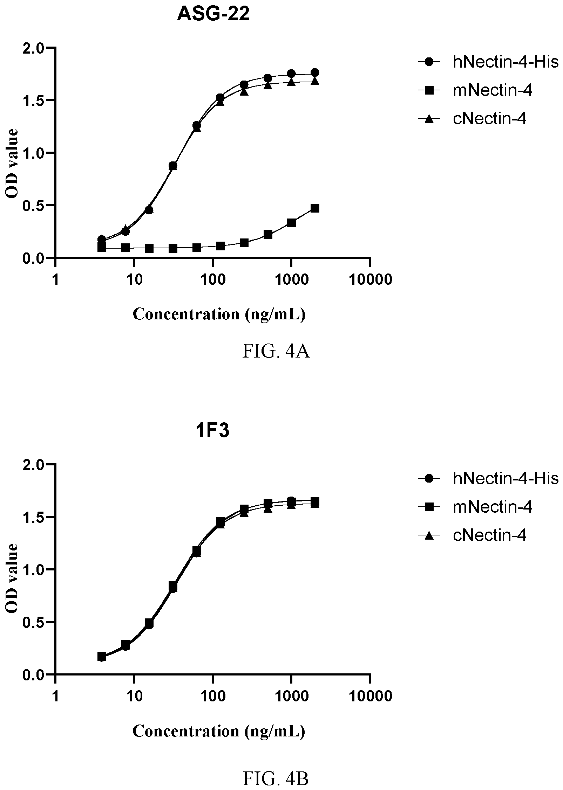

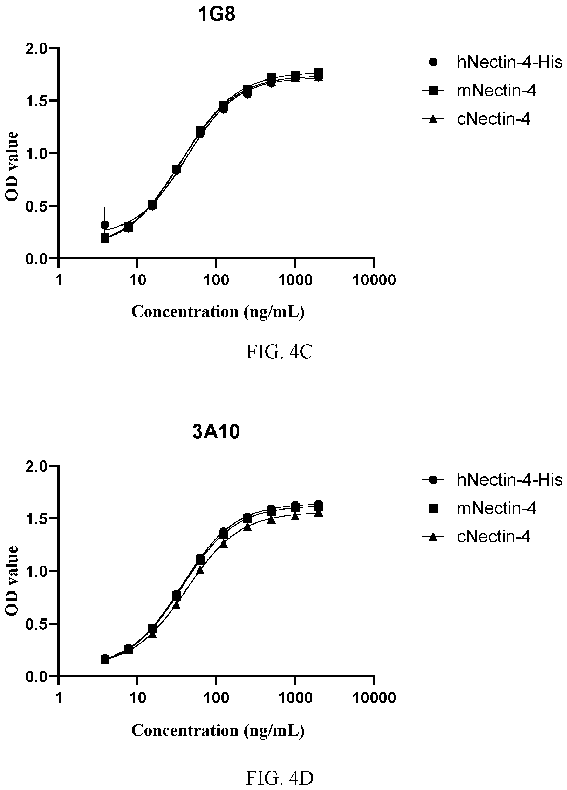

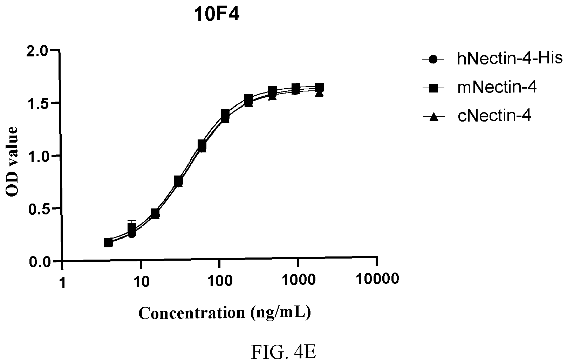

The binding of different antibodies to different species of Nectin-4 proteins was detected by ELISA. hNectin-4-His, monkey cNectin-4 protein (ACRO, NE4-C52H4) and mouse mNectin-4 protein (ACRO, NE4-M52H3) were immobilized; antibodies ASG-22, 1F3, 1G8, 3A10, and 10F4 were serially diluted, and HRP-labeled anti-human-Kappa secondary antibody was added for chromogenic reaction. The results are shown in Table 18 and FIG. 4. The antibodies of interest exhibited similar binding activity to human Nectin-4, cynomolgus monkey Nectin-4, and mouse Nectin-4, while the reference antibody exhibited weak binding to mouse Nectin-4.

**Table 18: Binding of anti-Nectin-4 antibody to Nectin-4 of different species**

| Sample | EC₅₀ (µg/mL) | | | | |
|---|---|---|---|---|---|
| | ASG-22 | 1F3 | 1G8 | 3A10 | 10F4 |
| hNectin-4-His | 0.035 | 0.036 | 0.043 | 0.037 | 0.041 |
| cNectin-4 | 0.033 | 0.034 | 0.036 | 0.042 | 0.041 |
| mNectin-4 | 1.229 | 0.034 | 0.037 | 0.037 | 0.041 |

## Claims

1. An anti-Nectin-4 antibody or antigen-binding fragment, comprising one or more of the following HCDR1, HCDR2 and HCDR3, or variants thereof:
(1) according to the Kabat numbering system, the HCDR1 sequence comprising or consisting of the sequence set forth in any one of SEQ ID NO: 8, SEQ ID NOs: 181-195, SEQ ID NOs: 234-248, and SEQ ID NOs: 304-317, the HCDR2 sequence comprising or consisting of the sequence set forth in any one of SEQ ID NO: 9, SEQ ID NOs: 196-231, SEQ ID NOs: 251-303, and SEQ ID NOs: 318-358, and the HCDR3 sequence comprising or consisting of the sequence set forth in any one of SEQ ID NO: 10, SEQ ID NO: 12, SEQ ID NO: 14, SEQ ID NO: 16, SEQ ID NO: 18, SEQ ID NO: 20, SEQ ID NO: 22, and SEQ ID NO: 24;
(2) the HCDR1, the HCDR2, and the HCDR3 contained in the heavy chain variable region set forth in SEQ ID NO: 7,
for example, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 10;
(3) the HCDR1, the HCDR2, and the HCDR3 contained in the heavy chain variable region set forth in SEQ ID NO: 11,
for example, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 12;
(4) the HCDR1, the HCDR2, and the HCDR3 contained in the heavy chain variable region set forth in SEQ ID NO: 13,
for example, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 14;
(5) the HCDR1, the HCDR2, and the HCDR3 contained in the heavy chain variable region set forth in SEQ ID NO: 15,
for example, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 16;
(6) the HCDR1, the HCDR2, and the HCDR3 contained in the heavy chain variable region set forth in SEQ ID NO: 17,
for example, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 18;
(7) the HCDR1, the HCDR2, and the HCDR3 contained in the heavy chain variable region set forth in SEQ ID NO: 19,
for example, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 20;
(8) the HCDR1, the HCDR2, and the HCDR3 contained in the heavy chain variable region set forth in SEQ ID NO: 21,
for example, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 22;
(9) the HCDR1, the HCDR2, and the HCDR3 contained in the heavy chain variable region set forth in SEQ ID NO: 23,
for example, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 24;
(10) the HCDR1 sequence comprising or consisting of the sequence set forth in X1X2X3MS, the HCDR2 sequence comprising or consisting of the sequence set forth in X1'IX2'X3'X4'X5'X6'X7'X8'X9'YADSVKG, and the HCDR3 sequence comprising or consisting of the sequence set forth in SEQ ID NO: 12, 14, or 16, wherein X1, X2, X3, X1', X2', X3', X4', X5', X6', X7', X8', X9', and X10' denote amino acids independently selected from any one of G, A, V, L, I, S, T, C, M, N, Q, K, R, D, E, F, Y, H, P and W,
for example, X1 denotes S, N, T, D, or G, X2 denotes Y, F, or S, X3 denotes A, G, S, or Y, X1' denotes A, R, G, I, or W, X2' denotes S, K, Y, or D, X3' denotes G, P, S, A, Q, or W, X4' denotes S, T, G, Y, H, D, W, or I, X5' denotes G, D, T, S, A, or K, X6' denotes G, S, D, A, or W, X7' denotes S, Y, T, N, D, V, E, or G, X8' denotes T, A, N, K, I, R, S, or P, and X9' denotes Y, S, H, R, N, G, F, or D;
alternatively, X1 denotes S, N, T, D, or G, X2 denotes Y, N, F, or S, X3 denotes A, G, S, or absence, X1' denotes A, R, G, W, or S, X2' denotes S, K, Y, or D, X3' denotes G, P, S, T, A, Q, or Y, X4' denotes S, T, G, Y, H, D, W, or I, X5' denotes G, D, T, S, F, or K, X6' denotes G, S, D, A, W, or absence, X7' denotes S, Y, T, N, D, V, E, or G, X8' denotes T, A, N, K, I, R, S, or P, and X9' denotes Y, S, H, R, N, F, or D;
alternatively, X1 denotes S, N, D, G, or T, X2 denotes Y, F, or S, X3 denotes A, D, W, S, or Y, X1' denotes A, S, G, or V, X2' denotes S, K, I, Y, or D, X3' denotes G, P, S, A, Q, Y, T, or D, X4' denotes S, T, G, Y, H, D, or W, X5' denotes G, D, T, S, or K, X6' denotes G, S, D, A, or Y, X7' denotes S, Y, T, N, D, V, or G, X8' denotes T, A, N, K, I, R, or S, and X9' denotes Y, S, H, R, N, G, F, or D;
alternatively, the HCDR1 sequence comprises or consists of the sequence set forth in any one of SEQ ID NO: 8 and SEQ ID NOs: 181-195, the HCDR2 sequence comprises or consists of the sequence set forth in any one of SEQ ID NO: 9 and SEQ ID NOs: 196-231, and the HCDR3 sequence comprises or consists of the sequence set forth in SEQ ID NO: 14;
alternatively, the HCDR1 sequence comprises or consists of the sequence set forth in any one of SEQ ID NO: 8, SEQ ID NO: 181, SEQ ID NO: 185, SEQ ID NO: 186, SEQ ID NO: 187, SEQ ID NO: 194, and SEQ ID NOs: 234-248, the HCDR2 sequence comprises or consists of the sequence set forth in any one of SEQ ID NO: 9, SEQ ID NO: 202 and SEQ ID NOs: 251-303, and the HCDR3 sequence comprises or consists of the sequence set forth in SEQ ID NO: 12;
alternatively, the HCDR1 sequence comprises or consists of the sequence set forth in any one of SEQ ID NO: 235, SEQ ID NO: 238, SEQ ID NO: 241, and SEQ ID NOs: 304-317, the HCDR2 sequence comprises or consists of the sequence set forth in any one of SEQ ID NOs: 318-358, and the HCDR3 sequence comprises or consists of the sequence set forth in SEQ ID NO: 16;
wherein the variant sequences have 3, 2, or 1 amino acid difference (preferably a conservative amino acid substitution) from or at least 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, or 99% identity to the corresponding CDR sequences, and the variants retain the binding affinity for Nectin-4, and
optionally, the anti-Nectin-4 antibody or antigen-binding fragment is derived from a bird and a mammal; or, the anti-Nectin-4 antibody is derived from a human, a mouse, a donkey, a rabbit, a goat, a camel, a llama, a horse, or a chicken origin.

2. The anti-Nectin-4 antibody or antigen-binding fragment according to claim 1, wherein the sequences of the framework regions HFR1-HFR4 in the heavy chain variable region are set forth in SEQ ID NOs: 29-32, respectively.

3. The anti-Nectin-4 antibody or antigen-binding fragment according to claim 1, wherein the sequence of the framework region HFR1 in the heavy chain variable region is set forth in the amino acid sequence of positions 1-30 of the sequence set forth in any one of SEQ ID NOs: 41-180, and the sequences of HFR2-HFR4 are set forth in SEQ ID NOs: 30-32, respectively.

4. The anti-Nectin-4 antibody or antigen-binding fragment according to claim 1, wherein the anti-Nectin-4 antibody or antigen-binding fragment comprises a heavy chain variable region selected from the group consisting of the following or a variant thereof:
(1) the heavy chain variable region comprising or consisting of the sequence set forth in SEQ ID NO: 7;
(2) the heavy chain variable region comprising or consisting of the sequence set forth in SEQ ID NO: 11;
(3) the heavy chain variable region comprising or consisting of the sequence set forth in SEQ ID NO: 13;
(4) the heavy chain variable region comprising or consisting of the sequence set forth in SEQ ID NO: 15;
(5) the heavy chain variable region comprising or consisting of the sequence set forth in SEQ ID NO: 17;
(6) the heavy chain variable region comprising or consisting of the sequence set forth in SEQ ID NO: 19;
(7) the heavy chain variable region comprising or consisting of the sequence set forth in SEQ ID NO: 21;
(8) the heavy chain variable region comprising or consisting of the sequence set forth in SEQ ID NO: 23;
(9) the heavy chain variable region comprising or consisting of the sequence set forth in any one of SEQ ID NOs: 41-180; and
(10) the heavy chain variable region comprising or consisting of the sequence set forth in SEQ ID NO: 179,
wherein the variant sequences have 3, 2, or 1 amino acid difference (preferably a conservative amino acid substitution) from or at least 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, or 99% identity to the corresponding variable region sequences, and the variants retain the binding affinity for Nectin-4.

5. The anti-Nectin-4 antibody or antigen-binding fragment according to any one of claims 1-4, wherein the amino acid sequence of the heavy chain constant region of the anti-Nectin-4 antibody is set forth in SEQ ID NO: 37; alternatively, the heavy chain of the anti-Nectin-4 antibody comprises the heavy chain variable region set forth in any one of SEQ ID NO: 7, SEQ ID NO: 11, SEQ ID NO: 13, SEQ ID NO: 15, SEQ ID NO: 17, SEQ ID NO: 19, SEQ ID NO: 21, SEQ ID NO: 23, and SEQ ID NOs: 41-180, and the heavy chain constant region set forth in SEQ ID NO: 37; alternatively, the amino acid sequence of the heavy chain of the anti-Nectin-4 antibody is set forth in any one of SEQ ID NO: 232, SEQ ID NO: 233, SEQ ID NO: 249, and SEQ ID NOs: 360-363.

6. The anti-Nectin-4 antibody or antigen-binding fragment according to any one of claims 1-5, wherein the antibody further comprises the LCDR1, the LCDR2, and the LCDR3 contained in the light chain variable region set forth in SEQ ID NO: 25, for example, according to the Kabat numbering system, the LCDR1 sequence comprising or consisting of the sequence set forth in SEQ ID NO: 26, the LCDR2 sequence comprising or consisting of the sequence set forth in SEQ ID NO: 27, and the LCDR3 sequence comprising or consisting of the sequence set forth in SEQ ID NO: 28; alternatively, the sequences of the framework regions LFR1-LFR4 in the light chain variable region are set forth in SEQ ID NOs: 33-36, respectively; alternatively, the antibody or antigen-binding fragment comprises the light chain variable region set forth in SEQ ID NO: 25; alternatively, the antibody or antigen-binding fragment further comprises the light chain constant region set forth in SEQ ID NO: 38.

7. The anti-Nectin-4 antibody or antigen-binding fragment according to any one of claims 1-6, wherein the antigen-binding fragment is selected from Fab, Fab', F(ab')₂, F(ab)₂, Fd, Fv, dAb, Fab/c, a complementarity determining region fragment, scFv, scFv multimers, disulfide-stabilized Fv (dsFv), (dsFv)₂, bispecific dsFv (dsFv-dsFv'), a diabody, a disulfide-stabilized diabody (ds-Diabody), a multispecific antibody formed from a portion of an antibody comprising one or more CDRs, a single domain antibody (sdAb), a nanobody, a domain antibody, or a bivalent domain antibody.

8. A polypeptide specifically binding to Nectin-4, wherein the polypeptide specifically binding to Nectin-4 is selected from the group consisting of:
(1) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in any one of SEQ ID NO: 8, SEQ ID NOs: 181-195, SEQ ID NOs: 234-248, and SEQ ID NOs: 304-317, the HCDR2 sequence comprises or consists of the sequence set forth in any one of SEQ ID NO: 9, SEQ ID NOs: 196-231, SEQ ID NOs: 251-303, and SEQ ID NOs: 318-358, and the HCDR3 sequence comprises or consists of the sequence set forth in any one of SEQ ID NO: 10, SEQ ID NO: 12, SEQ ID NO: 14, SEQ ID NO: 16, SEQ ID NO: 18, SEQ ID NO: 20, SEQ ID NO: 22, and SEQ ID NO: 24;
(2) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 10;
(3) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in any one of SEQ ID NO: 8, SEQ ID NO: 181, SEQ ID NO: 185, SEQ ID NO: 186, SEQ ID NO: 187, SEQ ID NO: 194, and SEQ ID NOs: 234-248, the HCDR2 sequence comprises or consists of the sequence set forth in any one of SEQ ID NO: 9, SEQ ID NO: 202, and SEQ ID NOs: 251-303, and the HCDR3 sequence comprises or consists of the sequence set forth in SEQ ID NO: 12;
(4) a polypeptide, comprising sequences of an HCDR1, an HCDR2 and an HCDR3, or variants thereof, wherein the polypeptide serves as a portion of the anti-Nectin-4 antibody specifically binding to Nectin-4, wherein the HCDR1 sequence comprises or consists of the sequence set forth in any one of SEQ ID NO: 8 and SEQ ID NOs: 181-195, the HCDR2 sequence comprises or consists of the sequence set forth in any one of SEQ ID NO: 9 and SEQ ID NOs: 196-231, and the HCDR3 sequence comprises or consists of the sequence set forth in SEQ ID NO: 14;
(5) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in any one of SEQ ID NO: 8, SEQ ID NO: 235, SEQ ID NO: 238, SEQ ID NO: 241, and SEQ ID NOs: 304-317, the HCDR2 sequence comprises or consists of the sequence set forth in any one of SEQ ID NO: 9 and SEQ ID NOs: 318-358, and the HCDR3 sequence comprises or consists of the sequence set forth in SEQ ID NO: 16;
(6) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 18;
(7) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 20;
(8) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 22;
(9) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 24;
(10) a polypeptide, comprising the sequence set forth in SEQ ID NO: 7 or a variant thereof, wherein the polypeptide serves as a portion of an anti-Nectin-4 antibody specifically binding to Nectin-4;
(11) a polypeptide, comprising the sequence set forth in SEQ ID NO: 11 or a variant thereof, wherein the polypeptide serves as a portion of an anti-Nectin-4 antibody specifically binding to Nectin-4;
(12) a polypeptide, comprising the sequence set forth in SEQ ID NO: 13 or a variant thereof, wherein the polypeptide serves as a portion of an anti-Nectin-4 antibody specifically binding to Nectin-4;
(13) a polypeptide, comprising the sequence set forth in SEQ ID NO: 15 or a variant thereof, wherein the polypeptide serves as a portion of an anti-Nectin-4 antibody specifically binding to Nectin-4;
(14) a polypeptide, comprising the sequence set forth in SEQ ID NO: 17 or a variant thereof, wherein the polypeptide serves as a portion of an anti-Nectin-4 antibody specifically binding to Nectin-4;
(15) a polypeptide, comprising the sequence set forth in SEQ ID NO: 19 or a variant thereof, wherein the polypeptide serves as a portion of an anti-Nectin-4 antibody specifically binding to Nectin-4;
(16) a polypeptide, comprising the sequence set forth in SEQ ID NO: 21 or a variant thereof, wherein the polypeptide serves as a portion of an anti-Nectin-4 antibody specifically binding to Nectin-4;
(17) a polypeptide, comprising the sequence set forth in SEQ ID NO: 23 or a variant thereof, wherein the polypeptide serves as a portion of an anti-Nectin-4 antibody specifically binding to Nectin-4; and
(18) a polypeptide, comprising the sequence set forth in any one of SEQ ID NOs: 41-180 or a variant thereof, wherein the polypeptide serves as a portion of an anti-Nectin-4 antibody specifically binding to Nectin-4;
optionally, the polypeptide further comprises the sequences set forth in SEQ ID NO: 26, SEQ ID NO: 27 and SEQ ID NO: 28, or the sequence set forth in SEQ ID NO: 25 or a variant thereof,
wherein the variant sequences have 3, 2, or 1 amino acid difference (preferably a conservative amino acid substitution) from or at least 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, or 99% identity to the corresponding sequences, and the variants retain the binding affinity for Nectin-4.

9. A biomaterial, wherein the biomaterial is
(1) a nucleic acid molecule, encoding the anti-Nectin-4 antibody or antigen-binding fragment according to any one of claims 1-7, or encoding the polypeptide specifically binding to Nectin-4 according to claim 8;
(2) a vector, comprising the nucleic acid molecule encoding the anti-Nectin-4 antibody or antigen-binding fragment according to any one of claims 1-7 or encoding the polypeptide specifically binding to Nectin-4 according to claim 8; or
(3) a host cell, comprising the nucleic acid molecule encoding the anti-Nectin-4 antibody or antigen-binding fragment according to any one of claims 1-7 or encoding the polypeptide specifically binding to Nectin-4 according to claim 8.

10. A conjugate, comprising the anti-Nectin-4 antibody or antigen-binding fragment according to any one of claims 1-7 and a conjugated moiety, wherein the conjugated moiety is a purification tag (such as a His tag), a detectable label, a drug, a toxin, a cytokine, an enzyme, or a combination thereof; alternatively, the conjugated moiety is a radioisotope, a fluorescent substance, a chemiluminescent substance, a colored substance, a chemotherapeutic agent, a biotoxin, a therapeutic agent, a prodrug, a peptide, a protein, an enzyme, a virus, a lipid, a biological response modifier, or polyethylene glycol.

11. A kit, comprising the anti-Nectin-4 antibody or antigen-binding fragment according to any one of claims 1-7, the polypeptide specifically binding to Nectin-4 according to claim 8, or the conjugate according to claim 10, wherein optionally, the kit further comprises a second antibody specifically recognizing the anti-Nectin-4 antibody; optionally, the second antibody further comprises a detectable label, such as a radioisotope, a fluorescent substance, a chemiluminescent substance, a colored substance, or an enzyme; optionally, the kit is for use in detecting the presence or level of Nectin-4 in a sample; optionally, the kit further comprises an antibody or antigen-binding fragment thereof for an additional antigen, and/or a cytotoxic agent, and optionally, instructions for use.

12. A pharmaceutical composition, comprising the anti-Nectin-4 antibody or antigen-binding fragment according to any one of claims 1-7, the polypeptide specifically binding to Nectin-4 according to claim 8, or the conjugate according to claim 10, wherein optionally, the pharmaceutical composition further comprises a pharmaceutically acceptable carrier and/or excipient; alternatively, the pharmaceutical composition is in a form suitable for administration by subcutaneous injection, intradermal injection, intravenous injection, intramuscular injection, or intralesional injection.

13. Use of the anti-Nectin-4 antibody or antigen-binding fragment according to any one of claims 1-7, the polypeptide specifically binding to Nectin-4 according to claim 8, or the conjugate according to claim 10 in treating and/or preventing a disease or in preparing a medicament for treating and/or preventing a disease, wherein alternatively, the disease is a disease associated with the abnormal expression of Nectin-4; alternatively, the disease is a tumor expressing or overexpressing Nectin-4; alternatively, the disease is a cancer expressing or overexpressing Nectin-4; alternatively, the disease is a solid tumor or a hematologic tumor; and alternatively, the disease is selected from breast cancer, pancreatic cancer, bladder cancer, urothelial carcinoma, melanoma, lung cancer, head and neck cancer, cervical cancer, ovarian cancer, choriocarcinoma, skin cancer, esophageal cancer, gastric cancer, uterine cancer, gallbladder cancer, liver cancer, hepatocellular carcinoma, urethral carcinoma, renal pelvis cancer, ureteral cancer, colorectal cancer, colon cancer, and prostate cancer.

14. A method for treating a disease, comprising: administering to a patient in need an effective amount of the anti-Nectin-4 antibody or antigen-binding fragment thereof according to any one of claims 1-7, the polypeptide specifically binding to Nectin-4 according to claim 8, or the conjugate according to claim 10, wherein alternatively, the disease is a disease associated with the abnormal expression of Nectin-4; alternatively, the disease is a tumor expressing or overexpressing Nectin-4; alternatively, the disease is a cancer expressing or overexpressing Nectin-4; alternatively, the disease is a solid tumor or a hematologic tumor; and alternatively, the disease is selected from breast cancer, pancreatic cancer, bladder cancer, urothelial carcinoma, melanoma, lung cancer, head and neck cancer, cervical cancer, ovarian cancer, choriocarcinoma, skin cancer, esophageal cancer, gastric cancer, uterine cancer, gallbladder cancer, liver cancer, hepatocellular carcinoma, urethral carcinoma, renal pelvis cancer, ureteral cancer, colorectal cancer, colon cancer, and prostate cancer.
